(19) Europäisches Patentamt
European Patent Office
Office européen des brevets

(11) EP 2 327 787 A1

(12) EUROPEAN PATENT APPLICATION

(43) Date of publication:
**01.06.2011 Bulletin 2011/22**

(21) Application number: **10013126.7**

(22) Date of filing: **30.11.2006**

(51) Int Cl.:
***C12N 15/86*** *(2006.01)* ***A61K 39/215*** *(2006.01)*
***A61K 39/12*** *(2006.01)*

(84) Designated Contracting States:
**AT BE BG CH CY CZ DE DK EE ES FI FR GB GR HU IE IS IT LI LT LU LV MC NL PL PT RO SE SI SK TR**

(30) Priority: **01.12.2005 EP 05026255**

(62) Document number(s) of the earlier application(s) in accordance with Art. 76 EPC:
**06829221.8 / 1 951 882**

(71) Applicants:
- **Consejo Superior de Investigaciones Cientificas 28006 Madrid (ES)**
- **Fort Dodge Veterinaria, S.A. 17813 Vall de Bianya Gerona (ES)**

(72) Inventors:
- **Enjuanes Sanches, Luis 28109 Madrid (ES)**
- **Zuniga Lucas, Sonia 28760 Madrid (ES)**
- **Plana Durán, Joan 17811 Santa Pau Girona (ES)**
- **Sola Gurpegui, Isabel 28700 Madrid (ES)**

(74) Representative: **UEXKÜLL & STOLBERG Patentanwälte Beselerstrasse 4 22607 Hamburg (DE)**

Remarks:
This application was filed on 01-10-2010 as a divisional application to the application mentioned under INID code 62.

# (54) Nucleic acids encoding TGEV and PRRSV sequences for improved expression of PRRSV sequences

(57) The present invention relates to nucleic acids comprising:
(a) sequences of a replication competent transmissible gastroenteritis virus (TGEV), which sequences encode a TGEV replicase under the control of expression regulatory sequences, wherein the replicase is expressed in a host cell and will initiate replication of the nucleic acid and thus increase the number of nucleic acids in the cell; and
(b) a sequence encoding at least one neutralizing epitope of ORF5 of porcine reproductive and respiratory syndrome virus (PRRSV), which sequence includes a disulfide bridge forming residue; and
(c) a sequence encoding at least one further polypeptide capable of increasing an immune response against PRRSV.

The present invention further relates to vectors, virus particles and host cells comprising these nucleic acids as well as their use for the preparation of vaccines, specifically for the preparation of vaccines.

EP 2 327 787 A1

## Description

The present invention is directed to nucleic acids comprising sequences of a replication competent transmissible gastroenteritis virus (TGEV), which sequences encode a TGEV replicase under the control of expression regulatory sequences so that expression of the replicase in a cell containing the nucleic acid will initiate replication of the nucleic acid and thus increase the number of nucleic acids in the cell. The nucleic acids of the present invention further encode neutralizing epitopes of PRRSV proteins and one or more polypeptides capable of increasing an immune response against PRRSV. The use of nucleic acids encoding polypeptides of two different PRRSV proteins provides virus constructs with improved stability *in vitro*. The present invention is further directed to the use of these nucleic acids for the preparation of pharmaceutical compositions in general and specifically for the preparation of vaccines with improved efficacy.

## TECHNICAL BACKRGOUND

Therapy approaches that involve the insertion of a functional gene into a cell to achieve a therapeutic effect are also referred to as gene therapy approaches, as the gene serves as a drug. Gene therapy is a technique primarily for correcting defective genes responsible for disease development.

A carrier molecule also referred to as a vector is used to deliver the therapeutic gene to the patient's target cells. Currently, the most common vector is a virus that has been genetically altered to carry human or animal genes. Viruses have evolved a way of encapsulating and delivering their genes to human or animal cells in a pathogenic manner. Scientists have taken advantage of this capability and manipulate the virus genome to remove disease-causing genes and insert therapeutic genes.

These viral vectors were used for expressing heterologous genes that cause an immunogenic response in the subject receiving the vector and thus immunize that subject. In that case the viral vector serves as a vaccine.

Transmissible gastroenteritis virus is a member of the family of coronaviruses. Coronaviruses are ssRNA(+) viruses which have the largest genome so far found in RNA viruses with a length between 25 and 31 kilobases kb (see SIDDELL S.G. 1995, The Coronaviridae). When a coronavirus infects a cell, the genomic RNA (gRNA) replicates in the cytoplasm and a set of subgenomic RNAs (sgRNA) of positive and negative polarity is produced (SETHNA *et al*., 1989; SAWICKI & SAWICKI, 1990; and VAN DER MOST and SPAAN, 1995).

Due to the fact that the coronaviruses replicate in the cytoplasm, use of coronaviruses as a vector for gene therapy and vaccination has been suggested (ENJUANES *et al*., 2003). Specifically, defective interfering (DI) genomes of coronaviruses were produced. These DI genomes are deletion mutants which require the presence of a complementing or helper virus for replication and/or transcription (see CHANT *et al*., 1994; WO97/34008; Spanish patent application P9600620; IZETA *et al*., 1999; SÁNCHEZ *et al*., 1999).

The entire genome of a coronavirus was cloned in the form of an infectious cDNA (ALMAZAN *et al*., 2000 and WO01/39797). The cloning of the entire genome allowed the preparation of infectious vectors containing heterologous sequences suitable for expression of large proteins in a host cell.

The potential of the cloned viral genome for expression of heterologous sequences was reviewed in ENJUANES *et al*., 2003.

Using the cloned virus the structure of the genome and relevance of the coronaviral genes for infection were assessed by preparing deletion mutants. It was found that genes 3a, 3b and 7 are non-essential for replication of the viral nucleic acid and that absence of the genes reduces pathogenicity of the virus (ORTEGO *et al*., 2002 and 2003; SOLA *et al*., 2003).

Porcine reproductive and respiratory syndrome virus (PRRSV) was first identified in 1991 as the causative agent of a new disease in pigs (WENSVOORT *et al*., 1991). Since then, PRRSV has become one of the leading causes of economic losses in swine operations worldwide and it is currently accepted as the most important infectious disease of swine, causing reproductive failure in adult animals and severe pneumonia in neonatal pigs. PRRSV is a member of *Arteriviridae* family that belongs to *Nidovirales* order. Two genotypes (American and European) are recognized (MURTAUGH *et al*., 1995). PRRSV is an enveloped virus with a single-stranded positive-sense RNA genome of 14.5 Kb. The 5' two-thirds of the genome encode the replicase polyproteins (ppla and pplab), the rest of the genomic RNA encodes three minor membrane-associated glycoproteins (Gp2, Gp3 and Gp4) and the three mayor structural proteins (Gp5, M and N).

Pigs are generally infected with PRRSV by following exposure of the mucosal surface or the respiratory tract to the virus. A hallmark of the swine antibody response against PRRSV is the abundant non-neutralizing antibodies detected early in the infection, followed by a low neutralizing antibody titer that appears more than 3 weeks after infection. Experimental data showing the importance of neutralizing antibodies in protection against PRRSV infection have been collected in the last years (LOPEZ and OSORIO, 2004, ANSARI *et al*., 2006). PRRSV glycoprotein Gp5 contains most of the virus neutralizing epitopes. Proteins Gp4 and M of PRRSV also induce neutralizing antibodies, nevertheless, Gp5 specific antibodies neutralize more efficiently PRRSV than those binding other viral proteins (OSTROWSKI *et al*., 2002).

PRRSV infection also induces weaker and delayed T cell mediated, immune responses in relation to those elicited by other viruses. Both responses are required for complete virus clearance.

Although the immune response to PRRSV is poorly understood, some vaccines are being commercialized. Current vaccines against PRRSV have several drawbacks. PRRSV, either wild type or attenuated, induces a low level of cell-mediated immunity (MEIER *et al.*, 2003) and neutralizing antibodies (NA) do not develop until a late phase of the infection (MEIER *et al.*, 2003, VEZINA *et al.*, 1996, YOON *et al.*, 1995). Further, genetic diversity of PRRSV is thought to influence the efficacy of vaccine under field conditions (LABARQUE *et al.*, 2004, PESCH *et al.*, 2005), although some degree of cross protection exists (MENGELING *et al.*, 2003 a, 2003b). Modified live vaccines protect against challenge with homologous isolates, but generally have a limited effect against challenge with heterologous viruses (MENG, 2000). Furthermore, live vaccines provide partial protection against clinical disease, but did not prevent infection (OSORIO *et al.*, 1998) and, more importantly, they can revert to virulence (BOTNER *et al.*, 1997, NIELSEN *et al.*, 2001). Killed PRRSV vaccines, on the other hand, have proved to be less effective in prevention of both infection and disease (OSTROWSKI *et al.*, 2002)

The problem underlying the present invention thus resides in providing effective vaccine vectors with good safety and immunogenicity against PRRSV.

## SUMMARY OF THE INVENTION

According to a first aspect of the present invention a nucleic acid is provided which comprises:

(a) sequences of a replication competent transmissible gastroenteritis virus (TGEV), which sequences encode a TGEV replicase under the control of expression regulatory sequences, wherein the replicase is expressed in a host cell and will initiate replication of the nucleic acid and thus increase the number of nucleic acids in the cell; and

(b) a sequence encoding at least one neutralizing epitope of ORF5 of porcine reproductive and respiratory syndrome virus (PRRSV), which sequence includes a disulfide bridge forming residue; and

(c) a sequence encoding at least one further polypeptide capable of increasing an immune response against PRRSV.

According to a second aspect of the present invention a nucleic acid is provided which comprises:

(a) sequences of a replication competent transmissible gastroenteritis virus (TGEV), which sequences encode a TGEV replicase under the control of expression regulatory sequences so that expression of the replicase in a cell containing the nucleic acid will initiate replication of the nucleic acid and thus increase the number of nucleic acids in the cell; and

(b) sequences encoding multimers of one or several neutralizing epitopes of ORF5 of PRRSV and at least one further polypeptide capable of increasing an immune response against PRRSV.

According to a third aspect of the present invention a nucleic acid is provided which comprises:

(a) sequences of a replication competent transmissible gastroenteritis virus (TGEV), which sequences encode a TGEV replicase under the control of expression regulatory sequences so that expression of the replicase in a cell containing the nucleic acid will initiate replication of the nucleic acid and thus increase the number of nucleic acids in the cell; and

(b) a sequence encoding at least one neutralizing epitope of ORF5 of porcine reproductive and respiratory syndrome virus (PRRSV); and

(c) a sequence encoding at least one further polypeptide capable of increasing an immune response against PRRSV, which stabilizes the expression of the sequence of (b), such that more than 60 % of the cells infected with this nucleic acid sequence express the gene product of the sequence of (b) after 20 passages of the virus *in vitro.*

In a preferred embodiment the neutralizing epitope is defined by the amino acid sequence TYQYIYN (SEQ ID NO: 10).

In another preferred embodiment the nucleic acid comprises the sequences of ORF5 and ORF 6 of PRRSV each under the control of a separate expression regulatory sequences.

In a more preferred embodiment the nucleic acid consists of (1) the sequence of ORF5 of PRRSV under the control of the transcription regulating sequence of gene 3a, (2) the sequence of ORF6 of PRRSV under the control of the expression regulatory sequence $TRS_{22N}$ set forth as SEQ ID NO: 19 and (3) the sequence of the S gene derived from the attenuated strain PTV of TGEV.

The replication competent TGEV sequences need not but may further encode other TGEV proteins. The TGEV sequences may thus encode a fully infectious TGEV virus and the sequences of the neutralizing epitope or multimers of neutralizing epitopes just comprise a replication competent nucleic acid. The present invention further relates to vectors comprising a respective nucleic acid and host cells comprising the vector. The host cells may be capable of complementing TGEV genes that may have been deleted from the nucleic acids of the present invention. The host cell thus may be a packaging cell line or may contain a helper virus expressing TGEV genes, so that a TGEV virus particle is formed that comprises the sequences of at least one neutralizing epitope of PRRSV. Virus particles obtained by association of the TGEV coat proteins with the replication competent but non-infectious nucleic acids of the present invention are an especially preferred embodiment of the present invention (corresponding virus particles have also been referred to as pseudo-viruses). Finally, the present invention is also directed to the medical use of the nucleic acids, the virus vectors and the host cells specifically to the use as a vaccine for treating or protecting animals, such as a swine against infectious diseases. The vaccine can thus be administered to an animal to reduce or eliminate the symptoms of a subsequent infection of a wildtype virus.

## DETAILED DESCRIPTION OF THE INVENTION

The present invention is thus directed to a nucleic acid comprising:

(a) sequences of a replication competent transmissible gastroenteritis virus (TGEV), which sequences encode a TGEV replicase under the control of expression regulatory sequences, wherein the replicase is expressed in a host cell and will initiate replication of the nucleic acid and thus increase the number of nucleic acids in the cell; and

(b) a sequence encoding at least one neutralizing epitope of ORB5 of porcine reproductive and respiratory syndrome virus (PRRSV), which sequence includes a disulfide bridge forming residue; and

(c) a sequence encoding at least one further polypeptide capable of increasing an immune response against PRRSV.

The present inventors have surprisingly found that expression of at least one neutralizing epitope or multimers of such an epitope of ORF5 of PRRSV and at least one further polypeptide capable of increasing an immune response against PRRSV in the context of the TGEV vector backbone leads to an efficient and very safe vaccine against PRRSV with improved efficacy.

The invention is thus further directed to a nucleic acid comprising:

(a) sequences of a replication competent transmissible gastroenteritis virus (TGEV), which sequences encode a TGEV replicase under the control of expression regulatory sequences so that expression of the replicase in a cell containing the nucleic acid will initiate replication of the nucleic acid and thus increase the number of nucleic acids in the cell; and

(b) a sequence encoding at least one neutralizing epitope of ORF5 of porcine reproductive and respiratory syndrome virus (PRRSV); and

(c) a sequence encoding at least one further polypeptide capable of increasing an immune response against PRRSV, which stabilizes the expression of the sequence of (b), such that more than 60 % of the cells infected with this nucleic acid sequence express the gene product of the sequence of (b) after 20 passages of the virus *in vitro.*

In one embodiment the nucleic acid is stabilized by the at least one further polypeptide capable of increasing an immune response against PRRSV to an extent, such that at least 65%, 70%, 75%, 80%, 85%, 90%, or 100% of the cells infected with the' nucleic acid sequence of the invention express the at least one neutralizing epitope of ORF5 of PRRSV after 20 passages of the virus *in vitro*. In a more preferred embodiment, at least 80% of the cells express the at least one neutralizing epitope of ORF5 of PRRSV after 20 passages of the virus *in vitro.*

In the present application the term "passage" is used to refer to a process, wherein a monolayer of TGEV sensitive cells (such as ST cells) in a culture vessel, such as a Petri dish, is infected with the virus, the supernatant is collected after virus replication, for example after 24 hours, and transferred to a fresh monolayer of cells. The passaging may include cloning steps, for example transfection to obtain viruses from the DNA clones (using for example BHK cells) or plaque purification of the virus.

The use of the TGEV-based vector will allow the delivery of the epitopes to the desired tissues with high antigen expression levels and the vector will be engineered to generate a safe vaccine.

One of the problems during PRRSV infection is that the pigs only secrete low amounts of IFN-γ very late during infection. Type I interferon (IFN-α/β) induction is essential to promote antiviral cell (PFEEFER *et al.*, 1998) and humoral immune responses (LE BON *et al.*, 2001). It has further been described that IFN-α and IL-12 are involved in the T cell differentiation to IFN-γ antigen-specific secreting cells (COUSENS *et al.*, 1999). However, it has been shown that TGEV is a potent IFN-α inducer. IFN induction by TGEV is a process mediated by the M protein (CHARLEY and LAUDE, 1988; LAUDE *et al.*, 1990). In addition, TGEV vectors present antigens at mucosal sites, eliciting mucosal and systemic immune responses. Thus, the use of TGEV as a vector for PRRSV epitopes will advantageously improve the induction of protection against PRRSV.

The TGEV-based vector can exhibit a modified vector-tropism. In one embodiment of the invention the vector is a respiratory tract directed vector. This is achieved by using the spike (S) gene from a respiratory virus. In an alternative embodiment the vector is an enteric tract directed vector. This is achieved by using the S gene from an enteric strain of TGEV. The S gene may further be a sequence directing the vector to both the respiratory and the enteric tract (see Figure 9).

In a preferred embodiment a modified S gene is used (SEQ ID No:1), which provides enteric and respiratory tropism and is very stable upon passage in swine testis (ST) cells in culture. This gene is a chimeric protein derived from the S protein from TGEV clone $C_{11}$ (which is virulent with enteric and respiratory tropism) and clone PTV. The recombinant S protein was engineered using the first 1208 nt from the TGEV $C_{11}$ S gene and the rest of the chimeric sequence from the PTV virus. The final chimeric protein was obtained by providing a recombinant virus carrying this protein to pigs and recovery of a virus having the chimeric sequence as set forth as SEQ ID NO: 1. This protein provides enteric and respiratory tropism to TGEV, is very stable upon passage in tissue culture on swine testis (ST), provides high titers (>$10^8$) for the TGEV when grown in tissue culture on ST cells and does not loose the dual tropism in vitro (as can be seen from Figure 9).

In its broadest aspect the nucleic acid of the present invention is characterized as a nucleic acid encoding replication competent TGEV sequences that means sequences encoding a TGEV replicase under the control of expression regulatory sequences so that expression of the replicase in a cell containing the nucleic acid will initiate replication of the nucleic acid and thus increase the number of nucleic acids in the cell. Once a cell is infected by the nucleic acids of the present invention, the gene for the replicase will be expressed and the nucleic acid will be replicated. The more copies of the nucleic acid are present in the cell the more epitopes will be expressed.

The term "transcription regulatory sequence", as used herein, means a sequence or a fragment of a sequence capable of driving the synthesis of subgenomic viral RNAs associated with the transcription regulatory sequence. Examples for such transcription regulatory sequences are the transcription-regulating sequences (TRS) naturally associated with the viral RNAs. In a preferred embodiment, the TRS will be the TRS of gene 3a and in the case of a dicistronic vector encoding two antigens, the second TRS will be a synthetic TRS derived from N gene ($TRS_{22N}$; SEQ ID NO: 19) that was optimized for gene expression both in the minigenome and full-length cDNA expression systems (ALONSO *et al.*, 2002).

In accordance with the present invention "neutralizing epitope" means epitopes which are involved in virus neutralization. Antibodies which recognize Gp5 (encoded by ORF5) of PRRSV neutralize PRRSV more effectively than the ones specific for other viral proteins (OSTROWSKI *et al.*, 2002). It is to be understood that the nucleic acid of the invention encodes at least a neutralizing epitope of ORF5 of PRRSV, but can also encode more neutralizing epitopes or a larger part of ORF5, for example the whole Gp5 protein.

In a preferred embodiment the neutralizing epitope is defined by the amino acid sequence TYQYIYN (SEQ ID NO: 10).

In one embodiment the nucleic acids of the invention encode at least one neutralizing epitope of ORF5 of PRRSV and a disulfide bridge forming residue which can be used to connect a further polypeptide to said neutralizing epitope. For example, an epitope of ORF6, which encodes the M protein of PRRSV, or the complete M protein can be coupled to the epitope of ORF 5 via a disulfide bridge.

In another embodiment the nucleic acids according to the invention encode multimers of neutralizing epitopes of ORF5. A "multimer" comprises at least 2 copies of one epitope. A "multimer" may comprise repetitions of Gp5 or other PRRSV derived protein sequences inducing neutralizing antibodies to PRRSV. This definition includes synthetic protein domains (or peptides) derived from PRRSV Gp5 or other PRRSV proteins with a modified sequence by point mutagenesis leading to an immunogenic structure providing higher protection because of an enhanced neutralizing response or removal of decoy epitopes.

Typically, a nucleic acid will encode for multimers comprising 2 to 5 repetitions of the sequence encoding the neutralizing epitope of ORF5 of PRRSV. These multimers may be connected by a nucleic acid sequence encoding a flexible linker of 2 to 8, preferably 3 to 6 amino acids. These "linker residues" will improve the flexibility of the epitopes. A specifically preferred linker is the sequence Gly-Gly-Pro-Gly-Gly (SEQ ID NO: 15).

In a further embodiment the nucleic acids encode neutralizing epitopes of ORF5 that have been modified to knock out glycosylation sites, in view of a recent report describing that lack of glycosylation leads to an increase in the induction of PRRSV neutralizing antibodies (ANSARI *et al*., 2006). In a preferred embodiment such glycosylation sites are amino acid 46 and/or 53 of Gp5 from PRRSV Olot 91 strain.

The glycosylation of the Gp5 protein epitopes can interfere with the induction of antibodies resulting in a decreased immune response. Therefore, the epitopes encoded by the nucleic acids of the invention are able to provide increased stimulatory effects of the immune response compared to naturally occurring epitopes.

In a preferred embodiment of the invention the nucleic acid encodes at least one neutralizing epitope of ORF5 and ORF6 but no further PRRSV polypeptides. Thus, in further preferred embodiments of the invention the nucleic acid comprises a neutralizing epitope of ORF5 and at least one epitope of ORF 6 of PRRSV. In a further preferred embodiment the nucleic acid comprises a neutralizing epitope of ORF5 and a neutralizing epitope of ORF 6 of PRRSV. In yet another embodiment the nucleic acid consists of a neutralizing epitope of ORF5 and a neutralizing epitope of ORF 6 of PRRSV. In yet another preferred embodiment the nucleic acid consists of a neutralizing epitope of ORF5 and whole ORF 6 of PRRSV.

This results in a very effective vaccine against PRRSV. In another preferred embodiment the nucleic acid encodes whole ORF5 and ORF6 but no further PRRSV polypeptide.

In a further preferred embodiment the nucleic acid encodes one neutralizing epitope of ORF5 and whole ORF6 but no further PRRSV polypeptide. In yet another preferred embodiment the neutralizing epitope of ORF5 is defined by SEQ ID NO: 10.

In another preferred embodiment the nucleic acid encodes at least one neutralizing epitope of ORF5, whole ORF6 and additionally at least one neutralizing epitope of Gp4.

Vaccines comprising Gp5 of PRRSV alone have proven to be efficient with regard to protecting piglets against PRRSV infection. This was shown by the prevention of loss of weight in vaccinated piglets after infection with PRRSV (see Figure 1) and by the prevention of an infection with PRRSV in vaccinated piglets (see Figure 2). However, the recombinant virus comprising Gp5 alone has proven be unstable upon passaging of the virus in tissue culture. As can be seen from Figure 3, Gp5 mRNA could no longer be detected in ST cells infected with the recombinant virus from passage 5 in tissue culture (p5), whereas it is still detectable in ST cells infected with the recombinant virus from passage 2 (p2). This may be due to deletions occurring in the virus during the passages.

In contrast, the use of nucleic acids encoding Gp5 (ORF5) and M (ORF6) of PRRSV has shown to provide constructs with substantially improved stability. More specifically, in addition to conferring protection against PRRSV infection, dicistronic constructs comprising nucleic acids encoding Gp5 and M of PRRSV are more stable even after being passaged 20 times in tissue culture (see Figures 5, 6 and 8). The virus further could be rescued from infected piglets. The stability of the construct was further shown by RT-PCR analysis (see Figure 7), which shows that transcripts of both the ORF5 and ORF6 gene could be detected for virus clones rescued from ST cells after 20 passages *in vitro*. This data clearly show that the dicistronic constructs of the present invention comprising nucleic acids encoding Gp5 and M of PRRSV are not only able to provide a virus that retains its capability of both growing in cell cultures even after 20 passages, but also still expresses both proteins encoded by the dicistronic construct after 20 passages. This improved stability in cell culture is especially important for the propagation of the recombinant virus *in vitro* for vaccination purposes. Further, due to the improved stability, it is also possible to recover recombinant TGEV expressing PRRSV Gp5 and M from lung and gut tissues of vaccinated piglets capable of being further propagated in cell culture using ST cells (see Figures 9 and 10).

Other PRRSV derived proteins that could be expressed to induce a protective immune response are the glycoproteins Gp2, Gp3 and Gp4. It has been shown that these proteins are incorporated into virions as a multimeric complex (WISSINK *et al*., 2005) and are essential for virus infectivity. Therefore, in a further embodiment of the invention the nucleic acids encode at least one neutralizing epitope of Gp5 and at least one neutralizing epitope of Gp2, Gp3 or Gp4. In a preferred embodiment the nucleic acids encode a neutralizing epitope of Gp5 and Gp2. In another embodiment the nucleic acids encode a neutralizing epitope of Gp5 and Gp3 and in yet another embodiment the nucleic acids encode a neutralizing epitope of Gp5 and Gp4. Further embodiments comprise the combinations Gp5 with Gp2 and Gp3, Gp5 with Gp2 and Gp4 or Gp5 with Gp3 and Gp4.

In a further embodiment of the invention the nucleic acid comprises a nucleic acid comprising:

(a) sequences of a replication competent transmissible gastroenteritis virus (TGEV), which sequences encode a TGEV replicase under the control of expression regulatory sequences, wherein the replicase is expressed in a host cell and will initiate replication of the nucleic acid and thus increase the number of nucleic acids in the cell;

(b) residues 118 to 138 of SEQ ID NO: 2 or a sequence having a similarity of 90% to these residues under the control of an expression regulatory sequence; and

(c) SEQ ID NO: 3 or a sequence having a similarity of 90% to SEQ ID NO: 3 under the control of an expression regulatory sequence; wherein

the sequences of (a) and (b) and (c) are each under the control of a different expression regulatory sequence.

In one embodiment the nucleic acid characterized by the specific residues of SEQ ID NO: 2 and SEQ ID NO: 3 further comprises nucleic acid sequence SEQ ID NO: 12, which encodes the lysosomal targeting signal of lysosomal integral membrane protein-II (LIMP-II) or a sequence having a similarity of 90% to SEQ ID NO:12. LIMP-11 directs the fusion protein to the lysosome (see Figure 21). Antigens directed to the lysosome will be presented by MHC II, improving the humoral immune response against them (RODRIGUEZ *et al.*, 2001). The sequence encoding the LIMP-II protein is preferably fused to the SEQ ID NO: 2, so that the nucleic acid is capable of expressing a fusion protein which comprises the ORF5 sequence of PRRSV and the LIMP-II sequence. In one aspect of this embodiment of the present invention, the nucleic acid may contain sequences encoding a multimer of the epitope of ORF5 as defined above (i.e. 2 to 5 repetitions of the sequence having the residues 118 to 138 of SEQ ID NO: 2 optionally interrupted by a linker of 2 to 8, preferably 3 to 6 amino acids) and SEQ ID NO:3, but the nucleic acid does not contain any further sequences derived from PRRSV, i.e. no other sequences having a homology of 80%, preferably 90 or 95%, to the known sequence of the strain PRRSV Olot 91.

The use of virulent or attenuated viruses and of recombinant antigens of PRRSV may lead to a delay in the induction of virus neutralizing antibodies. In one embodiment of the invention the nucleic acids encode polypeptides which improve antigen presentation. In a preferred embodiment the lysosomal targeting signal of lysosomal integral membrane protein-II (LIMP-II) is used for such purpose. In a further embodiment the whole LIMP-II protein is used and other embodiments comprise any fragment of LIMP-II containing the lysosomal targeting signal. In a more preferred embodiment a fusion protein comprising the lysosomal targeting signal of LIMP-II and ORF5 and/or ORF6, or at least one neutralizing epitope thereof, is encoded by the nucleic acids of the invention, wherein the lysosomal targeting signal of LIMP-II is either fused to the ORF5 or ORF6 and in the case that both, ORF5 and ORF6, are present, the lysosomal targeting signal of LIMP-II is fused to one of them while ORF5 and ORF6, or the neutralizing epitopes thereof, are connected by a disulfide bridge.

In a further embodiment the polypeptide capable of increasing an immune response against PRRSV is an interleukin. It has been recently described that IL-10 and IL-12 play a role in pulmonary defense mechanisms against PRRSV infection in piglets (CHUNG and CHAE, 2003). The inability of swine to resolve a PRRSV infection could be related to the low levels of IFN-$\gamma$ during infection (SURADHAT *et al.*, 2003). IL-12 is an inducible cytokine composed of two linked subunits (p35 an p40) that induce IFN expression by T and natural killer cells. Accordingly, preferred interleukins are for example IL-2, IL-10 or IL-12. In a preferred embodiment it is interleukin 12 (IL-12) and in a most preferred embodiment it is only the p35 subunit of IL-12.

The replication competent TGEV vector may be infectious or not. A nucleic acid that contains at least all sequences necessary for replication of the nucleic acid, produces one or several coat proteins and associates with the coat proteins to a viral particle that will allow infection of other cells is referred to as an infectious nucleic acid in accordance with the present invention.

In an especially preferred aspect, the present invention provides a virus particle that comprises the above nucleic acid and at least one TGEV coat protein. The virus particle may comprise more than one and even all TGEV coat proteins. A corresponding virus particle will be capable of entering a host cell by way of infection. However, the nucleic acid of such a virus particle may still be infectious or non-infectious, as it need not encode all of the TGEV coat proteins necessary to produce a virus particle. If the nucleic acid is a non-infectious nucleic acid in the sense of the present application, the virus particle is prepared using a packaging host cell or a helper virus that complements the TGEV genes. The use of packaging host cells or helper viruses for obtaining virus particles comprising an incomplete genome of a virus is well known in the art. This way of proceeding has specific advantages, as the virus particle is per se infectious (i.e. can infect a cell once), but the nucleic acid is not capable of producing further infectious virus particles. In other words, the sequences derived from TGEV do not encode proteins that will be capable of associating with the nucleic acid to form a new virus particle. These virus particles thus are extremely safe and still provide a high immunogenic response against the epitopes expressed by the nucleic acids.

According to an alternative embodiment of the present invention the TGEV infectious viral particles obtainable from the association of TGEV proteins and the nucleic acid sequences are attenuated viral particles. This has the advantage that the subject to be treated using the nucleic acids of the present invention will be vaccinated at the same time against TGEV and against PRRSV.

The nucleic acids of the present invention may comprise sequences encoding all proteins of TGEV. Alternatively, the nucleic acids may comprise sequences only encoding the TGEV proteins needed for a replication competent TGEV vector. The nucleic thus preferably encodes the TGEV replicase. According to an especially preferred embodiment, the nucleic acid encodes a replication competent, but non-infectious TGEV vector that comprises sequences encoding the TGEV replicase and the TGEV N protein and none of the other TGEV proteins. This vector has the specific advantage that the TGEV vector will be highly amplified in the host cell and thus produce large amounts of the epitopes. At the same time this vector is extremely safe, as it is non-infectious.

The term "nucleic acids encoding TGEV proteins" is used herein to refer to nucleic acid sequences as disclosed in PENZES *et al*. (2001), or nucleic acid sequences having a similarity of at least 60%, preferably at least 75% and most preferably at least 95% to these sequences. For example specific alternative sequences may be used to differentiate between TGEV vaccinated animals and TGEV infected animals (as outlined in more detail below). In the TGEV based vector exemplified in the present application corresponding nucleotide substitutions have been introduced using RT-PCR at positions 6752, 18997, 20460, and 21369, respectively. Especially nucleic acid sequences encoding the TGEV replicase, N protein, M protein, E protein or S protein of TGEV as used herein means nucleic acid sequences as disclosed in PENZES *et al*., 2001 (with or without the amendments mentioned above). It is of course also possible to use other TGEV strains or to include further deletions, substitutions, insertions or additions into the nucleic acid sequence. According to a further aspect the TGEV sequences thus differ from the sequences disclosed in PENZES *et al*. but still have a similarity of at least 60%, preferably at least 75% and most preferably at least 95% to these sequences.

The term "nucleic acids encoding PRRSV proteins" is used herein to refer to PRRSV wild-type nucleic acid sequences or nucleic acid sequences having a similarity of at least 60%, preferably at least 75% and most preferably at least 95% to these sequences.

For the purposes of the present application sequence similarity is determined using the ClustalW computer program available from the European Bioinformatics Institute (EBI), unless otherwise stated.

The infectious TGEV vector need not contain genes 3a, 3b and 7, as these are known to be non-essential. The proteins encoded by genes 3a, 3b and 7 of TGEV may modulate the immune response against TGEV and where it is desirable to modulate TGEV interaction with the host, these genes may be maintained in the TGEV vector.

Additionally, the infectious TGEV vector has been engineered, expressing PRRSV antigens in different positions of the TGEV genome, such as replacing nsp2 protein or between nsp1 and nsp2 proteins of the replicase polyproteins, similarly as described for MHV and HCoV-229E (GRAHAM *et al*., 2005; HERTZIG *et al*., 2004).

Further, since the delay in the appearance of neutralizing antibodies after PRRSV infection could be due to the presence of decoy (immunodominant) epitopes or to the presence of epitopes recognized by regulatory T cells inhibiting a strong immune response, additional infectious TGEV vectors have been engineered which expresses PRRSV M and modified versions of Gp5. In this engineered Gp5 proteins, the decoy epitope and T-regulatory cell epitopes have been deleted, maintaining the ability of the mutated Gp5 to interact with M protein and, therefore, improving stability of the recombinant viruses.

The protein coding sequences within the nucleic acids of the present invention are preferably linked to sequences controlling the expression of these genes in the host cells or organisms. The genes encoding the epitopes or further polypeptides may for example be flanked by transcription regulatory sequences (TRS) and/or internal ribosome entry site (IRES) sequences to increase transcription and/or translation of the protein coding sequences. Respective TRS and IRES sequences are well known in the art. Preferably the TRS is the TRS of gene 3a, whereas in the case of a dicistronic vector encoding two antigens, the second TRS will preferably be a synthetic TRS derived from N gene ($TRS_{22N}$; SEQ ID NO: 19).

The nucleic acids of the present invention may be in the form of DNA or RNA. Within the scope of the present invention specifically recombinant. RNA molecules are encompassed which are encoded by one of the above nucleic acids.

In a preferred embodiment the nucleic acid comprises the sequences of ORF5 and ORF 6 of PRRSV each under the control of a separate expression regulatory sequences.

In a more preferred embodiment the nucleic acid consists of (1) the sequence of ORF5 of PRRSV under the control of the transcription regulating sequence of gene 3a, (2) the sequence of ORF 6 of PRRSV under the control of the expression regulatory sequence $TRS_{22N}$ set forth as SEQ ID NO: 19 and (3) the sequence of the S gene derived from the attenuated strain PTV of TGEV. An example of such a S protein is that of SEQ ID NO: 1.

According to a further aspect the present invention is directed towards vectors comprising one of the above nucleic acids. The vector can be a cDNA vector and preferably is a BAC derived vector, such as BAC-$TGEV^{FL}$. The vector is preferably capable of replicating the nucleic acid within a specific host cell or a number of host cells.

Host cells, which comprise a vector comprising one of the above nucleic acids are a further subject of the present invention. The cell may be a bacterial cell, a yeast cell, an insect cell, an animal cell or a human cell. According to a preferred embodiment the cell is a porcine swine testis (ST) cell line, such as the cell line deposited under ATCC CRL1746.

The present invention is further directed to polypeptides comprising the neutralizing epitopes of ORF5 and ORF6. In one embodiment such a polypeptide consists of the neutralizing epitope of ORF5 and ORF6 and is encoded by SEQ ID NO: 13 or a sequence having a similarity of 90% to SEQ ID NO: 13. In another embodiment such a polypeptide comprises the neutralizing epitopes of ORF5 and ORF6 and additionally the lysosomal targeting sequence of LIMP-II. In a preferred embodiment such a polypeptide consists of the neutralizing epitope of ORF5 and ORF6 and the lysosomal targeting sequence of LIMP-II and is encoded by SEQ ID NO:14 or a sequence having a similarity of 90% to SEQ ID NO: 14.

The present invention further is directed to pharmaceutical compositions comprising one of the nucleic acids, viral RNAs, polypeptides or vectors of the present invention or a host cell as described above. The pharmaceutical composition may further comprise one or more pharmaceutically acceptable carrier(s), excipient (s) and/or adjuvant (s).

In an especially preferred embodiment the present invention relates to vaccines capable of protecting an animal against the disease caused by an infectious virus comprising a nucleic acid, a viral RNA, a vector, a polypeptide or a host cell of the present invention. The vaccine may also comprise one or more pharmaceutically acceptable carrier(s), excipient(s) and/or adjuvant(s).

Adjuvants and carriers suitable for administering genetic vaccines and immunogens via the mucosal route are known in the art. Conventional carriers and adjuvants are for example reviewed in KIYONO *et al*., 1996. The addition of chemokines that are used to modulate immune responses are also encompassed by the present invention. Respective compounds and their medical use has been reviewed in TOKA *et al*., 2004. It is specifically advantageous to use one of granulocyte/macrophage colony-stimulating factor, interleukin-2 (IL-2), IL-12, IL-18. IL-12 is an inducible cytokine composed of two linked subunits (p35 an p40) that induce IFN expression by T and natural killer cells. It has been described that the co-administration of recombinant single-chain IL-12 (rIL-12) as adjuvant with a vaccine strain of PRRSV enhanced PRRSV-specific INF-$\gamma$ producing cells (Foss *et al*., 2002). IL-12 also reduced the titer of PRRSV in cell-culture and decreased viremia in PRRSV-infected animals, suggesting that IL-12 enhanced the development of antigen-specific cell-mediated immunity and promoted the production of IFN-$\gamma$ in the lungs of PRRSV infected animals (CARTER and CURIEL, 2005). A limitation of IL-12 administration is that the p40 subunit is expressed in excess in relation to the p35 subunit and, apparently, p40 dimers bind to IL-12 receptor and act as an IL-12 antagonist. Recently, is has been shown that expression of IL-12 p35 subunit as a molecular adjuvant enhanced both humoral and cell-mediated immune responses without the concerns associated with IL-12 p40 (OSORIO and GHIASI, 2005). Combinatorial approaches utilizing several cytokines and chemokines might also be applied. In addition, as more is discovered regarding the requirements for memory development of T cells, boosters involving key cytokines such as IL-15 and IL-23 may prove beneficial to long-term maintenance of the memory pool.

The vaccine is preferably suitable for treating a mammal, for example a swine. The vaccination of sows is especially preferred.

In accordance with the present invention vaccines are provided, which are preferably capable of inducing both a systemic immune response and a mucosal immune response against PRRSV and/or TGEV.

The vaccine may further be a multivalent vaccine which is capable to provide protection against one or several other pig pathogens. The multivalent vaccine thus may further comprise antigens derived from other viral and/or bacterial pathogens and/or proteins which will provide immunity against pathogens. Examples antigens from further viral pathogens comprise antigens derived from one of Swine Influenza Viruses, Porcine Parvovirus, Porcine Circovirus Type 2, Classical Swine Fever, African Swine Fever, Foot-and-Mouth Disease, Pseudo-Rabies Virus, Porcine Circovirus Type 1, Porcine Adenoviruses, Porcine Enteroviruses, Porcine Respiratory Coronavirus, Porcine Rotavirus, Encephalomyocarditis Virus, Porcine Epidemic Diarrhea Virus, Blue Eye Disease Viruses, Hepatitis E Virus and/or West Nile Virus, Nipah virus. The use of antigens derived from one or several of Swine Influenza Viruses, Porcine Parvovirus, Porcine Circovirus Type 2, Classical Swine Fever, African Swine Fever and/or Foot-and-Mouth Disease is specifically preferred.

Examples antigens from bacterial pathogens comprise antigens derived from one of Mycoplasma hyopneumoniae, Actinobacillus pleuropneumoniae, Actinobacillus suis, Haemophilus parasuis, Pasteurella multocida type A (toxins), Pasteurella multocida type D (toxins), Bordetella bronchiseptica, Isospora suis, Brachyspira hyodysenteriae, Brachyspira pilosicoli, Lawsonia intracellularis, Erysipelothrix rhusiopathiae, Escherichia coli, Salmonella enterica, Mycoplasma hyorinis, Streptococcus suis, Clostridium perfringens, Clostridium difficile, Clostridium novyi, Brucella abortus and/or Candidatus helicobacter suis. The use of antigens derived from one or several of Mycoplasma hyopneumoniae, Actinobacillus pleuropneumoniae, Actinobacillus suis, Haemophilus parasuis, Pasteurella multocida type A (toxins), Pasteurella multocida type D (toxins), Bordetella bronchiseptica, Isospora suis, Brachyspira- hyodysenteriae, Brachyspira pilosicoli, Lawsonia intracellularis, Erysipelothrix rhusiopathiae, Escherichia coli and/or Salmonella enterica is specifically preferred.

The further viral or bacterial antigen may be present in the multivalent vaccine as an attenuated or inactivated virus or bacterium. Alternatively the multivalent vaccine may comprise a nucleic acid sequence encoding one or several of the further viral or bacterial antigens. That sequence may be within the same nucleic acid molecule that also encodes the TGEV and PRRSV sequences or it may be present in the vaccine as a separate nucleic acid molecule.

The multivalent vaccine may further comprise nucleic acid sequences encoding proteins which will confer protection against pig pathogens. Respective nucleic acid sequences could for example code for one or several antibodies having specificity for bacterial or viral diseases. Alternatively or additionally, the nucleic acid may code for the sequence of the porcine prion protein and may thus be used to vaccinate against diseases caused by prions. Again, the sequences encoding proteins which will confer'protection against pig pathogens may be within the same nucleic acid molecule that also encodes the TGEV and PRRSV sequences or may be present in the vaccine as a separate nucleic acid molecule.

These vaccines may be administered in accordance with methods routinely used in the art. Specifically vaccine may be administered by intramuscular, intravenous, intranasal, intravaginal, oronasal administration: or any other common method known in the art.

The vaccine may be a modified live vaccine or an inactivated (killed) vaccine.

Modified live vaccines (MLV) are produced from an isolate of viruses or bacteria. The viruses becomes attenuated, which means that it cannot cause disease, but is still capable of replicating in the host cells and thus stimulates immunity (PANLEY *et al*., 1989; PASTORET *et al*., 1997).

Inactivated (killed) vaccines are produced by growing the viruses or bacteria and subsequently inactivating or killing the organisms using either heat or chemicals. In inactivated (killed) vaccines an adjuvant is added to the antigenic phase of the killed organisms to support stimulation of the immune system, since dead viruses or bacteria are not easily recognized by the immune system in absence of an adjuvant. The adjuvant further holds the killed organisms at the injection site and thus provides sufficient time for the immune cells to respond to it (PARLEY *et al*., 1989; PASTORET *et al*., 1997). Inactivated vaccines may be killed viruses, killed bacteria (also known as bacterins), or killed toxins (or toxoids).

In a preferred embodiment the vaccine is an inactivated vaccine diluted with porcine circovirus (PCV) Type1-Type2 inactivated vaccine (FENAUX *et al*., 2003, 2004) that was previously adjuvanted with sulfolipo-cyclodextrin. In a preferred embodiment the inactivated vaccine is diluted with PCV Type1-Type2 inactivated vaccine that was previously adjuvanted with 20% sulfolipo-cyclodextrin. In a further especially preferred embodiment the inactivated vaccine is diluted 1:3 with the porcine circovirus (PCV) Type1-Type2 inactivated vaccine.

The vaccine preferably contains the TGEV/PRRSV nucleic acids in concentration producing a live viral titer in the range of about $10^4$ to $10^9$, most preferably about $10^5$ to $10^8$. The live viral titer is determined as plaque forming units (Pfu) in ST cells.

The vaccines of the present invention allow one of ordinary skill to diagnose whether an animal is infected with a wild-type virus or has been vaccinated. According to a further aspect, the present invention is thus directed to methods for diagnosing whether an animal is infected with a virus or has been vaccinated using a vaccine of the present invention, which methods comprise steps, wherein the diagnosis uses antibodies specific for proteins of the wild-type virus not expressed by the vaccine strain (i. e., 3a, 3b, 7 or E). Differentiation of TGEV vaccinated animals from TGEV infected animals could alternatively be carried out using RT-PCR and sequence markers introduced into the recombinant TGEV genome at positions 6752, 18997, 20460, and 21369, which should encode G, C, T, and C, respectively.

The differentiation between vaccinated animals and wild-type PRRSV infected animals can be carried out using antibodies specific for proteins not present in the recombinant virus.

Spike (S) genes normally have the disadvantage that they provide tropism to either the respiratory or the enteric tract. However, clones are described in the art that provide dual tropism to both the respiratory and the enteric tract. However, such S genes do not provide high titers of expression for the corresponding virus in cell culture or in vivo or they loose the dual tropism after several passages in cell culture. So far no S gene has been described that can provide tropism to both the respiratory and the enteric tract, that is very stable upon passage in tissue culture on swine testis (ST) cells and provides high titers (>$10^8$) for the TGEV when grown in tissue culture on ST cells and does not loose the dual tropism *in vitro*.

Accordingly, in a further aspect the invention is directed to a nucleic acid comprising a sequence having at least 95% similarity to the sequence of SEQ ID NO: 1, wherein the protein encoded by the sequence having at least 95% similarity to the sequence of SEQ ID NO: 1 is a spike protein, which spike protein, when present as part of a TGEV virus, is capable of inducing TGEV infections in the respiratory tract and the enteric tract of pigs, wherein the infections are characterized by a viral titer of at least $1 \times 10^7$ PFU in ST cells infected with TGEV derived from 1 gram of animal respiratory tract tissue 2 days post infection of the animal; and of a titer of at least $1 \times 10^6$ PFU in ST cells infected with TGEV derived from 1 gram of animal enteric tract tissue 2 days post infection of the animal. More preferably, the nucleic acid comprises the sequence shown in SEQ ID NO:1.

The capacity of the TGEV for infecting respiratory tract and enteric tract tissue of animals was determined as described in Example 17 using a plaque titration assay on ST cells, wherein ST cells in culture were infected with virus obtained from the tissues of infected animals two days post infection. In this context, the virus titer obtained in the ST cells is indicative of the activity of the recombinant TGEV in vivo in the infected animal.

In a further embodiment, the nucleic acid described above comprising a sequence having at least 95% similarity to the sequence of SEQ ID NO: 1, wherein the protein encoded by the sequence having at least 95% similarity to the sequence of SEQ ID NO: 1. is a spike protein, which spike protein, when present as part of a TGEV virus, is capable of inducing TGEV infections in the respiratory tract and the enteric tract of pigs, wherein the infections are characterized by a viral titer of at least $1 \times 10^7$ PFU in ST cells infected with TGEV derived from 1 gram of animal respiratory tract tissue 2 days post infection of the animal; and of a titer of at least $1 \times 10^6$ PFU in ST cells infected with TGEV derived from 1 gram of animal enteric tract tissue 2 days post infection of the animal, does not comprise a sequence encoding ORF5 of PRRSV or a fragment thereof encoding a neutralizing epitope.

In a further aspect the present invention is directed towards vectors comprising the nucleic acids described above comprising a sequence having at least 95% similarity to the sequence of SEQ ID NO: 1, wherein the protein encoded by the sequence having at least 95% similarity to the sequence of SEQ ID NO: 1 is a spike protein, which spike protein, when present as part of a TGEV virus, is capable of inducing TGEV infections in the respiratory tract and the enteric tract of pigs, wherein the infections are characterized by a viral titer of at least $1 \times 10^7$ PFU in ST cells infected with TGEV derived from 1 gram of animal respiratory tract tissue 2 days post infection of the animal; and of a titer of at least $1 \times 10^6$ PFU in ST cells infected with TGEV derived from 1 gram of animal enteric tract tissue 2 days post infection of the animal. In a preferred embodiment, these vectors may be a cDNA vector, more preferably a BAC-TGEV vector. In another preferred embodiment, the vector is capable of replicating the nucleic acid within a host cell. The present invention is also directed to host cells comprising a vector as those described above.

A further aspect of the present invention is the spike protein encoded by the sequence having at least 95% similarity to the sequence of SEQ ID NO: 1, which spike protein, when present as part of a TGEV virus, is capable of inducing TGEV infections in the respiratory tract and the enteric tract of pigs, wherein the infections are characterized by a viral titer of at least $1 \times 10^7$ PFU in ST cells infected with TGEV derived from 1 gram of animal respiratory tract tissue 2 days post infection of the animal; and of a titer of at least $1 \times 10^6$ PFU in ST cells infected with TGEV derived from 1 gram of animal enteric tract tissue 2 days post infection of the animal.

The invention is further directed to virus particles comprising the nucleic acids as described above.

Further, pharmaceutical preparations comprising a nucleic acid comprising a sequence having at least 95% similarity to the sequence of SEQ ID NO: 1, wherein the protein encoded by the sequence having at least 95% similarity to the sequence of SEQ ID NO: 1 is a spike protein, which spike protein, when present as part of a TGEV virus, is capable of inducing TGEV infections in the respiratory tract and the enteric tract of pigs, wherein the infections are characterized by a viral titer of at least $1 \times 10^7$ PFU in ST cells infected with TGEV derived from 1 gram of animal respiratory tract tissue 2 days post infection of the animal; and of a titer of at least $1 \times 10^6$ PFU in ST cells infected with TGEV derived from 1 gram of animal enteric tract tissue 2 days post infection of the animal, a vector comprising such a nucleic acid, a host cell comprising such a vector, or a spike protein encoded by such a nucleic acid are a further aspect of the present invention.

Additionally encompassed by the present invention are vaccines comprising a sequence having at least 95% similarity to the sequence of SEQ ID NO: 1, wherein the protein encoded by the sequence having at least 95% similarity to the sequence of SEQ ID NO: 1 is a spike protein, which spike protein, when present as part of a TGEV virus, is capable of inducing TGEV infections in the respiratory tract and the enteric tract of pigs, wherein the infections are characterized by a viral titer of at least $1 \times 10^7$ PFU in ST cells infected with TGEV derived from 1 gram of animal respiratory tract tissue 2 days post infection of the animal; and of a titer of at least $1 \times 10^6$ PFU in ST cells infected with TGEV derived from 1 gram of animal enteric tract tissue 2 days post infection of the animal, a vector comprising such a nucleic acid, a host cell comprising such a vector, or a spike protein encoded by such a nucleic acid.

## Brief description of the Figures:

Figure 1 shows the results of weight measurements of piglets after in vivo inoculation with rTGEVs expressing PRRSV Gp5. Piglets were inoculated with rTGEVs expressing Gp5 using $TRS_{3a}$ ($\Delta$3-$TRS_{3a}$-ORF5; square symbol) or $TRS_{22N}$ ($\Delta$3-$TRS_{22N}$-ORF5; triangular symbol). The weight of the piglets was measured at 1, 2, 3 and 4 weeks after challenge with PRRSV. Piglets vaccinated with recombinant virus expressing PRRSV Gp5 gain body weight efficiently (i.e. they develop normally), whereas the piglets vaccinated with a control ("C-") show impaired development with only marginal gain of body weight.

Figure 2 shows the results of an ELISA assay detecting antibodies specific for PRRSV N (ORF7). Serum samples from piglets infected with rTGEVs expressing PRRSV Gp5 (either $\Delta$3-$TRS_{3a}$-ORF5 or $\Delta$3-$TRS_{22N}$-ORF5) or piglets infected with a virus vector not expressing ORF5 and ORF6 (non-vaccinated control) were collected at 2, 3 and 4 weeks after challenge with PRRSV. No or only minor amounts of antibodies against protein N of PRRSV could be detected in vaccinated piglets after challenging the piglets with PRRSV compared to the non-vaccinated control.

Thus, while $\Delta$3-$TRS_{3a}$-ORF5 is able to provided full protection against PRRSV protection, $\Delta$3-$TRS_{22N}$-ORF5 provides partial protection against PRRSV. In contrast, the animals of the control group were not protected against infection with PRRSV.

Figure 3 shows the results of Northern-blot analysis of the stability of rTGEVs expressing PRRSV Gp5. Recombinant TGEV viruses expressing Gp5 (using either $TRS_{3a}$ or $TRS_{22N}$) were passaged in tissue culture. ST cells were infected with virus from passage 2 (p2) and 5 (p5). As seen in the Figure, virus from p2 expressed Gp5 mRNA, whereas virus from p5 contains deletions and did not express Gp5 mRNA.

Figure 4 shows the schematic structure of the cDNA encoding the pBAC-TGEV$^{FL}$-$S_{PTV}$-$TRS_{3a}$-ORF5-$TRS_{22N}$-$ORF6_{(C306T)}$. As shown the heterologous PRRSV genes ORF5 and ORF6 were cloned in the same rTGEV viral vector.

Figure 5 shows the expression of Gp5 (ORF5) of PRRSV in ST cells infected with recombinant TGEV expressing PRRSV Gp5 and M proteins (pBAC-TGEV$^{FL}$-$S_{PTV}$-$TRS_{3a}$-ORF5-$TRS_{22N}$-$ORF6_{(C306T)}$) as evaluated by immunofluorescence analysis (antibodies specific for the TGEV N protein were used to visualize the virus (red colour) and antibodies specific for the PRRSV ORF5 (Gp5) protein were used to visualize Gp5 expressing cells (green colour)). After 20 passages in tissue culture (including the cloning steps), the virus was recovered and used for infection of ST cells. This analysis showed that 80% of the infected cells expressed Gp5, and that 100% of these cells were positive for the M protein (not shown in the graph). This graph also shows that the dicistronic construct has improved stability *in vitro* and is stable even after 20 passages in culture.

Figure 6 show the results of a FACS analysis of the infected cells of Figure 5. Antibodies specific for TGEV N protein were used to detect the recombinant virus, whereas Gp5 expressing cells were visualized using a polyvalent antibody specific for Gp5. The data clearly show that 80% of the cells infected with recombinant TGEV expressing PRRSV Gp5 and M proteins (pBAC-TGEV$^{FL}$-$S_{PTV}$-$TRS_{3a}$-ORF5-$TRS_{22N}$-$ORF6_{(C306)}$) passaged 20 times in tissue culture (including the cloning steps) express PRRSV Gp5. This again shows the improved stability *in vitro* of the recombinant rTGEV expressing PRRSV Gp5 and M proteins.

Figure 7 shows the results on an RT-PCR analysis of the stability of the recombinant TGEV expressing ORF5 and ORF6 (M) after being passaged in ST cells. The recombinant TGEV was passaged 20 times in tissue culture. Virus recovered from the culture cells was plaque cloned and mRNA expression for six clones (c1 to c6) was determined by RT-PCR. As shown in the Figure, 100% of the clones expressed mRNA-ORF5 and mRNA-ORF6. This shows that the genome of the recombinant TGEV expressing ORF5 and ORF6 remains stable after 20 passages in cell culture.

Figure 8 shows the results of a Western blot analysis for the detection of expression of PRRSV Gp5 protein (ORF5; left blot) and M protein (ORF6; right blot) in cell lysates of swine testis cells infected with the recombinant virus rTGEV-$S_{PTV}$-$TRS_{3a}$-ORF5-$TRS_{22N}$-$ORF6_{(C306T)}$ (abbreviated as rTGEV-$S_{PTV}$-ORFs5+6 in the

Figure) or a control virus (rTGEV-$S_{PTV}$-FL). Further, several controls are used in the Western blot ("mock": swine testis cells without viral infection; "PRRSV": purified and concentrated PRRS virus wild type; "MA104+PRRSV": African green monkey kidney cell line infected with PRRS virus wild type). The respective protein could be detected in lysates obtained from cells infected with the recombinant virus rTGEV-$S_{PTV}$-$TRS_{3a}$-ORF5-$TRS_{22N}$-$ORF6_{(C306)}$, cells infected with the PRRSV virus and cells infected with MA104+PRSSV) as indicated by the arrows.

Figure 9 shows the results of an analysis of the growth of recombinant TGEV expressing PRRSV Gp5 and M after *in vivo* inoculation. The virus used for this analysis contains the S gene as set forth in SEQ ID NO: 1 providing enteric and respiratory tropism. The Figure shows the growth kinetics of virus in ST cells after recovery from lung (circular symbol) and gut (square symbol) of vaccinated piglets. Tissue samples were collected 1, 2, 3 and 4 days after inoculation with recombinant TGEV expressing PRRSV Gp5 and M proteins.

Figure 10 shows the results of a further analysis of the stability of recombinant TGEV expressing PRRSV Gp5 and M (pBAC-TGEV$^{FL}$-$S_{PTV}$-$TRS_{3a}$-ORF5-$TRS_{22N}$-$ORF6_{(C306T)}$) after *in vivo* administration of the virus recovered from the pigs. Antibodies were used in an immunofluorescence assay to detect PRRSV Gp5 (green colour in the upper panel), PRRSV M (green color in the bottom panel) and TGEV N (red colour) positive ST cells infected with virus recovered from lung of vaccinated piglets. The results show that the recombinant rTGEV expressing PRRSV Gp5 and M proteins is stable even after *in vivo* inoculation and recovery from vaccinated animals. The data indicate that 80% of the infected cells expresses Gp5 protein, and 100% of the infected cells also expresses M protein.

Figure 11 shows the scheme followed for setting up two vaccine formulations using the recombinant virus rTGEV-$S_{PTV}$-$TRS_{3a}$-ORF5-$TRS_{22N}$-$ORF6_{(C306T)}$.

Figure 12 shows a table representing the results of an evaluation of the replication and propagation of the recombinant virus rTGEV-$S_{PTV}$-$TRS_{3a}$-ORF5-$TRS_{22N}$-$ORF6_{(C306T)}$ in target tissues (lung) of two days old piglets inoculated with $2 \times 10^7$ pfu/ml via the intranasal route. The results were obtained by virus titration and histopathology.

Figure 13 shows examples of immunohistochemistry performed on sections of lung tissue obtained from pigs of group #1 and #2, respectively, showing interstitial pneumonia characteristic of coronavirus infection. The sections are characterized by thickening of alveolar walls due to lymphocyte and macrophage infiltration and alveolar spaces filled with mononuclear infiltration (lymphocytes and macrophages) and occasionally with polymorphonuclear neutrophiles.

Figure 14 shows the results of competition ELISA used for detecting specific anti-gp5 antibodies in animals vaccinated with modified live vaccine compared to non-vaccinated animals.

Absorbance at D0 PV was used to calculate the percentage of binding in each sera using a specific calculation. Lower binding percentages represent higher levels of anti-gp5 antibodies in the tested sera. The differences in the absorbance values and the % binding therefrom between animals from the vaccinated group and animals of the non-vaccinated group are statistically relevant (t test: p value $< 0.05$).

Figure 15 shows the results of as specific immunoperoxidase monolayer assay (IPMA) performed for the detection of PRRSV in porcine alveolar lung macrophages infected with the serum samples obtained from vaccinated and control pigs.

Figure 16 shows results of the detection of anti-TGEV (picture A) and anti-PRSSV antigens (picture B) by indirect ELISA displayed as absorbance of the chromogenic substrate. "Vacc." designates sera from vaccinated animals, "Ctrl." designates sera from non-vaccinated control animals, "CP" and "CN" designate positive and negative controls, respectively. The sera from vaccinated and control animals were diluted 60-fold.

Figure 17 reflects the percentage of animals having a positive immunological response against TGEV and PRSSV using a 1:60 dilution. Panel A is a table summarizing the results, whereas the graphs show the percentages of positive animals in graphical form (graph B for anti-TGEV and graph C for anti-PRRSV). "Vacc." designates sera from vaccinated animals, "Ctrl." designates sera from non-vaccinated control animals.

Figure 18 shows the results of competition ELISA used for detecting specific anti-gp5 antibodies in animals vaccinated with inactivated vaccine compared to non-vaccinated animals. Absorbance at D0 PV was used to calculate the percentage of binding in each sera using a specific calculation. Lower binding percentages represent higher levels of anti-gp5 antibodies in the tested sera. The differences in the absorbance values and the % binding therefrom between animals from the vaccinated group and animals of the non-vaccinated group are statistically relevant (t test: p value $< 0.05$).

Figure 19 shows the results of a quantitative real-time RT-PCR (qRT-PCR) used for quantification of PRRSV titers in serum samples obtained from vaccinated and non-vaccinated animals.

Figure 20 shows the schematic structure of the cDNA encoding a ORF5-LIMP-II fusion protein and its effect on antigen presentation by the infected cell.

Figure 21 shows the schematic structure of the cDNA encoding an Ub-ORF5 fusion protein and its effect on antigen presentation by the infected cell.

Figure 22 shows the construction of recombinant TGEVs with chimeric S proteins. The Figure shows the differences between the 5' sequence regions of the S genes between the parental TGEV isolates TGEV-PUR46-PTV and TGEV-PUR46-C11 (see upper box in the left side). The nucleotides highlighted in the Figure reflect the differences between these two sequences, while the light vertical box represents a deletion of three nucleotides. The positions of the nucleotides are indicated by the numbers on top of the Figures. The Figure further shows the sequence of several S gene mutants compared to that of the parental TGEV isolate TGEV-PUR46-PTV. The titer of each recombinant virus in vivo in the gut and in the lung of infected newborn piglets is also indicated in the Figure.

Figure 23 shows the growth kinetics of the parental TGEVs and the recombinant TGEV-PUR46-S7.1. Growth of these viruses was determined after inoculation of three day old newborn piglets with $3 \times 10^8$ pfu per piglet. Animals were sacrificed at the indicated days, and virus titers per gram of tissue were determined by plaque titration on ST cells. (■), Virus in the lungs; (●), virus in the gut. The indicated values correspond to medium values of three independent experiments.

The following examples illustrate the construction and use of the nucleic acids according to the invention.

## EXAMPLE 1

### Growth of Eukaryotic Cells

TGEV growth, titration, and purification were performed in ST (swine testicle) cells, a cell line obtained from epithelial cells of fetal pig testicles (MCCLURKIN and NORMAN, 1966). ST cells were obtained from L. KEMENY (National Animal Disease Centre, Ames, Iowa, USA).

Plasmid transfections assays were performed in Baby Hamster Kidney cells (BHK-21) stably transformed with the gene coding for the porcine aminopeptidase N (BHK-pAPN) (LAUDE *et al*., 1990). ST cells were cultivated in DMEM (Dulbecco's Modified Eagle Medium) supplemented with 10% fetal calf serum (FCS) (GIBCO-BRL), 50 mg/mL gentamicine, 2 mM glutamine, and 1% non-essential amino acids.

The BHK-21 stably transformed with the gene encoding for the porcine aminopeptidase N (BHK-pAPN) were grown in DMEM (Dulbecco's Modified Eagle Medium) supplemented with 2% fetal calf serum (FCS) (GIBCO-BRL), 50 mg/mL gentamicine, 2 mM glutamine, and 1% non-essential amino acids and Geneticine (G418) (1.5 mg/ml) as a selection agent.

## EXAMPLE 2

### Transformation of bacteria by plasmid electroporation

Bacterial strains:

*Escherichia coli* DH10B (Gibco/BRL) (HANAHAN *et al*., 1991) was the host for all the plasmids constructed. The genotype of this bacterial strain is: $F^-$mcr A Δ (mrr-hsdRMS-mcrBC) ϕ80d*lac*ZΔM15 Δ*lac*X74 *deo*R *rec*A1 *end*A1 *ara*D139 (*ara*, *leu*) 7697 *gal*U *galK* $\lambda^-$ *rsp*L *nup*G.

Preparation of electroporation-competent bacteria:

For amplification and production of electroporation-competent *E. coli* DH10B bacteria, the bacteria were grown in a SOB medium. 10 mL of SOB medium (20 g/L tryptone, 5 g/L yeast extract, 0.5 g/L NaCl) were inoculated with a colony from a fresh plate and were incubated for 12 h at 37°C under agitation. With 2 mL of this culture, 1 L of SOB medium was inoculated, and the culture was grown at 37°C to an optical density of 600 nm between 0.8 and 0.9 absorbance units. Then the culture was cooled on ice for 20 min, and the bacteria were centrifuged in the Sorvall GSA rotor at 4.000 × g for 15 min at 4°C. The bacteria were resuspended in 1 L of cold 10% glycerol. The bacteria suspension was centrifuged again and resuspended in 500 mL of cold 10% glycerol. The bacteria were sedimented and resuspended in 250 mL of cold 10% glycerol. Finally, the bacteria were centrifuged and resuspended in 3 mL of 10% glycerol. The final suspension was divided into aliquots of 50 μL and 100 μL and were kept at -70°C until they were used for electroporation. The transformation efficiency of the bacteria was calculated by electroporation with a known concentration of a pBluescript plasmid as a reference, and was found to be reproducibly at about $10^9$ colonies/μg of DNA.

Transformation of bacteria by plasmid electroporation:

50 μL of transformation-competent bacteria were mixed witch 1 μL of each reaction mixture, or 10 ng of purified plasmid were added to the bacteria and were incubated on ice for 1 min. Then, the mixture was transferred to 0.2 cm electroporation trays (Bio=Rad) and were transformed by a 2.5 kV electric pulse, 25 μF and 200 Ω in a "Gene Pulser" electroporator (Bio-Rad). After adding 1 mL of cold LB medium, the bacteria were incubated at 37°C under agitation for 1 h. Between 50 and 100 μL of the suspension of transformed bacteria were seeded in Petri dishes with LB (Luria-Bertani Medium) in a solid medium (15 g/L agar) supplemented with ampicillin (100 μg/mL) or chloramphenicol (34 μg/mL) The' bacteria grew for 16 h at 37°C (BULLOCK *et al*., 1987).

For production and purification of plasmids, the bacteria transformed with plasmids that conferred ampicillin or chloramphenicol resistance were grown from an isolated colony on a plate in liquid LB medium supplemented with 100 μg/mL of ampicillin or 34 μg/mL of chloramphenicol.

**EXAMPLE 3**

**Plasmids for cloning of PCR products**

The pGEM-T (Promega) plasmid was used to clone PCR products. This plasmid contains the T7 and SP6 bacteriophage promoters separated by the LacZ gene, interrupted by two protuberant T sequences between a multicloning sequences. This plasmid confers ampicillin resistance for its selection.

**EXAMPLE 4**

**Manipulation of DNA**

Cloning and restriction enzymes:

For the manipulation and cloning of DNA, the restriction enzymes BamHI, *Bbs* I, *Blp* I, *Eco* RI, *Mlu* I, *Swa* I, *Xcm* I, *Xho* I were acquired from Roche or from New England Biolabs. Dephosphorylation of the DNA termini was done with shrimp alkaline phosphatase (SAP) (USB). A DNA ligase such as T4 phage DNA ligase (New England Biolabs) was used. All the treatments with restriction enzymes, dephosphorylation, and DNA ligation were carried out using standard protocols previously described (SAMBROOK *et al*., 1989).

Polymerase chain reaction (PCR):

To amplify DNA from a template, frequently plasmids, 50-100 ng of DNA plasmid was mixed with the corresponding oligonucleotides (10 μM), 0.25 mM deoxynucleotide triphosphates (ATP, GTP, TTP, and CTP), 1.25 mM $MgCl_2$, PCR buffer (10 mM Tris-HCl, pH 8.3, 50 mM KCl) and 2.5 U of Taq Gold DNA polymerase (*Thermus aquaticus)* (Roche) in a final volume of 50 μL. The reactions were carried out in the GeneAmp PCR System 9600 thermocycler from Perkin Elmer.

Separation of DNA by agarose gel electrophoresis:

To separate DNA fragments, 1% agarose gels were used with ethidium bromide (1 μg/mL) in a 1X TAE buffer (40 mM Tris-acetate, 1mM EDTA).

Purification of DNA:

The bacterial plasmids grown in the presence of the selection antibiotics were purified using either the "Qiaprep Spin Miniprep kit" (Qiagen) to prepare small quantities of plasmid DNA, or the "Qiafilter Midi-Plasmid Kit" system (Qiagen) to prepare medium quantities of plasmid DNA. The DNA obtained from agarose gels was purified using the "QiaEx II Gel Extraction Kit" system (Qiagen). Purification of the PCR products was carried out by means of the system "QIA quick PCR Purification Kit" (Qiagen). In all cases, the manufacturer's instructions were followed.

**EXAMPLE 5**

**RNA analysis**

For analysis of the RNA produced in infections with TGEV clone PUR46-MAD, confluent monolayers of ST (swine testis) cells grown in 60 mm diameter culture plates (NUNC) were infected with viral inocula at a MOI [multiplicity of infection] of 1. The cells were lysed at 16 hpi [hours post infection] using a "RNeasy Mini Kit" (Qiagen), following the protocol provided by the manufacturer (Qiagen). The RNA was purified and resuspended in 40 μL of water treated with DEPC [diethyl pyrocarbonate] and 20 U of RNAse inhibitor (Roche).

**EXAMPLE 6**

**Transfection and Recovery of infectious TGEV from cDNA Clones**

BHK-pAPN cells (DELMAS, 1994) were grown in Dulbecco's modified Eagle medium (DMEM) supplemented with 2% fetal calf serum (FCS) and containing Geneticin (G418) (1.5 mg/ml) as a selection agent. BHK-pAPN cells were grown to 60% confluence in 35-mm-diameter plates and transfected with 10μg or 4μg of pBAC-

$TGEV^{FL}$-$S_{PTV}$-$TRS_{3a}$-ORF5-$TRS_{22N}$-$ORF6_{(C306T)}$ plasmid with 15μl or 12μl of lipofectin (GIBCO Life Technologies) or lipofectamine 2000 (Invitrogen) according to the manufacturer's specifications. The cells were incubated at 37°C, 5% $CO_2$ and after 6 h the transfection medium was replaced with fresh DMEM containing 5% (vol/vol) FCS. Two days later (referred to as passage 0), the cells supernatants were harvested and passaged four times on fresh ST monolayer to increase rTGEV titer. Virus titers were determined by plaque titration.

Alternatively, ST cells were grown in 25 $cm^2$ culture flasks using DMEM (Dulbecco's Minimal Essential Medium), 10% FCS at 90% of confluence and infected at a MOI [multiplicity of infection] of 1 plaque forming units [pfu] per cell. The supernatant was recovered after 48 hours and titrated by plaque limit dilution.

Plaque titration:

Titration of viral stocks was made by plaque limit dilution assay on ST cells to quantify the number of infective particles. ST cells were grown in 24-multiwell culture plate at 90 % of confluence. Recombinant TGEV viruses were serially diluted 10-fold (10E-1, 10E-2, 10E-3, 10E-4, 10E-5, 10E-6, etc.). The different virus dilutions were added to each well of the 24-well plate and incubated for 1 hour at 37°C, 5% $CO_2$. After that hour the supernatant containing the virus was removed from the ST monolayer and quickly an overlay AGAR was added onto the monolayer. The overlay AGAR was prepared using 1 part of 2X DMEM (Dulbecco's Minimal Essential Medium) and 1 part of 1% purified AGAR in $ddH_2O$. After overlaying the cells the multi-well plate was kept for 15 minutes at room temperature to solidify the agarose and was then placed in a controlled incubator for 48 hours at 37°C, 5% $CO_2$.

In order to count the viral plaques, the infected ST cell monolayers were fixed with 10% formol and stained with crystal violet, 0.1%, for 30 minutes. The well was washed with distilled water and dried at room temperature before finally counting the plaques to determine the virus titer.

TGEV and PRRSV protein expression were analyzed by standard immunofluorescence techniques.

**EXAMPLE 7**

**Generation of rTGEV**

The porcine transmissible gastroenteritis virus (TGEV) used here belongs to the group of Purdue isolates and was obtained in Indiana in 1946 (DOYLE and HUTCHINGS, 1946). The virus was adapted to grow in cell cultures (HAELTERMAN and PENSAERT, 1967) and was provided by E.H. BoHL (Ohio State University, Wooster Ohio). This TGEV isolate has been passaged in ST cells 115 times, and has been cloned five times consecutively in Dr. Luis Enjuanes laboratory (Centro Nacional de Biotecnologia, Madrid, Spain). The clone selected was labeled PUR46-CC120-MAD, abbreviated PUR46-MAD. It is an attenuated virus that grows well in cell cultures, and reaches titers between $10^8$ and $10^9$ PFU/mL.

rTGEV viruses were generated from pBAC-TGEV constructs containing the S gene from the virulent TGEV strain PUR-C11 ($S_{c11}$) as described (ALAMAZAN *et al.,* 2000; GONZALEZ *et al.*, 2002). Viruses containing the S gene (encoding the TGEV spike protein) from the attenuated strain PTV ($S_{PTV}$) were derived from the corresponding pBAC-TGEV vectors carrying $S_{c11}$ by replacing this gene by $S_{PTV}$ from the respiratory strain.

**EXAMPLE 8**

**Construction of a recombinant TGEV vector expressing Gp5 and M protein of PRRSV**

In order to increase the cloning capacity of the TGEV single genome, the non-essential genes ORF3a and ORF3b were eliminated from the full-length cDNA clone, creating a deletion in the TGEV genome. The heterologous genes ORF5 and ORF6 encoding PRRSV proteins Gp5 and M were inserted into the cDNA construct replacing the deleted TGEV ORFs 3a and 3b.

ORF5 (606nt) (SEQ ID NO: 2) was cloned into cDNA constructs replacing the TGEV genes 3a and 3b. The gene was amplified by PCR using oligonucleotides PpuMI-ORF5 VS (5' -AACAGGTCCTACCA-TGAGATGTTCTCACAAATTGGGG-3') (SEQ ID NO: 4) and Blp-ORF5 RS mut (5' -CCGCTAAGCCTAGGCTTCCCATTGCTCAGCCGAAGTCC-3') (SEQ ID NO: 5). The PCR product was digested with PpuMI and BlpI and cloned into the same restriction sites of plasmid pSL-TGEV-AvrII, which comprises nt 22965 to 25865 from the TGEV genome including the 3a and 3b deletion, generating plasmid pSL-AvrII-Δ3-PpuMI-ORF5. To obtain the infectious cDNAs, this plasmid was digested with AvrII and the insert containing ORF5 was cloned into the same restriction site of pBAC-$S_{c11}$ or pBAC-$S_{PTV}$(PacI/MluI) to obtain vectors with enteric and respiratory tropism, respectively. The sequences of pBAC plasmids and of the infectious TGEV cDNA clone have been previously reported (WO01/39797).

ORF6 from PRRSV Olot91 strain (SEQ ID NO: 3) was amplified by overlapping PCR to introduce a silent mutation eliminating an AvrII restriction site due to cloning restrictions, taken into account the *Sus scrofa* codon usage. In a first PCR, oligonucleotides BlpIORF6 VS (5'-CGGCTGAGCAATGGGAAGCCTAGAAAATTAT-TACATATGGTATAACTAAACAAAATGGGAAGCCTAGACGATTTTTG-3') (SEQ ID NO:6), underlined sequence being the $TRS_{22N}$, and ORF6-C306T-RS (5'-GCCGGCCTAGACAACACAATC-3') (SEQ ID NO: 7) were used. Using a similar procedure, in a second PCR oligonucleotides ORF6-C306T-VS -(5'-GATTGTGTTGTCTAGGCCGGC-3') (SEQ ID NO: 8) and BlpIORF6rs new (5'-GCTAAGCTTACCGGCCATACTTGACGAGG-3') (SEQ ID NO: 9) were used. Using these PCR products and oligonucleotides BlpIORF6 VS and BlpIORF6rs new, the final product (574 nt) was obtained. This PCR product was digested with BlpI and cloned into the same restriction site of pSL-AvrII-Δ3-PpuMI-ORF5, generating plasmid pSL-$TRS_{3a}$-ORF5-$TRS_{22N}$-$ORF6_{(c306T)}$. To obtain the infectious cDNA pBAC-$S_{PTV}$-$TRS_{3a}$-ORF5-$TRS_{22N}$-$ORF6_{(C306T)}$, plasmid pSL-$TRS_{3a}$-ORF5-$TRS_{22N}$-ORF6($_{c306T}$) was digested with AvrII and the insert containing ORF5 and ORF6 was cloned into the same restriction sites of pBAC-$S_{PTV}$(PacI/MluI) (Fig. 4). The same cloning procedure was used to obtain the cDNA encoding the TGEV with enteric tropism (adapted to grow in cell culture) expressing PRRSV Gp5 and M.

The recombinant virus with respiratory tropism, rTGEV-$S_{PTV}$-$TRS_{3a}$-ORF5-$TRS_{22N}$-$ORF6_{(C306T)}$, was rescued and plaque cloned three times. The presence of the mRNAs for ORF5 and ORF6 was confirmed by RT-PCR. One expressing clone was selected and, after two passages in cell culture, mRNA of ORF5 and ORF6, respectively, were detected, indicating that the virus was stable. Nevertheless, to improve expression levels by adapting the recombinant virus to grow in ST cells, two more plaque cloning steps were performed. Expression of Gp5 by the viral clones was evaluated by immunofluorescence (see Figure 5) and the amount of infected cells expressing Gp5 was quantified. The selected virus (rTGEV-$S_{PTV}$-$TRS_{3a}$-$ORF_5$-$TRS_{22N}$-$ORF6_{(c306T)}$) expressed high amounts of Gp5 in the 80 % of the infected swine testis (ST) cells as evaluated by fluorescence-activated cell sorting (FACS).

**EXAMPLE 9**

***In vitro* studies for characterizing TGEV virus rTGEV-$S_{PTV}$-$TRS_{3a}$-ORF5-$TRS_{22N}$-$ORF6_{(C306T)}$**

For characterizing the recombinant TGEV virus rTGEV-$S_{PTV}$-$TRS_{3a}$-ORF5-$TRS_{22N}$-$ORF6_{(C306T)}$ immunofluorescence assays and Western Blot analysis were performed in order to detect the expression of the different PRRSV proteins gp5 and M by the viral vector.

Immunofluorescence assays

ST cells were grown to 30% confluence in 8 or 12 well chambers. The cells were infected at a MOI of 5 pfu/cell at 37°C in MEME (Minimum Essential Medium Earle's) containing 2% Fetal Clone III serum (purchased from Hyclone). -8 hour post infection the inoculum was removed and the cells were then washed with PBS and fixed by addition of 4% paraformaldehyde for 30 min at RT. For dual-labeling in witch one primary antibody was derived from mouse and the other from rabbit, the primary antibodies were combined in a diluent containing PBS-SFB 20%/0.2% saponin. The antibodies were allowed to adsorb for 90 min. at RT and the cells were then washed three times with PBS. Afterwards, the cells were incubated for 30 min at room temperature with a 1:1000 dilution of anti-rabbit and anti-mouse secondary antibodies conjugated to rhodamine and flouresceine. The chambers of the plate were washed five times with PBS, mounted and analyzed by fluorescence microscopy.

Western Blot analysis

ST cells were grown to 100% confluence in 12.5 $cm^2$ tissue culture flasks. The cells were infected at a MOI of 5 at 37°C in MEME (Minimum Essential Medium Earle's) containing 2% Fetal Clone III serum (Hyclone) for 8 hours. Cells were disrupted with 1 × sample loading buffer. Cell lysates were analyzed by 12.5% gradient sodium dodecyl sulphate-polyacrylamide gel electrophoresis (SDS-PAGE). Separated proteins were transferred onto a PDVF membrane using 0.02 % SDS (100 V; Amp limit 0.30; Time 1:30 hours). After transfer the PDVF membranes were blocked with PBS:milk 5% for 2 hours at RT. and were then incubated over night at 4°C with a 1:100 dilution of polyclonal antibody specific for Gp5 PRRSV protein (8676) or a 1:50 dilution of a polyclonal antibody specific for M PRRSV protein (7718). After this incubation the PDVF membranes were washed three times with PBS:milk 5%:0.05 Tween-20. The PDVF membranes were then incubated with goat-anti-rabbit antibody conjugated with peroxidase for 1 hour and washed afterwards five times with PBS:milk 5%:0.05 Tween-20. Bound antibodies were detected with Inmobilon Western Chemiluminescent HRP substrate (Millipore). The results are shown in Figure 8.

PRRSV gp5 and M proteins were detected in lysates obtained from ST cells infected with the recombinant virus or MA104 cells infected with PRRS wild type virus. The MA104 cells were also used as control, because this system is more related to the model of infection in cell culture, i.e. ST cells infected with rTGEV-ORF5-ORF6 virus.

The recombinant TGEV virus rTGEV-$S_{PTV}$-$TRS_{3a}$-ORF5-$TRS_{22N}$-ORF6 ($_{C306T}$) is stable after being passaged 20 times in tissue culture and also after rescue from infected piglets (see Figures 5 and 6). After 20 passages in tissue culture the virus still expresses the PRRSV ORF5 and ORF6 genes (see Figure 7).

## EXAMPLE 10

### Production of a modified live vaccine (MLV)

Starting from a Master Seed Virus of rTGEV-$S_{PTV}$-$TRS_{3a}$-ORF5-$TRS_{22N}$-ORF6 the modified live vaccine containing the supernatant of swine testis (ST) cells infected with the recombinant rTGEV-$S_{PTV}$-$TRS_{3a}$-ORF5-$TRS_{22N}$-ORF6 expressing the recombinant PRRSV proteins gp5 and M was produced as follows (see also Figure 11):

The rTGEV-$S_{PTV}$-$TRS_{3a}$-ORF5-$TRS_{22N}$-ORF6 virus was obtained from the infection of ten 100 $cm^2$ culture dishes of ST cells with Master Seed Virus at a high MOI [multiplicity of infection] of 1. The supernatants were recovered, centrifuged and frozen at -80°C ($\pm$10*C). The recombinant virus obtained was titrated. The obtained recombinant virus from the supernatants have been diluted 1:10 with DMEM culture medium to obtain a final dose of ~ $1 \times 10^7$ PFU/ml.

This vaccine formulation designated MLV has been evaluated in further *in vivo* experiments (see Examples 12 and 13).

## EXAMPLE 11

### Production of an inactivated (killed) vaccine

The inactivated vaccine containing extracts of lysed swine testis (ST) cells infected with the recombinant rTGEV-$S_{PTV}$-$TRS_{3a}$-ORF5-$TRS_{22N}$-ORF6 expressing the recombinant PRRSV proteins gp5 and M was produced as follows (see also Figure 11):

The rTGEV-$S_{PTV}$-$TRS_{3a}$-ORF5-$TRS_{22N}$-ORF6 antigen was obtained from the infection of ten 100 $cm^2$ culture dishes of ST cells with Master Seed Virus at a high MOI [multiplicity of infection] of 3. Cells were recovered, washed with sodium phosphate buffer (PBS) and sedimented. The cellular pellet was frozen at -80°C ($\pm$10°C). Considering that approximately 70% of the infected ST cells are producing gp5 and M recombinant proteins, the pellet was resuspended in sodium bicarbonate, pH 8.3, at a concentration of $3.9 \times 10^6$ infected producing cells/mL. Cells were lysed and the cellular extract was centrifuged 30 min at 15000 $\times$ g and 4°C.

Supernatants were inactivated by binary ethylenimine (BEI) at a final concentration of 5% during 72 h with continuous stirring at 37°C.

Inactivated antigen was diluted 1:3 with porcine circovirus (PCV) Type1-Type2 inactivated vaccine (FENAUX *et al.*, 2003; FENAUX *et al.*, 2004) previously adjuvanted with 20% sulfolipo-cyclodextrin (SLCD; obtained from Fort Dodge, Iowa, USA) as follows: 32 mL ST-rTGEV-$S_{PTV}$-$TRS_{3a}$-ORF5-$TRS_{22N}$-ORF6 inactivated antigen with a $3.7 \times 10^6$ infected producing cells/mL were mixed with 64 mL of PCV Type1-Type2 vaccine. The mixture was stirred for 3.25 h at RT.

This vaccine formulation (inactivated) has been evaluated in an *in vivo* experiment (see Example 14).

## EXAMPLE 12

### In vivo pathogenicity evaluation in piglets of the recombinant virus rTGEV-$S_{PTV}$-$TRS_{3a}$-ORF5-$TRS_{22N}$-ORF6 expressing two heterologous PRRSV proteins

This evaluation was performed to evaluate the replication and propagation of rTGEV-$S_{PTV}$-$TRS_{3a}$-ORF5-$TRS_{22N}$-ORF6 in target tissues (gut and lung) *in vivo* by virus titration and by immunohistochemistry as well as the expression of the heterologous PRRSV proteins (Gp5 and M) and the TGEV genome in these tissues was evaluated by immunohistochemistry. The evaluation was further performed to confirm that rTGEV-$S_{PTV}$-$TRS_{3a}$-ORF5-$TRS_{22N}$-ORF6 replicates in lungs of two days old piglets inoculated with $2 \times 10^7$ pfu/ml via the intranasal route. The virus titers in the lung and histopathological lesions are also evaluated.

17 two-days-old piglets, hybrids Large White x Belgian Land-race, free of antibodies against TGEV and PRRSV, were selected and divided into three groups. Pigs of group #1 (7 animals) were inoculated with $2 \times 10^7$ pfu/ml of the control virus rTGEV$S_{PTV}$-FL, pigs of group #2 (7 animals) were inoculated with $2 \times 10^7$ pfu/ml of the recombinant virus rTGEV-$S_{PTV}$-$TRS_{3a}$-ORF5-$TRS_{22N}$-ORF6, whereas pigs of group #3 (3 animals) represent the control group.

Following the inoculation on day 0, animals from each group were subjected to slaughter and necropsy as shown in Table 1.

**Table 1**

| Group | Number of pigs | D0 + 1d | D0 + 2d | D0 + 3d | D0 + 4d |
|---|---|---|---|---|---|
| # 1 | 7 | 1 | 2 | 2 | 2 |
| # 2 | 7 | 1 | 2 | 2 | 2 |
| # 3 | 3 | -- | -- | -- | 3 |

On days 1, 2, 3, and 4 post inoculation pigs from groups #1 and #2 have been slaughtered, whereas pigs from group #3 have been slaughtered only at day 4 post inoculation.

After necropsy lung sections were examined macroscopically. The type and extent of the lung lesions were described and evaluated following the scoring system described by HANNAN *et al*., 1982. The so-called pneumonic score of the lung lesions is presented in Figure 12.

Tissue samples from the lungs of these animals were homogenized with PBS in order to determine the recombinant virus titer in the target tissue (lung). The viruses recovered from the lung tissues were titrated in ST cell monolayers as plaque forming units (pfu/ml).

Tissue samples were also placed in formaldehyde (10% buffered-formalin) for histopathological studies. Tissue samples of 2-3 mm were allocated in plastic cassettes, dehydrated in graded alcohol series and paraffin-embedded using an automatic tissue processor system (Cytadel). Tissue blocks were prepared and 4-5 $\mu$m sections were cut from these blocks using an automatic microtome (Finesse). The sections were stained with hematoxylin-eosin using an automatic stainer (Linistain GLX).

Additional paraffin-embedded sections were taken in silanized slides and were prepared for a immunohistochemistry (THC) technique for detecting TGEV and PRRSV antigens expressed by the recombinant virus. The sections were heated to 60°C for 10 minutes until the paraffin melts. The samples were then immersed twice in xylol for 10 minutes and twice in absolute ethanol for 10 minutes. After immersing the samples in a solution for endogenous peroxidase inhibition (methanol + 3% (v/v) hydrogen peroxide ($H_2O_2$) ) for 30 minutes in the dark, the samples were washed three times in Tris-Saline Buffer (TBS) (0.05 M), before immersing the samples for 15 minutes in a sodium citrate solution previously heated in the microwave, followed by washing the samples three times in Tris-Saline Buffer (TBS) (0.05 M). Afterwards, the samples were immersed in TBS (0.05 M)-BSA solution for 10 minutes to block unspecific binding. Before adding the primary antibody, two drops of Avidin Solution (Vector Laboratories, Blocking Kit) were added to the samples followed by incubation for 15 minutes, followed by addition of two drops of Biotin Solution (Vector Laboratories, Blocking Kit) and incubation for 15 minutes. The samples were then immersed in the primary antibody solution 3BB3 monoclonal antibody against TGEV M protein (dilution 1/100) and were incubated over night at 4°C. The samples were washed three times in Tris-Saline Buffer (TBS) (0.05 M) and then immersed for 1 hour in the secondary antibody solution anti-mouse IgG (Vector Laboratories, Vectastain ABC Kit) (dilution 1/100). After washing the samples three times in Tris-Saline Buffer (TBS) (0.05 M), they were immersed in Avidin-Biotin-Peroxidase solution (Vector Laboratories, Vectastain ABC Kit) for 1 hour, again followed by 3 washes in Tris-Saline Buffer (TBS) (0.05 M). After immersing the samples in DAB (3,3'-diaminobenzidine, SIGMA) solution for 7 minutes, washing the samples three times in Tris-Saline Buffer(TBS) (0.05 M), they were immersed in distilled $H_2O$ for 10 minutes. The samples were then stained with Hematoxylin for 1-2 minutes, immersed in distilled $H_2O$ for 10 minutes, immersed in ethanol 96% for 5 minutes, immersed in ethanol 100% for 5 minutes and finally immersed in xylol for 5 minutes. Prior to evaluating the samples with an optic microscope, the samples were prepared with hydrophobic mounting medium and cover glasses.

Detection of S protein of the TGEV vector in the samples from pigs of groups #1 and #2 as confirmed by immunohistochemistry shows that the recombinant virus replicates in the lung of pigs after vaccination via the intranasal route (see Figure 12). Virus titers detected in the lung samples and histopathological lesions found in tissue sections confirm these results. The interstitial pneumonia detected by histopathological examination could be associated to the viral infection and is a characteristic lesion of a coronavirus infection (examples of immunohistochemistry of tissue sections of one pig of group #1 and #2 are shown in Figure 13).

The simultaneous detection of TGEV antigen by immunohistochemistry and high titers of recombinant virus by virus titration confirms the replication of the recombinant virus in the lung.

Further, due to the improved stability of the recombinant TGEV virus could be recovered from lung and gut tissues of vaccinated piglets. The virus rescued from these tissues could be further propagated in cultures of ST cells (see Figure 9) and the ST cells infected with the rescued virus expresses both Gp5 (about 80 % of the infected cells) and M (about 100% of the infected cells; see Figure 10).

**EXAMPLE 13**

**Evaluation of the efficacy of the rTGEV-$S_{PTV}$-$TRS_{3a}$-ORF5-$TRS_{22N}$-ORF6 modified live vaccine in pigs against the challenge with the field isolate PRRSV strain Olot/91**

This evaluation was performed to evaluate the efficacy of the rTGEV-$S_{PTV}$-$TRS_{3a}$-ORF5-$TRS_{22N}$-ORF6 expressing different heterologous PRRSV proteins as modified live vaccine (MLV) in the prevention of reproductive and respiratory disease in one-week-old piglets inoculated via the intranasal route and then challenged with the field isolate PRRSV strain Olot/91. The efficacy of the vaccine was evaluated by the production of antibodies in serum (assessed by ELISA, IPMA, and serum neutralization).

39 one-week-old piglets, seronegative or with low antibody titers with regard to TGEV and PRRSV, were selected and divided into three groups. Pigs of group #1 (17 animals) were vaccinated with the control virus rTGEV-$S_{PTV}$-FL, pigs of group #2 (17 animals) were vaccinated with the recombinant virus rTGEV-$S_{PTV}$-$TRS_{3a}$-ORF5-$TRS_{22N}$-ORF6, whereas pigs of group #3 (5 animals) represent the control group.

The vaccination of the pigs was performed following the experimental design shown in Table 2 with "$D_0$" representing the first vaccination day of one-week-old pigs.

**Table 2**

| Group | Number of pigs | 1st vaccination ($D_0$) | 2nd vaccination ($D_0$+3w) | Challenge ($D_0$+6w) |
|---|---|---|---|---|
| # 1 | 15 | rTGEV-$S_{PTV}$-FL | rTGEV-$S_{PTV}$ -FL | PRRSV |
| # 2 | 15 | rTGEV-$S_{PTV}$-$TRS_{3a}$-ORF5-$TRS_{22N}$-ORF6 | rTGEV-$Sp_{TV}$-$TRS_{3a}$-ORF5-$TRS_{22N}$-ORF6 | PRRSV |
| # 3 | 5 | --- | --- | --- |

Pigs are vaccinated with 0.5 ml of viral suspension in each nostril for a total volume of 1 ml/piglet in each vaccination via the intranasal route twice, with the first vaccination taking place at one week of age and the second vaccination (revaccination) taking place at 4 weeks of age. The intranasal route was chosen in order to enhance replication and propagation in the target tissues (lung) of the recombinant virus in the inoculated pigs. Pigs were randomly assigned to each treatment group. All pigs were challenged ten weeks after the first vaccination with the pigs being 11 weeks of age at the time of challenge. The pigs were inoculated with PRRSV Spanish strain (Olot/91) at $10^{4.7}$-$10^{4.8}$ $TCID_{50}$/pig via the intranasal route. The inoculation was carried out in standing position without sedation and using a 10 mL syringe. The inoculum was divided into two equal parts, one for each nostril. The intranasal route was selected, because it is considered to be the natural route of infection.

After vaccination and after challenge, the pigs were observed daily for clinical signs to check the safety of the vaccine. Upon observation of any anomaly, a complete clinical examination was conducted by the principal investigator.

Blood samples were taken at D0, D21, and D28 PI in tubes to obtain serum for the determination of serological titers and to study the immune response against TGEV and PRRSV.

The effect of the PRRSV challenge was evaluated by measuring the presence of specific anti-gp5 antibodies using the competition ELISA INGEZIM PRRS gp5 Compac (INGENASA). Using a chromogenic substrate, the presence of specific anti-gp5 antibodies was detected in sera from vaccinated and control animals taken at D0 PV (post vaccination), D0 PI, D14 PI, and D28 PI (Figure 14). Absorbance at D0 PV (where no competition has taken place) was used to calculate the percentage of binding in each sera as follows:

$$\%\,binding = \frac{absorbance \;\; in \;\; serum \;\; of \;\; Dx}{absorbance \;\; of \;\; serum \;\; of \;\; D0 \;\; PV} \times 100$$

Lower binding percentages represent higher levels of anti-gp5 antibodies in the tested sera. As can be seen in Figure 14, vaccinated animals developed higher anti-gp5 titers and a faster antibody response at 14 days post-challenge than non-vaccinated control animals.

A specific immunoperoxidase monolayer assay (IPMA) was performed essentially as described in WENSVOORT *et al.* (1986) for the detection of PRRSV in porcine alveolar lung macrophages infected with the serum samples obtained from different pigs included in this example. The only difference to the method described by WENSVOORT *et al.* (1986) is the use of Protein-A conjugated with horseradish peroxidase as secondary antibody instead of a sheep anti-pig immunoglobulin conjugated with horseradish peroxidase. The results obtained are shown in Figure 14.

As can be seen from Figure 15, most of the pigs were seropositive to PRRSV at D28 PI. However, in the group of animals vaccinated with the MLV 5 out of 16 animals (corresponding to 31%) were seropositive to PRRSV even at D14 PI, whereas none of the control animals vaccinated with rTGEV-$S_{PTV}$-FL was seropositive at D14 PI indicating a faster antibody response against PRRSV in vaccinated animals.

**EXAMPLE 14**

**Evaluation of the efficacy of a killed rTGEV-$S_{PTV}$-$TRS_{3a}$-ORF5-$TRS_{22N}$-ORF6 vaccine in pigs against the challenge with the field isolate PRRSV Olot/91**

This evaluation was performed to evaluate the efficacy of the inactivated subunit vaccine (killed vaccine) derived from rTGEV-$S_{PTV}$-$TRS_{3a}$-ORFS-$TRS_{22N}$-ORF6 expressing different heterologous PRRSV proteins. The inactivated vaccine was used for the prevention or reduction of Porcine Reproductive and Respiratory Syndrome (PRRS) in piglets inoculated with the subunit vaccine via the intramuscular (IM) route challenged with the field isolate PRRSV Olot/91. The efficacy of the vaccine was evaluated by the prevention or interstitial pneumonia, viremia and also by the ability to induce antibodies in serum (evaluated by ELISA, seroneutralization assay (SN), etc.).

The inactivated subunit vaccine derived from rTGEV-$S_{PTV}$-$TRS_{3a}$-ORF5-$TRS_{22N}$-ORF6 expressing the gp5 and M proteins of PRRSV used for this efficacy study was produced as described in Example 11.

<u>Vaccination</u>

27 six to seven week-old pigs were selected and divided into two groups. Pigs of group #1 (14 animals) were vaccinated twice (at 6-7 weeks of age and 3 weeks later) with the PRRSV inactivated-PCV vaccine (3ml via the IM route), whereas pigs of group #2 (13 animals) were kept as non-vaccinated control pigs.

After vaccination, pigs were observed daily for clinical signs to check the safety of the vaccine. Upon observation of any anomaly, a complete clinical examination was conducted by the principal investigator.

For serology studies, blood samples were further taken at D0, D21, and D49 post-vaccination (PV) and sera obtained from these samples were used to perform serology to PRRSV, TGEV and PCV2 (see below).

The humoral response induced by the rTGEV-$S_{PTV}$-$TRS_{3a}$-ORFS-$TRS_{22N}$-ORF6 vaccine was analyzed by indirect PRRSV ELISA detecting both anti-gp5 and anti-M antibodies using PRRSV concentrated by ultracentrifugation as a coating antigen (picture B in Figure 16 and picture C in 17).

PRRSV diluted antigen was incubated overnight at 5 $\pm$ 3°C. After washing, the empty surface of the wells was blocked with PBS, 5% BSA Fraction V and incubated 1 h at 37 $\pm$ 1°C. Sera from vaccinated and control animals at D0, D21 and D42 PV as well as PRRSV positive and negative controls were diluted in PBS, Tween-20 0.05% and incubated at 37 $\pm$ 1°C for 1 h. After washing, peroxidase-conjugated protein A diluted in PBS, Tween-20 0.05% (1:1000) was incubated at 37 $\pm$ 1°C for 1 h. After a final wash, the chromogenic substrate (o-phenylenediamine (OPD)) was added to the wells. The reaction was stopped by adding 3N $H_2SO_4$. The plates were read with an ELISA microplate reader using a 450-nm filter.

The humoral response against the TGEV vector induced by the rTGEV-$S_{PTV}$-$TRS_{3a}$-ORF5-$TRS_{22N}$-ORF6 vaccine was analyzed in the same way as described above for the indirect PRRSV ELISA using TGEV concentrated by ultracentrifugation as a coating antigen instead of PRRSV (picture A in Figure 16 and picture B in 17).

In order to evaluate the humoral response elicited against the porcine circovirus (PCV) Type1-Type2 vaccine included in the rTGEV-$S_{PTV}$-$TRS_{3a}$-ORF5-$TRS_{22N}$-ORF6 inactivated vaccine, the presence of anti-PCV2 ORF2 antibodies at D0 PV, D49 PV and D0 PI (D63 PV) was analyzed by IPMA (see above). This immunological technique quantifies antibodies against the PCV2 capsid protein (ORF2 product) present in Sf9 cells infected with the recombinant BAC-ORF2 PCV2 baculovirus.

Sf9 cells were infected with the recombinant baculovirus Bac-ORF2 PCV2 at a multiplicity of infection (MOI) of 1. Infected cells were grown at 27 $\pm$ 0.5°C for 4 days and then fixed. Sera from vaccinated and control animals were diluted in PBS, Tween-80 0.05% and incubated at 37 $\pm$ 1°C for 1 h. After washing, peroxidase-conjugated protein A diluted in PBS, Tween-20 0.05% (1:1000) was incubated at 37 $\pm$ 1°C for 1 h. After a final wash, the chromogenic substrate (3-amino-9-ethycarbazole) was added to the wells. The reaction was stopped by two washes with PBS. The reaction was positive when the cytoplasm of infected cells showed a red staining. Antibody titer corresponds to the reciprocal of the highest dilution showing a positive reaction.

At D0 PV, the majority of the animals were negative with regard to antibodies against PCV2 (58% of the vaccinated animals and 92% of the animals of the control group. After the second immunization (D49 PV and D63 PV), non-vaccinated animals of the control group remained almost seronegative with regard to PCV2, whereas vaccinated animals had seroconverted to PCV2 (see Table 3).

**Table 3:**

| **Group #1 rTGEV-$S_{PTV}$-$TRS_{3a}$-ORF5-$TRS_{22N}$-ORF6** | | | | **Group #2 non-vaccinated animals** | | | |
|---|---|---|---|---|---|---|---|
| pig # | D0 PV | D49 PV | D0 PI (D63PV) | pig # | D0 PV | D49 PV | D0 PI (D63PV) |
| 294 | 320 | 320 | 320 | 293 | 320 | ND | <20 |
| 295 | 320 | 320 | 80 | 301 | <20 | <20 | <20 |
| 296 | 320 | 1280 | 1280 | 303 | <20 | <20 | <20 |
| 297 | 1280 | 320 | 320 | 306 | <20 | <20 | <20 |
| 298 | 320 | 320 | 80 | 831 | <20 | <20 | <20 |
| 299 | <20 | 5120 | 5120 | 833 | <20 | <20 | 320 |
| 300 | <20 | 320 | 320 | 834 | <20 | 20 | 320 |
| 304 | <20 | <20 | 320 | 835 | <20 | <20 | <20 |
| 305 | <20 | 320 | 320 | 836 | <20 | <20 | <20 |
| 828 | <20 | 1280 | 1280 | 837 | <20 | <20 | <20 |
| 832 | <20 | 1280 | 1280 | 838 | <20 | <20 | <20 |
| 841 | <20 | 320 | 320 | 839 | <20 | <20 | <20 |
| ND: not determined | | | | 840 | <20 | <20 | <20 |

Challenge

Six weeks after the second vaccination, all pigs from both groups were challenged with PRRSV Olot91 strain ($10^{4.7}$ - $10^{4.8}$ $TCID_{50}$/pig) via the intranasal (IN) route.

Blood samples were taken at days 0, 7, 12, 20 and 27 PI in tubes to obtain serum for the determination of serological titers and to study the immune response against PRRSV (ELISA to measure antibodies to gp5 and N proteins, and SN test). The sera were also used to measure viremia of PRRSV by quantitative RT-PCR.

Serological studies

Production of neutralizing antibodies (NAb) in sera obtained from blood taken at D0 PV, D0 PI, D12 PI and D27 PI from vaccinated and control animals was analyzed by seroneutralization assay (SN; see Table 4). Prior to the challenge (D0 PV and D0 PI), no Nab was detected in any group. At D12 PI some animals developed NAb (21.4% in Group #1 (vaccinated animals) and 23% in Group #2 (non-vaccinated animals)), but even higher Nab titers and higher number of positive animals were observed at day 27 post-challenge (75% positive in Group#1, 46% positive in Group #2).

**Table 4**

| Group #1<br>rTGEV-$S_{PTV}$-$TRS_{3a}$-ORF5-$TRS_{22N}$-ORF6 | | | | | Group #2<br>non-vaccinated animals | | | | |
|---|---|---|---|---|---|---|---|---|---|
| Pig # | D0PV | .D0PI | D12PI | D27PI | Pig # | D0PV | D0PI | D12PI | D27PI |
| 294 | <2 | <2 | <2 | 4 | 293 | <2 | <2 | 4 | 4 |
| 295 | <2 | <2 | <2 | 16-32 | 301 | <2 | <2 | <2 | <2 |
| 296 | <2 | <2 | <2 | <2 | 303 | <2 | <2 | 2 | <2 |
| 297 | <2 | <2 | 16 | 16-32 | 306 | <2 | <2 | <2 | <2 |
| 298 | <2 | <2 | <2 | 16 | 831 | <2 | <2 | <2 | <2 |
| 299 | <2 | <2 | 2 | 4-8 | 833 | <2 | <2 | <2 (D7PI) | ND |
| 300 | <2 | <2 | <2 | 4 | 834 | <2 | <2 | <2 | <2 |
| 304 | <2 | <2 | 2 | 2 | 835 | <2 | <2 | <2 | 4 |
| 305 | <2 | <2 | <2 | 2 | 836 | <2 | <2 | <2 | 8 |
| 828 | <2 | <2 | <2 | <2 | 837 | <2 | <2 | <2 | 4 |
| 829 | <2 | <2 | <2 | ND | 838 | <2 | <2 | <2 | <2 |
| 830 | <2 | <2 | <2 | ND | 839 | <2 | <2 | <2 | 4-8 |
| 832 | <2 | <2 | <2 | 32-128 | 840 | <2 | <2 | 4 | 8-16 |
| 841 | <2 | <2 | <2 | <2 | | | | | |
| % positive | 0 | 0 | 21.4 | 75 | % positive | 0 | 0 | 23 | 46 |

ND: not determined

The presence of specific anti-gp5 antibodies was analyzed by the competition ELISA INGEZIM PRRS gp5 Compac (INGENASA) according to the manufacturer's instructions. This method measures the competition between the porcine problem sera and an anti-gp5-peroxidase labeled monoclonal antibody (MAb) for binding to a recombinant gp5 protein previously coated onto the surface of the wells of a test plate. Using a chromogenic substrate, the presence of specific anti-gp5 antibodies was detected in sera from vaccinated and control animals taken at D0 PV, D0 PI, D14 PI, D21 PI, and D28 PI (see Figure 18). Absorbance at D0 PV (where no competition has taken place) was used to calculate the percentage of binding in each sera as follows:

$$\%\,binding = \frac{absorbance\ in\ serum\ of\ Dx}{absorbance\ of\ serum\ of\ D0\ PV} \times 100$$

Lower binding percentages represent higher levels of anti-gp5 antibodies in the tested sera. As can be seen in Figure 18, vaccinated animals developed higher anti-gp5 titers and a faster antibody response at 14 days post-challenge than non-vaccinated control animals.

For quantification of PRRSV in serum (viremia) and in pulmonary lavages blood samples were taken at D0, D3, D7, D14, D20, and D28 PI in tubes for isolating serum and were kept at -80 ± 10°C until further analysis. Further, at D27 PI all pigs were slaughtered and necropsied. Lung gross lesions were evaluated and pulmonary lavages were performed. For obtaining the pulmonary lavages lungs were extracted and 100 mL of PBS were introduced by the trachea into the lungs followed by a mild massage and recovering of the PBS into sterile tubes by decantation. Aliquots of pulmonary lavages were kept at -80 ± 10°C.

RNA purification from serum and from pulmonary lavages were performed using the kit Nucleospin 96 RNA (Macherey-Nagel) following the manufacturer's instructions. RNA samples were kept at -80 ± 10°C.

For the quantification of PRRSV, a real-time RT-PCR technique (qRT-PCR) has been set up. First, specific primers (forward primer (Olot91F): 5' TTCCCTCTGCTTGCAATCG 3' (SEQ ID NO: 16) and reverse primer (Olot91R): 5' GGATGAAAGCGACGCAGTTC 3' (SEQ ID NO: 17)) and a MGB® probe (probe (Olot91S): 5' 6-FAM-ACGGCTTTTAATCAAGGC-MGB 3' (SEQ ID NO: 18)) comprising 6-FAM as reporter dye at the 5' end and a MGB (minor groove binder) moiety at the 3' end serving as non-fluorescent quencher were designed using the Applied Biosystems' Primer Express program for amplification of a 67 bp fragment of the PRRSV Olot/91 genome. Second, standard RNA was prepared by purifying RNA from the PRRSV challenge strain and adjusting the same to $10^4$ PRRSV $TCID_{50}/\mu l$ RNA. The RNA was divided in aliquots and kept at -80 ± 10°C. Reagent concentration and reaction conditions were optimized using the kit RNA UltraSense™ One-Step qRT-PCR System (Invitrogen).

The purified viral RNA was used as template, reverse transcribed at 50°C for 30 min and denatured at 95°C for 5 min. The program used for PCR consisted of 40 cycles of denaturation at 95°C for 20 sec and annealing at 56°C for 40 sec. The qRT-PCR was conducted in a 7500 Real-Time PCR System thermocycler. The results were analyzed with the SDS 1.2 software (Applied Biosystems) (see Figure 19).

As can be seen from Figure 19, due to vaccination a clear decrease of the PRRSV titers in serum (from 911 PRRSV $TCID_{50}$/ml to 144 PRRSV $TCID_{50}$/ml) could be observed at D14 PI in vaccinated animals. A further decrease could be observed at D20 and D28 PI.

Porcine alveolar macrophages obtained from vaccinated pigs by performing lung lavages contained much less virus (9606.74 PRRSV $TCID_{50}$/ml lavage) than those of control pigs (71518.33 PRRSV $TCID_{50}$/ml lavage). It has to be noted, that the presence of PRRSV in lung sections was also lower in vaccinated pigs (25%) than in control pigs (66.6%), indicating the ability of the inactivated vaccine to reduce the replication of PRRSV in its target tissue.

In the lungs obtained from the animals slaughtered at D27 PI macroscopic lung lesions were analyzed and scored according to the procedure described by HANNAN *et al*. (1982). Table 5 shows the means of the pneumonic score which reveal statistical differences between animals of the control group and vaccinated animals. Animals from the control group had higher mean pneumonic scores than vaccinated pigs.

**Table 5**

| Group | pneumonic score (mean value) |
|---|---|
| control group | 8.956 |
| vaccinated animals | 4.801 |
| p-value | statistically relevant (0.0184) |

**Example 15**

**Vector expressing ORF5-LIMP-II fusion protein**

The recombinant vector was engineered using the same cloning protocol as described above. The expression of the fusion protein was directed by TRS-3a, and the vector was designed with enteric tropism. The vector pBAC-Sc11-TRS3a-ORF5-LIMPII (see Figure 20) was obtained by cloning the PRRSV ORF5 fused to the last 20 aa (SEQ ID NO: 11) of porcine LIMP-II (GeneBank accession number AAS55916), containing the lysosomal targeting sequence of this protein.

Stable recombinant virus was recovered from pBAC-Sc11-TRS3a-ORF5-LIMPII cDNA.

Gp5-LIMPII expression was detected by Western-blot and immunofluorescence using specific inhibitors of lysosomal pH (i.e., chloroquine, $NH_4Cl$) . High and stable expression of the antigen was found.

This specific fusion protein was also expressed from the TGEV vector expressing Gp5-LIMPII and M protein. This resulted in further improvement of the stability of the dicistronic vector.

**Example 16**

**Construction of recombinant TGEV clones with mutated S genes**

A collection of recombinant TGEVs was constructed that only differ in the 5' S gene, but otherwise have an identical sequence. All the recombinant viruses were generated starting from the same infectious cDNA clone of TGEV, having the sequence as described by ALMAZAN *et al.* (2000) with the exception that the S protein was derived from the TGEV-PUR46-PTV isolate with an exclusively respiratory tropism (PENZES *et al*., 2001). The recombinant TGEVs were constructed as previously described (ALMAZAN *et al*., 2000) using an infectious cDNA cloned as a bacterial artificial chromosome (BAC).

The two parental TGEV isolates TGEV-PUR46-PTV (which infects the respiratory tract) and TGEV-PUR46-C11 (which infects the enteric and respiratory tract) differ at the 5' end of the S gene as shown in Figure 22 (see upper box in the left column). The highlighted nucleotides reflect differences between the two sequences, while the light vertical box represents a deletion of three nucleotides. In addition, some clones also have a deletion of 6 nucleotides already present in the S protein of the S gene of TGEV-PUR46-PTV (PENZES *et al*., 2001).

Starting from the virus rTGEV-$S_{PTV}$ a collection of recombinant viruses was generated by successively introducing sequences present in the S gene of TGEV-PUR46-C11 but not in PTV into the sequence of the S gene of the rTGEV-$S_{PTV}$ (see sequences in Figure 22). The nucleotides in the dark boxes represent the nucleotides that haven been amended.

The recombinant viruses were plaque purified three times on ST cells and amplified by two additional passages on these cells. The virus stocks obtained were used as Working Stocks [WS passage 0, acronym WS (PO)]. After propagating the viruses in ST cells the sequence of the recovered isolates was determined and is as indicated in Figure 22.

The viruses were provided to 3 day old newborn piglets. The medium virus titers in the lungs or the gut 2-3 days post-infection are shown for each recombinant virus. The clone referred to as "R7 clone 1" (or $S_{7.1}$) shows the highest activity in both the lung and the gut of piglets after 2-3 days post-infection.

**Example 17**

**Growth kinetics of selected recombinant TGEV clones with mutated S genes**

The growth of recombinant TGEV viruses rTGEV-PUR46-PTV, rTGEV-PUR46-C11 and TGEV-PUR46-S7.1 was further evaluated *in vitro* on ST cells and *in vivo* in piglets.

For *in vitro* studies the titer of ST cells infected with either TGEV-PUR46-C11 (TGEV C11) and TGEV-PUR46-S7.1 (TGEV7.1) was evaluated. The ST cells were inoculated with either virus from Working Stock (at passage 0; see Example 16 above) or virus after 6 additional passages in ST cells. Virus titers were titrated using a plaque assay on ST cells. The results are shown in Table 6 and represent medium values of three independent experiments.

**Table 6**

| | **Titer** (PFU/ml) | |
|---|---|---|
| **Virus** | WS (P0) | Passage 6 |
| rTGEV-PUR46-C11 | $3 \times 10^7$ | $4 \times 10^8$ |
| TGEV-PUR46-S7.1 | $5 \times 10^8$ | $3 \times 10^8$ |

As can be seen from Table 6 both TGEV-PUR46-C11 and TGEV-PUR46-S7.1 grow to high titers in cell culture on ST cells without any significant loss of activity even after 6 passages.

For *in vivo* studies 3 day old newborn piglets were inoculated with $3 \times 10^8$ pfu per piglet of TGEV-PUR46-C11 (TGEV C11) and TGEV-PUR46-S7.1 (TGEV7.1). The piglets were infected with either virus from the Working Stock (P0) or virus obtained after 6 additional passages on ST cells. Animals were sacrificed at day 2 post inoculation and the virus titers per gram of animal tissue (gut or lung) were titrated using a plaque assay on ST cells. The results are shown in Table 7 and represent medium values of three independent experiments.

**Table 7**

| | **Titer** (PFU/g tissue) | | | |
|---|---|---|---|---|
| | WS (P0) | | Passage 6 | |
| **Virus** | Lung | Gut | Lung | Gut |
| rTGEV-PUR46-C11 | $5 \times 10^6$ | $5 \times 10^6$ | $2 \times 10^6$ | $5 \times 10^5$ |
| TGEV-PUR46-S7.1 | $4 \times 10^7$ | $8 \times 10^6$ | $8 \times 10^6$ | $4 \times 10^6$ |

The results shown in Table 7 illustrate that both viruses grew well in both the lung and the gut, when the animals were infected with virus from the Working Stock (P0) Further, also the virus passaged six times on ST cells prior to infection grew to high titers in both organs. However, in both organs the $S_{7.1}$ provides significantly higher titers than the $S_{C11}$.

In a further experiment, three day old newborn piglets were inoculated with $3 \times 10^8$ pfu per piglet of rTGEV-PUR46-PTV, TGEV-PUR46-C11 and TGEV-PUR46-S7.1. Animals were sacrificed at days 1, 2, 3, and 4 post inoculation, respectively, and virus titers per gram of tissue were determined by plaque titration on ST cells (see Figure 23).

As can be seen from Figure 23, rTGEV-PUR46-PTV only has respiratory tropism. Further, the virus titer is not stable during the four days *in vivo* but decreases slowly (see left graph of Figure 22). Both the TGEV-PUR46-C11 and TGEV-PUR46-S7.1 have dual tropism and grow in the respiratory (lung) and the enteric tract (gut) (see Figure 23). However, whereas the titer of the TGEV-PUR46-C11 in both organs decreases significantly during the four days in the piglet, the titer of TGEV-PUR46-S7.1 remains almost stable (in the lung) or decreases to a lesser extent than the TGEV-PUR46-C11 (gut). In any case, the virus titers obtained for TGEV-PUR46-S7.1 are higher in both organs than those obtained for TGEV-PUR46-C11, i.e. the recombinant TGEV comprising S gene $S_{7.1}$ is more stable *in vitro* and *in vivo* than the TGEV-PUR46-C11.

The following represent further embodiments of the present invention:

1. Nucleic acid comprising:

(a) sequences of a replication competent transmissible gastroenteritis virus (TGEV), which sequences encode a TGEV replicase under the control of expression regulatory sequences, wherein the replicase is expressed in a host cell and will initiate replication of the nucleic acid and thus increase the number of nucleic acids in the cell; and

(b) a sequence encoding at least one neutralizing epitope of ORF5 of porcine reproductive and respiratory syndrome virus (PRRSV), which sequence includes a disulfide bridge forming residue; and

(c) a sequence encoding at least one further polypeptide capable of increasing an immune response against PRRSV.

2. Nucleic acid comprising:

(a) sequences of a replication competent transmissible gastroenteritis virus (TGEV), which sequences encode a TGEV replicase under the control of expression regulatory sequences so that expression of the replicase in a cell containing the nucleic acid will initiate replication of the nucleic acid and thus increase the number of nucleic acids in the cell; and

(b) sequences encoding multimers of one or several neutralizing epitopes of ORF5 of PRRSV and at least one further polypeptide capable of increasing an immune response against PRRSV.

3. Nucleic acid comprising:

(a) sequences of a replication competent transmissible gastroenteritis virus (TGEV), which sequences encode a TGEV replicase under the control of expression regulatory sequences so that expression of the replicase in a cell containing the nucleic acid will initiate replication of the nucleic acid and thus increase the number of nucleic acids in the cell; and

(b) a sequence encoding at least one neutralizing epitope of ORF5 of porcine reproductive and respiratory syndrome virus (PRRSV); and

(c) a sequence encoding at least one further polypeptide capable of increasing an immune response against PRRSV, which stabilizes the expression of the sequence of (b), such that more than 60 % of the cells infected with this nucleic acid sequence express the gene product of the sequence of (b) after 20 passages of the virus *in vitro.*

4. Nucleic acid according to any of embodiments 1 to 3, wherein the sequence encoding said neutralizing epitope of ORF 5 has been modified to knock out glycosylation sites that interfere with induction of antibodies.

5. Nucleic acid according to any of embodiments 1 to 4, wherein the nucleic acid encodes a TGEV replicase and a sequence encoding the TGEV N protein.

6. Nucleic acid according to any of embodiments 1 to 5, wherein the replication competent TGEV vector is not infectious.

7. Nucleic acid according to any of embodiments 1 to 5, wherein the replication competent TGEV vector is infectious.

8. Nucleic acid according to embodiment 7, wherein the nucleic acid further comprises one or more of the following TGEV genes: S, E, M and/or N or sequences having a similarity of at least 60% to the given sequence.

9. Nucleic acid according to embodiment 7 or 8, wherein the TGEV infectious viral particles obtainable from the association of TGEV proteins and the nucleic acid sequences are attenuated viral particles.

10. Nucleic acid according to any of embodiments 7 to 9, wherein the S gene is derived from a respiratory virus.

11. Nucleic acid according to any of embodiments 7 to 9, wherein the S gene is derived from an enteric strain of TGEV.

12. Nucleic acid according to any of embodiments 7 to 11, wherein the S gene provides enteric and respiratory tropism.

13. Nucleic acid according to embodiment 12, wherein the S gene has been modified and has the SEQ ID NO: 1.

14. Nucleic acid according to any one of embodiments 1 to 13, wherein the sequence comprises a neutralizing epitope of ORF5 and at least one epitope of ORF 6 of PRRSV.

15. Nucleic acid according to any one of embodiments 1 to 13, wherein the sequence comprises a neutralizing epitope of ORF5 and a neutralizing epitope of ORF 6 of PRRSV.

16. Nucleic acid according to embodiment 14, wherein the sequence consists of a neutralizing epitope of ORF5 and whole ORF 6 of PRRSV.

17. Nucleic acid according to embodiment 15, wherein the sequence consists of a neutralizing epitope of ORF5 and a neutralizing epitope of ORF 6 of PRRSV.

18. Nucleic acid according to any one of embodiments 1 to 17, wherein the nucleic acid comprises the sequences of ORF5 and ORF6 of PRRSV each under the control of a separate expression regulatory sequence.

19. Nucleic acid according to embodiment 18, wherein the sequence consists of (1) the sequence of ORF5 of PRRSV under the control of the transcription regulating sequence of gene 3a, (2) the sequence of ORF6 of PRRSV under the control of the transcription regulating sequence $TRS_{22N}$ set forth as SEQ ID NO: 19 and (3) the sequence of the S gene derived from the attenuated strain PTV of TGEV.

20. Nucleic acid according to any one of embodiments 1 to 19, wherein the nucleic acid further encodes the lysosomal targeting signal of lysosomal integral membrane protein-II (LIMPII).

21. Nucleic acid according to embodiment 21, wherein said nucleic acid encodes a fusion protein consisting of LIMPII and ORF5 and/or ORF6 of PRRSV.

22. Nucleic acid comprising:

(a) sequences of a replication competent transmissible gastroenteritis virus (TGEV), which sequences encode a TGEV replicase under the control of expression regulatory sequences, wherein the replicase is expressed in a host cell and will initiate replication of the nucleic acid and thus increase the number of nucleic acids in the cell;

(b) residues 118 to 138 of SEQ ID NO:2 or a sequence having a similarity of 90% to these residues under the control of an expression regulatory sequence; and

(c) SEQ ID NO:3 or a sequence having a similarity of 90% to SEQ ID NO:3 under the control of an expression regulatory sequence; wherein

the sequences of (a) and (b) and (c) are each under the control of a different expression regulatory sequence.

23. Nucleic acid according to embodiment 22 further comprising the nucleic acid sequence SEQ ID NO:12, which encodes the lysosomal targeting signal of lysosomal integral membrane protein-II (LIMP-II) or a sequence having a similarity of 90% to SEQ ID NO:12.

24. Nucleic acid according to any one of embodiments 1 to 15 and 18 to 23, wherein the nucleic acid further encodes the p35 subunit of IL-12 or other interleukins.

25. Recombinant RNA encoded by a nucleic acid according to any of embodiments 1 to 24.

26. Vector comprising a nucleic acid according to one of embodiments 1 to 25.

27. Vector according to embodiment 26, wherein the vector is a cDNA vector.

28. Vector according to embodiment 27, wherein the vector is a BAC-TGEV$^{FL}$ vector.

29. Vector according to embodiment 28, wherein the BAC-TGEV$^{FL}$ vector contains the nucleic acid sequence of embodiments 18 or 19.

30. Vector according to any of embodiments 26 to 29, wherein the vector is capable of replicating the nucleic acid within a host cell.

31. Host cell comprising a vector according to one of embodiments 26 to 30.

32. Host cell according to embodiment 31, wherein the cell is a bacterial cell, a yeast cell, an insect cell, an animal cell or a human cell.

33. Host cell according to embodiment 32, wherein the cell is a porcine swine testis cell line, such as the cell line deposited under ATCC CRL-1746.

34. Virus particle comprising a nucleic acid according to any of embodiments 1 to 24 and at least one TGEV coat protein.

35. Virus particle according to embodiment 34, comprising all TGEV coat proteins of the native TGEV virus particle.

36. Polypeptide encoded by SEQ ID NO:13 or encoded by a sequence having a similarity of 90% to SEQ ID NO:13.

37. Polypeptide encoded by SEQ ID NO:14 or encoded by a sequence having a similarity of 90% to SEQ ID NO:14.

38. Pharmaceutical preparation comprising a nucleic acid according to one of embodiments 1 to 24, a viral RNA or vector according to one of embodiments 25 to 30 or a host cell according to one of embodiments 31 to 33 or a virus particle according to embodiment 34 or 35 or a polypeptide according to embodiment 36 or 37.

39. Pharmaceutical preparation according to embodiment 38 further comprising a pharmaceutically acceptable carrier, excipient and/or adjuvants.

40. Vaccine capable of protecting an animal against PRRSV comprising a nucleic acid according to one of embodiments 1 to 24, a viral RNA or vector according to one of embodiment 25 to 30, a host cell according to one of embodiments 31 to 33 or a virus particle according to embodiment 34 or 35 or a polypeptide according to embodiment 36 or 37.

41. Vaccine according to embodiment 40, wherein the vaccine is a multivalent vaccine capable to provide protection against one or several pig pathogens other than PRRSV.

42. Vaccine according to embodiment 41, further comprising antigens derived from other viral and/or bacterial pathogens and/or proteins which will provide immunity against pathogens.

43. Vaccine according to embodiment 41 or 42, further comprising one or several antigens derived from other viral pathogens selected from Swine Influenza Viruses, Porcine Parvovirus, Porcine Circovirus Type 2, Classical Swine Fever, African Swine Fever, Foot-and-Mouth Disease, Pseudo-Rabies Virus, Porcine Circovirus Type 1, Porcine Adenoviruses, Porcine Enteroviruses, Porcine Respiratory Coronavirus, Porcine Rotavirus, Encephalomyocarditis Virus, Porcine Epidemic Diarrhea Virus, Blue Eye Disease Viruses, Hepatitis E Virus and/or West Nile Virus, Nipah virus.

44. Vaccine according to any one of embodiments 41 to 43, further comprising one or several antigens derived from other viral pathogens selected from Swine Influenza Viruses, Porcine Parvovirus, Porcine Circovirus Type 2, Classical Swine Fever, African Swine Fever and/or Foot-and-Mouth Disease.

45. Vaccine according to any one of embodiments 41 to 43, further comprising one or several antigens derived from bacterial pathogens selected from Mycoplasma hyopneumoniae, Actinobacillus pleuropneumoniae, Actinobacillus suis, Haemophilus parasuis, Pasteurella multocida type A (toxins), Pasteurella multocida type D (toxins), Bordetella bronchiseptica, Isospora suis, Brachyspira hyodysenteriae, Brachyspira pilosicoli, Lawsonia intracellularis, Erysipelothrix rhusiopathiae, Escherichia coli, Salmonella enterica, Mycoplasma hyorinis, Streptococcus suis, Clostridium perfringens, Clostridium difficile, Clostridium novyi, Brucella abortus and/or Candidatus helicobacter suis.

46. Vaccine according to any one of embodiments 41 to 43, further comprising one or several antigens derived from bacterial pathogens selected from Mycoplasma hyopneumoniae, Actinobacillus pleuropneumoniae, Actinobacillus suis, Haemophilus parasuis, Pasteurella multocida type A (toxins), Pasteurella multocida type D (toxins), Bordetella bronchiseptica, Isospora suis, Brachyspira hyodysenteriae, Brachyspira pilosicoli, Lawsonia intracellularis, Erysipelothrix rhusiopathiae, Escherichia coli and/or Salmonella enterica.

47. Vaccine according to any one of embodiments 41 to 46, wherein the further viral or bacterial antigen is present in the multivalent vaccine as an attenuated or inactivated virus or bacterium or as a nucleic acid sequence encoding one or several of the further viral or bacterial antigens.

48. Vaccine according to any one of embodiments 41 to 47, further comprising nucleic acid sequences encoding proteins which will confer protection against pig pathogens, such as nucleic acid sequences encoding one or several antibodies having specificity for bacterial or viral diseases or nucleic acid sequences encoding the porcine prion protein.

49. Vaccine according to one of embodiments 40 to 48 further comprising a pharmaceutically acceptable carrier, excipient and/or adjuvants.

50. Vaccine according to one of embodiments 40 to 49, wherein the vaccine is suitable for vaccinating a swine, preferably a sow.

51. Vaccine according to one of embodiments 40 to 50, wherein the vaccine is capable of inducing both a systemic immune response and a mucosal immune response against infectious viral agents.

52. Vaccine according to any one of embodiments 40 to 51, wherein the vaccine is a modified live vaccine or an inactivated vaccine.

53. Vaccine according to embodiment 52, wherein the inactivated vaccine is diluted with porcine circovirus (PCV) Type1-Type2 inactivated vaccine previously adjuvanted with sulfolipo-cyclodextrin.

54. Vaccine according to embodiment 53, wherein the inactivated vaccine is diluted with porcine circovirus (PCV) Type1-Type2 inactivated vaccine previously adjuvanted with 20 % sulfolipo-cyclodextrin.

55. Vaccine according to embodiment 53 or 54, wherein the inactivated vaccine is diluted 1:3 with the porcine circovirus (PCV) Type1-Type2 inactivated vaccine.

56. Nucleic acid comprising a sequence having at least 95% similarity to the sequence of SEQ ID NO: 1, wherein the protein encoded by the sequence having at least 95% similarity to the sequence of SEQ ID NO: 1 is a spike protein, which spike protein, when present as part of a TGEV virus, is capable of inducing TGEV infections in the respiratory tract and the enteric tract of pigs, wherein the infections are characterized by

- a viral titer of at least $1\times10^7$ PFU in ST cells infected with TGEV derived from 1 gram of animal respiratory tract tissue 2 days post infection of the animal;
- a viral titer of at least $1\times10^6$ PFU in ST cells infected • with TGEV derived from 1 gram of animal enteric tract tissue 2 days post infection of the animal.

57. Nucleic acid comprising the sequence shown in SEQ ID NO:1.

58. Nucleic acid according to embodiment 56 or embodiment 57, wherein the nucleic acid does not comprise a sequence encoding ORF5 of PRRSV or a fragment thereof encoding a neutralizing epitope.

59. Vector comprising a nucleic acid according to one of embodiments 56 to 58.

60. Vector according to embodiment 59, wherein the vector is a cDNA vector.

61. Vector according to embodiment 60, wherein the vector is a BAC-TGEV vector.

62. Vector according to any of embodiments 59 to 61, wherein the vector is capable of replicating the nucleic acid within a host cell.

63. Host cell comprising a vector according to one of embodiments 59 to 62.

64. Protein encoded by the nucleic acid of one of embodiments 56 to 58.

65. Virus particle comprising the nucleic acid of one of embodiments 56 to 58.

66. Pharmaceutical composition comprising a nucleic acid according to one of embodiments 56 to 58, a vector according to one of embodiments 59 to 62, a host cell according to embodiment 63, a protein according to embodiment 64 or a virus particle according to embodiment 65.

67. Vaccine comprising a nucleic acid according to one of embodiments 56 to 58, a vector according to one of embodiments 59 to 62, a host cell according to embodiment 63, a protein according to embodiment 64 or a virus particle according to embodiment 65.

**Bibliography:**

Alamazan F., Gonzales J.M., Penzes Z., Izeta A., Calvo E., Plana-Duran J., Enjuanes L. (2000). Engineering the largest RNA virus genome as an infectious bacterial artificial chromosome. Proc. Natl. Acad. Sci. USA 97: 5516-5521.

Alonso S., Izeta A., Sola I., Enjuanes L. (2002). Transcription regulatory sequences and mRNA expression levels in the coronavirus transmissible gastroenteritis virus. J. Virol. 76:1293-1308.

Ansari I.H., Kwon B., Osorio F.A., Pattnaik A.K. (2006). Influence of N-linked glycosylation of porcine reproductive and respiratory syndrome virus GP5 on virus infectivity, antigenicity, and ability to induce neutralizing antibodies. J Virol. 80(8):3994-4004.

Botner A., Strandbygaard B., Sorensen K.J., Have P., Madsen K.G., Madsen E.S., Alexandersen S. (1997). Appearance of acute PRRS-like symptoms in sow herds after vaccination with a modified live PRRS vaccine. Vet. Rec. 141:497-499.

Bullock W., Fernández J.M., Short J.M. (1987). XL1-Blue: a high efficiency plasmid transforming recA E.coli strain with P-galactosidase selection. Biotechniques 8:26-27.

Carter Q.L., Curiel R.E. (2005). Interleukin-12 (IL-12) ameliorates the effects of porcine respiratory and reproductive syndrome virus (PRRSV) infection. Vet Immunol Immunopathol 107:105-118.

Chang K.-Y., Tinoco I. (1994). Characterization of a "kissing" hairpin complex derived from the human immunodeficiency virus genome. Proc. Natl. Acad. Sci. USA 91:8705-8709.

Charley B., Laude H. (1988). Induction of alpha-interferon by transmissible gastroenteritis coronavirus: Role of transmembrane glycoprotein E1. J. Virol. 62:8-10.

Chung H.K., Chae C. (2003). Expression of interleukin-10 and interleukin-12 in piglets experimentally infected with porcine reproductive and respiratory syndrome virus (PRRSV). J. Comp. Path. 129(2-3):205-212.

Cousens L.P., Peterson R., Hsu S., Dorner A., Altman J.D., Ahmed R., Biron C.A. (1999). Two roads diverged: interferon alpha/beta- and interleukin 12-mediated pathways in promoting T cell interferon gamma responses during viral infection. J. Exp. Med 189:1315-1328.

Delmas B, Gelfi J., Kut E., Sjostrom H., Noren O., Laude H. (1994). Determinants essential for the transmissible gastroenteritis virus-receptor interaction reside within a domain of aminopeptidase-N that is distinct from the enzymatic site. J Virol. 68(8):5216-24.

Doyle L.P., Hutchings L.M. (1946). A transmissible gastroenteritis in pigs. J. Amer. Vet. Med. Assoc. 108:257-259.

Enjuanes L., et al. (2003). Virus based vectors for gene expression in mammalian cells; Coronaviruses; in Gene transfer and expression in mammalian cells; Ed. S.C. Makrides, p. 151-158.

Fenaux M., Opriessnig T., Halbur P.G., Meng X.J. (2003). Immunogenicity and pathogenicity of chimeric infectious DNA clones of pathogenic porcine circovirus type 2 (PCV2) and nonpathogenic PCV1 in weanling pigs. J. Virol. 77 (20):11232-43.

Fenaux M., Opriessnig T., Halbur P.G., Elvinger F., Meng X.J. (2004). A chimeric porcine circovirus (PCV) with the immunogenic gene of the pathogenic PCV type 2 (PCV2) cloned into the genomic backbone of the non-pathogenic PCV1 induces protective immunity against PCV2 infection in pigs. J. Virol. 78(12):6297-6303.

Foss D.L., Zilliox M.J., Meier W., Zuckermann F., Murtaugh M.P. (2002). Adjuvant danger signals increase the immune response to porcine reproductive and respiratory syndrome virus. Viral Immunol. 15(4): 557-566.

Gonzalez J.M., Penzes Z., Almazan F., Calvo E., Enjuanes L. (2002). Stabilization of a full-length infectious cDNA clone of transmissible gastroenteritis coronavirus by the insertion of an intron. J. Virol. 76:4655-4661.

Graham R.L., Sims A.C., Brockway S.M., Baric R.S., Denison M.R. (2005). The nsp2 replicase proteins of murine hepatitis virus and severe acute respiratory syndrome coronavirus are dispensable for viral replication. J. Virol. 79: 13399-13411

Haelterman E.O., Pensaert M.B. (1967). Pathogenesis of transmissible gastroenteritis of swine. Proc. 18th World Vet. Congress 2:569-572.

Hanahan D., Jessee J., Bloom F.R. (1991). Plasmid transformation of Escherichia coli and other bacteria. Methods Enzymol. 204:63-113.

Hannan P.C., Bhogal B.S., Fish J.P. (1982). Tylosin tartrate and tiamutilin effects on experimental piglet pneumonia induced with pneumonic pig lung homogenate containing mycoplasmas, bacteria and viruses. Res. Vet. Sci. 33:76-88

Hertzig T., Scandella E., Schelle B., Ziebuhr J., Siddell S.G., Ludewig B., Thiel V. (2004). Rapid identification of coronavirus replicase inhibitors using a selectable replicon RNA. J. Gen. Virol 85:1717-1725.

Izeta A., Smerdou C., Alonso S., Penzes Z., Méndez A., Plana-Durán J., Enjuanes L. (1999). Replication and packaging of transmissible gastroenteritis coronavirus-derived synthetic minigenomes. J. Virol. 73:1535-1545.

Kiyono et al. (1996). Mucosal Vaccines. Academic Press, New York.

Labarque G., Reeth K.V., Nauwynck H., Drexler C., Van Gucht S., Pensaert S. (2004). Impact of genetic diversity of European-type porcine reproductive and respiratory syndrome virus strain on vaccine efficacy. Vaccine 22: 4183-4190.

Laude H., Rasschaert D., Delmas B., Godet M., Gelfi J., Bernard C. (1990). Molecular biology of transmissible gastroenteritis virus. Vet. Microbiol. 23:147-154.

Le Bon A., Schiavoni G., D'Agostino G., Gresser I., Belardelli F., Tough D.F. (2001). Type I interferons potently enhance humoral immunity and can promote isotype switching by stimulating dendritic cells in vivo. Immunity 14: 461-470. Lopez O.J., Osorio F.A. (2004). Role of neutralizing antibodies in PRRSV protective immunity. Vet Immunol Immunopathol 102:155-163.

McClurkin A.W., Noman J.O. (1966). Studies on transmissible gastroenteritis of swine. II. Selected characteristics of a cytopathogenic virus common to five isolates from transmissible gastroenteritis. Can. J. Comp. Med. Vet. Sci. 30:190-198.

Meier W.A., Galeota J., Osorio F.A., Husmann R.J., Schnitzlein W.M., Zuckermann F.A. (2003). Gradual development of the interferon-gamma response of swine to porcine reproductive and respiratory syndrome virus infection or vaccination. Virology 309:18-31.

Meng X.J. (2000). Heterogeneity of porcine reproductive and respiratory syndrome virus: implications for current vaccine efficacy and future vaccine development. Vet. Microbiol. 74:309-329.

Mengeling W.L., Lager K.M., Vorwald A.C., Koehler K.J. (2003a). Strain specificity of the immune response of pigs following vaccination with various strains of porcine reproductive and respiratory syndrome virus. Vet. Microbiol. 93 (1):13-24 .

Mengeling W.L., Lager K.M., Vorwald A.C., Clouser D.F. (2003b). Comparative safety and efficacy of attenuated single-strain and multi-strain vaccines for porcine reproductive and respiratory syndrome. Vet. Microbiol. 93(1):25-38.

Murtaugh M.P., Xiao Z., Zuckermann F. (1995). Comparison of the structural protein coding sequences of the VR-2332 and Lelystad virus strains of the PRRS virus. Arch. Virol. 140:1451-1460.

Nielsen H.S., Oleksiewicz M.B., Forsberg R., Stadejek T., Botner A., Storgaard T. (2001). Reversion of a live porcine reproductive and respiratory syndrome virus vaccine investigated by parallel mutations. J. Gen. Virol. 82:1263-1272.

Ortego J., Escors D., Laude H., Enjuanes L. (2002). Generation of a replication-competent, propagation deficient virus vector based on the transmissible gastroenteritis coronavirus genome. J. Virol. 76:11518-11529.

Ortego J., Sola I., Almazan F., Ceriani J.E., Riquelme C., Balach M., Plana-Duran J., Enjuanes L. (2003). Transmissible gastroenteritis coronavirus gene 7 is not essential but influences in vivo replication and virulence. Virol 308: 13-22.

Osorio F.A., Zuckermann F., Wills R., Meier W., Christian S., Galeota J., Doster A.R. (1998). PRRSV: comparison of commercial vaccines in their ability to induce protection against current PRRSV strains of high virulence. In Allen D. Lennan Swine Conf., pp. 179-182.

Osorio Y., Ghiasi H. (2005). Recombinant herpes simplex virus type 1 (HSV-1) codelivering interleukin-12p35 as a molecular adjuvant enhances the protective immune response against ocular HSV-1 challenge. J. Virol. 79(6): 3297-3308.

Ostrowski M., Galeota J.A., Jar A.M., Platt K.B., Osorio F.A., Lopez O.J. (2002). Identification of neutralizing and nonneutralizing epitopes in the porcine reproductive and respiratory syndrome virus GP5 ectodomain. J. Virol. 76: 4241-4250.

Panley R., Hoglund S., Prasad G. (Eds.) (1989). Progress in Vaccinology. Veterinary Vaccines Volume 4, Springer Verlag.

Pastoret P.P., Blancou P., Vannier, Verschueren C. (Eds.) (1997) Veterinary Vaccinology, Elsevier Science B.V.

Penzes Z., Gonzales J.M., Calvo E., Izeta A., Smerdou C., Mendez A., Sanches C.M., Sola I., Almazan F., Enjuanes L. (2001). Complete genome sequence of Transmissible Gastroenteritis Coronavirus PUR46-MAD clone and evolution of the purdue virus cluster. Virus Genes 23(1):105-118.

Pesch S., Meyer C., Ohlinger V.F. (2005). New insights into genetic diversity of European porcine reproductive and respiratory syndrome virus (PRRSV). Vet. Microbiol. 107:31-48.

Pfeffer L.M., Dinarello C.A., Herberman R.B., Williams B.R., Borden E.C., Bordens R., Walter M.R., Nagabhushan T.L., Trotta P.P., Pestka S. (1998). Biological properties of recombinant alpha-interferons: 40th anniversary of the discovery of interferons. Cancer Res. 58:2489-2499.

Rodriguez F., Harkins S., Redwine J.M., De Pereda J.M., Whitton J.L. (2001). CD4+ T cells induced by a DNA vaccine: immunological consequences of epitope-specific lysosomal targeting. J. Virol. 75(21) : 10421-10430.

Sambrook et al. (1989). Molecular Cloning: A Laboratory Manual. Ed Cold Spring Harbor Laboratory.

Sánchez C.M., Izeta A., Sánchez-Morgado J.M., Alonso S., Sola I., Balasch M., Plana-Durán J., Enjuanes, L. (1999). Targeted recombination demonstrates that the spike gene of transmissible gastroenteritis coronavirus is a determinant of its enteric tropism and virulence. J. Virol. 73:7607-7618.

Sawicki & Sawicki (1990). Coronavirus transcription: subgenomic mouse hepatitis virus replicative intermediates function in RNA synthesis. J. Virol. 64(3):1050-6.

Sethna P.B., Hung S.-L., Brian D.A. (1989). Coronavirus subgenomic minus-strand RNAs and the potential for mRNA replicons. Proc. Natl. Acad. Sci. USA 86:5626-5630.

Siddell S.G. (1995). "The Coronaviridae. " The Viruses (H. Fraenkel-Conrat, and R. R. Wagner, Eds.) Plenum Press, New York.

Sola I, Alonso S., Zúñiga S., Balasch M., Plana-Duran J., Enjuanes L. (2003). Engineering the transmissible gastroenteritis virus genome as an expression vector inducing lactogenic immunity. J. Virol. 77:4357-4369.

Suradhat S., Thanawongnuwech R., Poovorawan Y. (2003). Upregulation of IL-10 gene expression in porcine peripheral blood mononuclear cells by porcine reproductive and respiratory syndrome virus. J. Gen. Virol. 84: 453-459.

Thacker E.L., Halbur P.G., Ross R.F., Thanawongnuwech R., Thacker B.J. (1999). Mycoplasma hyopneumoniae potentiation of porcine reproductive and respiratory syndrome virus-induced pneumonia. J Clin Microbiol. 37(3): 620-627.

Thacker E.L., Thacker B.J., Young T.F., Halbur P.G. (2000). Effect of vaccination on the potentiation of porcine reproductive and respiratory syndrome virus (PRRSV)-induced pneumonia by Mycoplasma hyopneumoniae. Vaccine 18(13):1244-52. Toka F.N. et al. (2004). Molecular adjuvants for mucosal immunity. Immunol Rev. 199:100-112.

van der Most R.G., Spaan W.J.M. (1995). Coronavirus replication, transcription, and RNA recombination. In "The Coronaviridae" (S. G. Siddell, Ed.), pp. 11-31. Plenum Press, New York.

Vezina S.A., Loemba H., Fournier M., Dea S., Archambault D. (1996). Antibody production and blastogenic response in pigs experimentally infected with porcine reproductive and respiratory syndrome virus. Vet. Q. 13: 121-130.

Wensvoort G., Terpstra C., Boonstra J., Bloemraad M., Van Zaane D. (1986). Production of monoclonal antibodies against swine fever virus and their use in laboratory diagnosis. Vet. Microbiol. 12(2):101-108

Wensvoort G., Terpstra C., Pol J.M., ter Laak E.A., Bloemraad M., de Kluyver E.P., Kragten C., van Buiten L., den Besten A., Wagenaar F., et al. (1991). Mystery swine disease in The Netherlands: the isolation of Lelystad virus. Vet. Q. 13:121-130.

Wissink E.H.J., Kroeke M.V., van Wijk H.A.R., Rijsewijk F.A.M., Meulenberg J.J.M., Rottier P.J.M. (2005). Envelope protein requirements for the assembly of infectious virions of porcine reproductive and respiratory syndrome virus. J. virol. 79(19):12495-12506.

Yoon K.J., Zimmernan J.J., Swenson S.L., McGinley M.J., Eernisse K.A., Brevik A., Rhinehart L.L., Frey M.L., Hill H.T., Platt K.B. (1995). Characterization of the humoral immune response to porcine reproductive and respiratory syndrome (PRRSV) virus infection. J. Vet. Diagn. Invest. 7:305-312.

## Claims

1. Polypeptide encoded by SEQ ID NO: 13 or encoded by a sequence having a similarity of 90% to SEQ ID NO:13.

2. Polypeptide encoded by SEQ ID NO:14 or encoded by a sequence having a similarity of 90% to SEQ ID NO:14.

3. Pharmaceutical preparation comprising a polypeptide according to claim 1 or 2.

4. Pharmaceutical preparation according to claim 3 further comprising a pharmaceutically acceptable carrier, excipient and/or adjuvants.

5. Vaccine capable of protecting an animal against PRRSV comprising a polypeptide according to claim 1 or 2.

6. Vaccine according to claim 5, wherein the vaccine is a multivalent vaccine capable to provide protection against one or several pig pathogens other than PRRSV.

7. Vaccine according to claim 6, further comprising antigens derived from other viral and/or bacterial pathogens and/or proteins which will provide immunity against pathogens.

8. Vaccine according to claim 5 or 6, further comprising one or several antigens derived from other viral pathogens selected from Swine Influenza Viruses, Porcine Parvovirus, Porcine Circovirus Type 2, Classical Swine Fever, African Swine Fever, Foot-and-Mouth Disease, Pseudo-Rabies Virus, Porcine Circovirus Type 1, Porcine Adenoviruses, Porcine Enteroviruses, Porcine Respiratory Coronavirus, Porcine Rotavirus, Encephalomyocarditis Virus, Porcine Epidemic Diarrhea Virus, Blue Eye Disease Viruses, Hepatitis E Virus and/or West Nile Virus, Nipah virus.

9. Vaccine according to any one of claims 5 to 8, further comprising one or several antigens derived from other viral pathogens selected from Swine Influenza Viruses, Porcine Parvovirus, Porcine Circovirus Type 2, Classical Swine Fever, African Swine Fever and/or Foot-and-Mouth Disease.

10. Vaccine according to any one of claims 5 to 8, further comprising one or several antigens derived from bacterial pathogens selected from Mycoplasma hyopneumoniae, Actinobacillus pleuropneumoniae, Actinobacillus suis, Haemophilus parasuis, Pasteurella multocida type A (toxins), Pasteurella multocida type D (toxins), Bordetella bronchiseptica, Isospora suis, Brachyspira hyodysenteriae, Brachyspira pilosicoli, Lawsonia intracellularis, Erysipelothrix rhusiopathiae, Escherichia coli, Salmonella enterica, Mycoplasma hyorinis, Streptococcus suis, Clostridium perfringens, Clostridium difficile, Clostridium novyi, Brucella abortus and/or Candidatus helicobacter suis.

11. Vaccine according to any one of claims 5 to 8, further comprising one or several antigens derived from bacterial pathogens selected from Mycoplasma hyopneumoniae, Actinobacillus pleuropneumoniae, Actinobacillus suis, Haemophilus parasuis, Pasteurella multocida type A (toxins), Pasteurella multocida type D (toxins), Bordetella bronchiseptica, Isospora suis, Brachyspira hyodysenteriae, Brachyspira pilosicoli, Lawsonia intracellularis, Erysipelothrix rhusiopathiae, Escherichia coli and/or Salmonella enterica.

12. Vaccine according to any one of claims 5 to 11, wherein the further viral or bacterial antigen is present in the multivalent vaccine as an attenuated or inactivated virus or bacterium or as a nucleic acid sequence encoding one or several of the further viral or bacterial antigens.

**13.** Vaccine according to one of claims 5 to 12, wherein the vaccine is suitable for vaccinating a swine, preferably a sow.

**14.** Vaccine according to one of claims 5 to 13, wherein the vaccine is capable of inducing both a systemic immune response and a mucosal immune response against infectious viral agents.

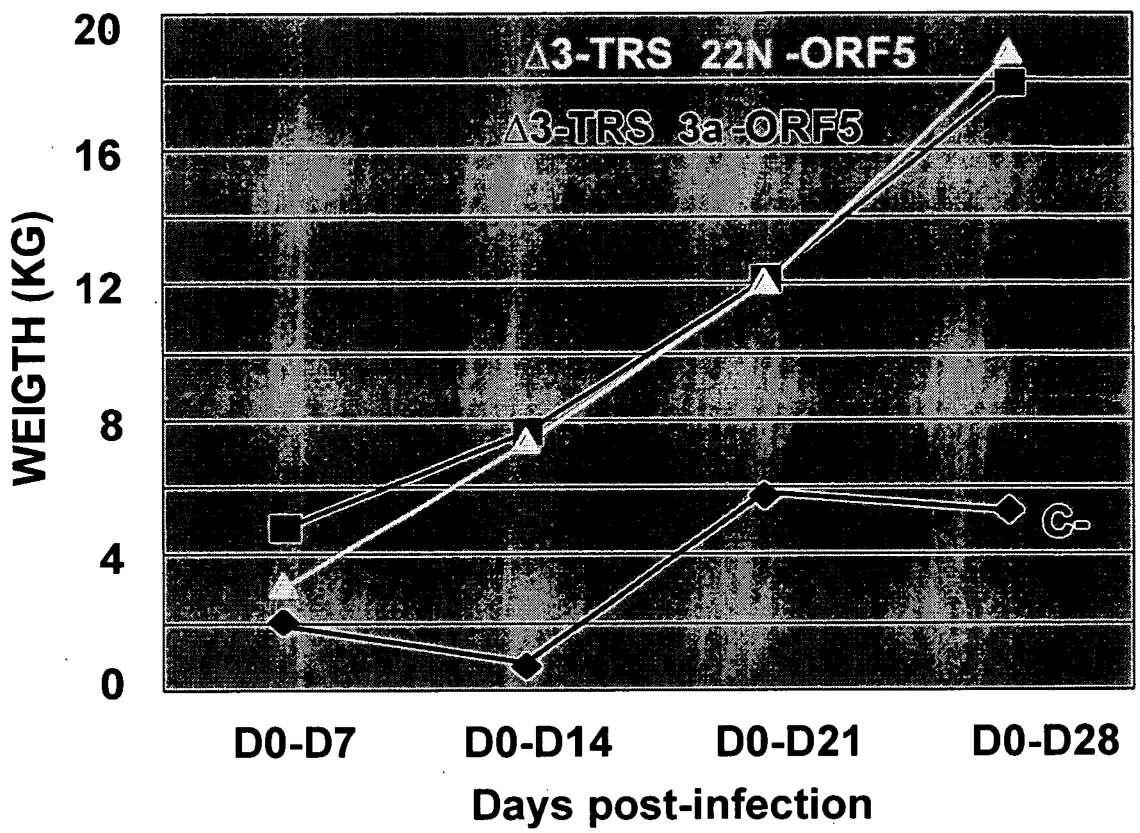
20
16
12
8
4
0
WEIGTH (KG)
Δ3-TRS 22N -ORF5
Δ3-TRS 3a -ORF5
C-
D0-D7
D0-D14
D0-D21
D0-D28
Days post-infection

Figure 1

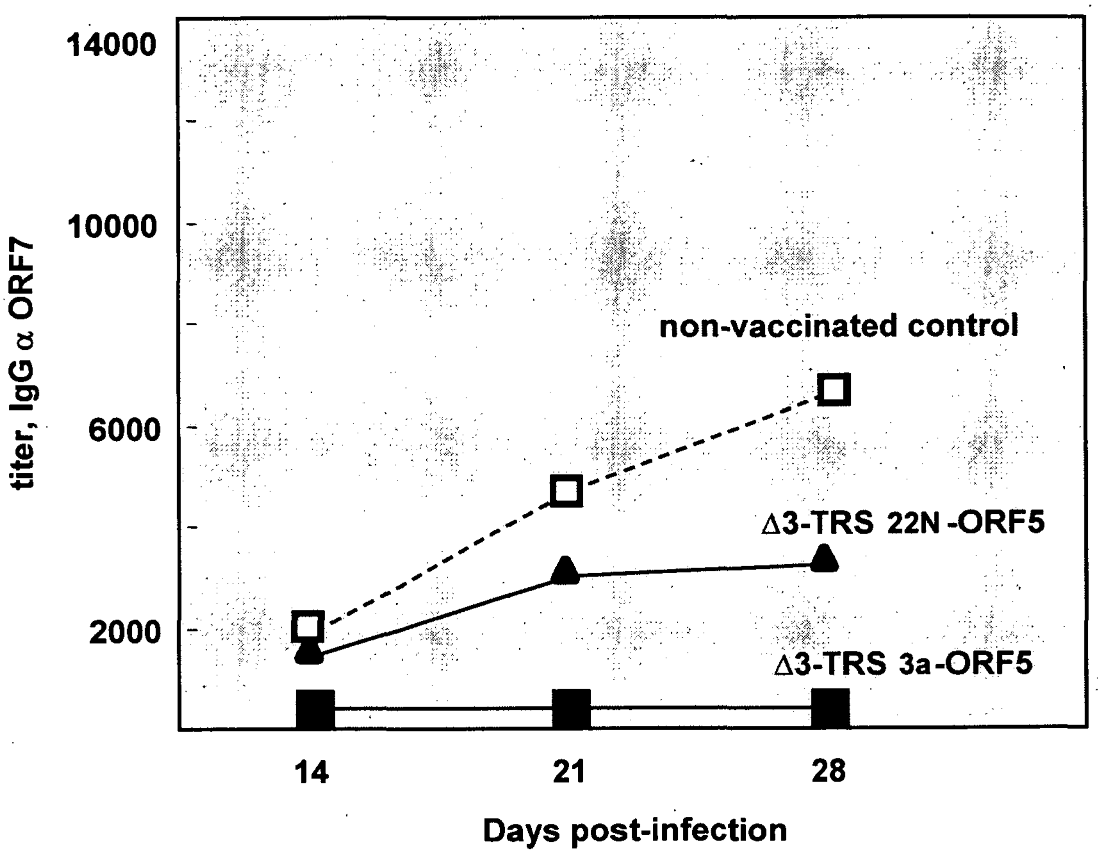
14000
10000
6000
2000
titer, IgG α ORF7
non-vaccinated control
Δ3-TRS 22N -ORF5
Δ3-TRS 3a-ORF5
14
21
28
Days post-infection

Figure 2

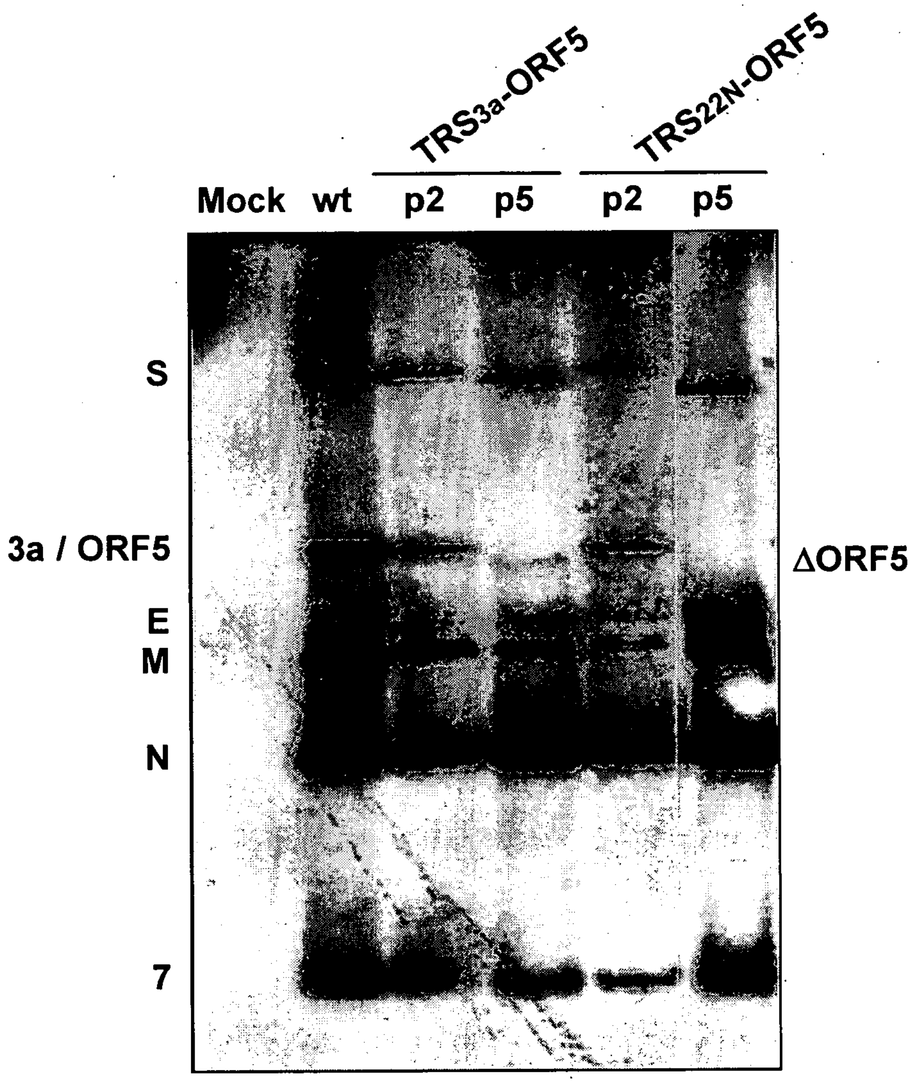


Figure 3

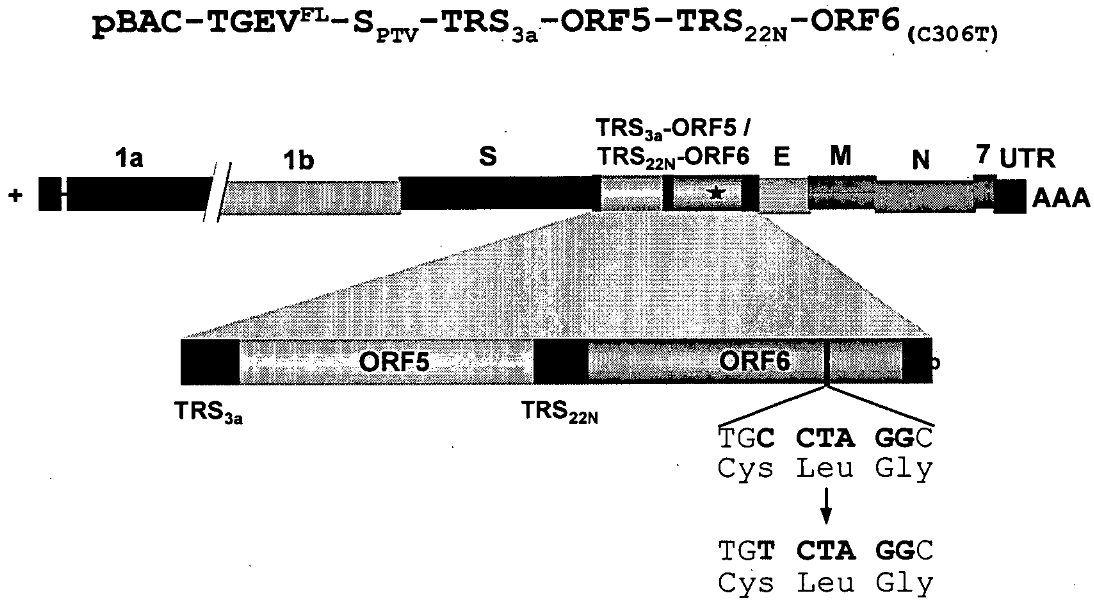
pBAC-TGEV^FL-S_PTV-TRS_3a-ORF5-TRS_22N-ORF6 (C306T)
1a
1b
S
TRS3a-ORF5 /
TRS22N-ORF6
E
M
N
7
UTR
+
AAA
ORF5
ORF6
TRS3a
TRS22N
TGC CTA GGC
Cys Leu Gly
TGT CTA GGC
Cys Leu Gly

Figure 4

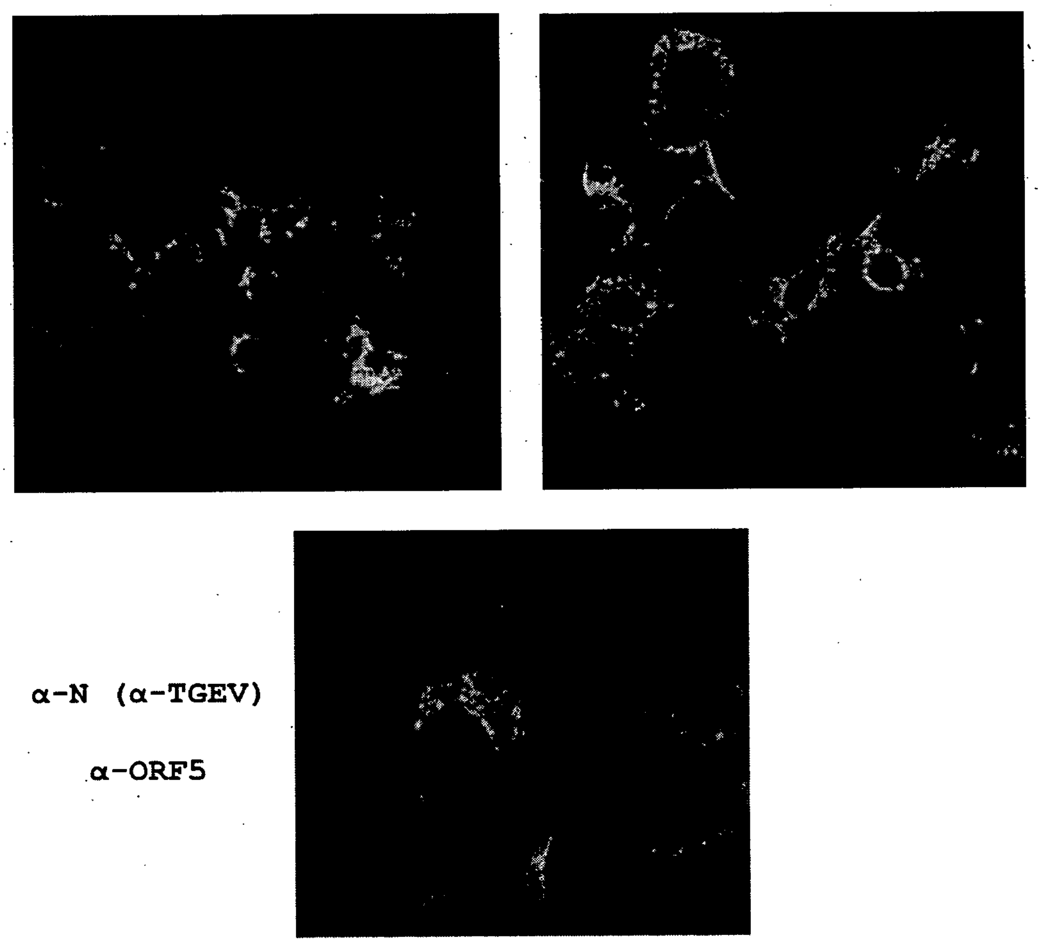

α-N (α-TGEV)

α-ORF5

Figure 5

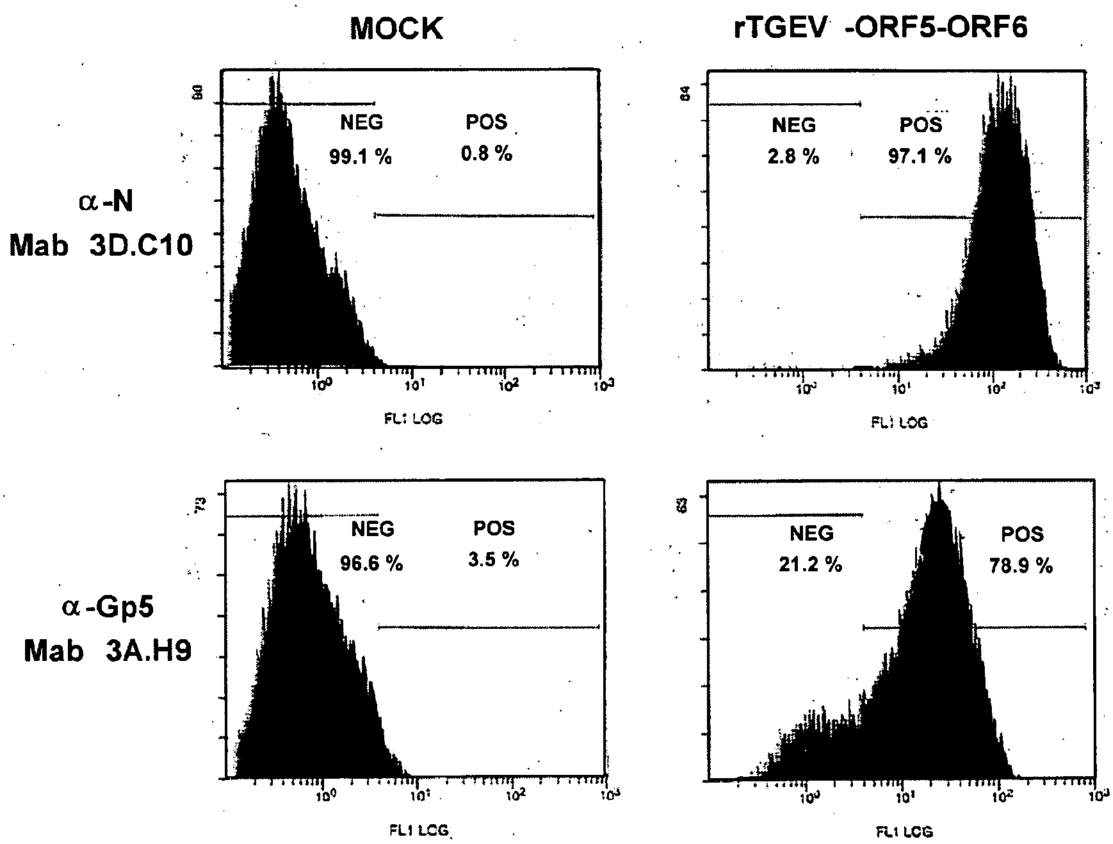
MOCK
rTGEV -ORF5-ORF6
α-N
Mab 3D.C10
α-Gp5
Mab 3A.H9
NEG
99.1 %
POS
0.8 %
NEG
2.8 %
POS
97.1 %
NEG
96.6 %
POS
3.5 %
NEG
21.2 %
POS
78.9 %
FL1 LOG

Figure 6

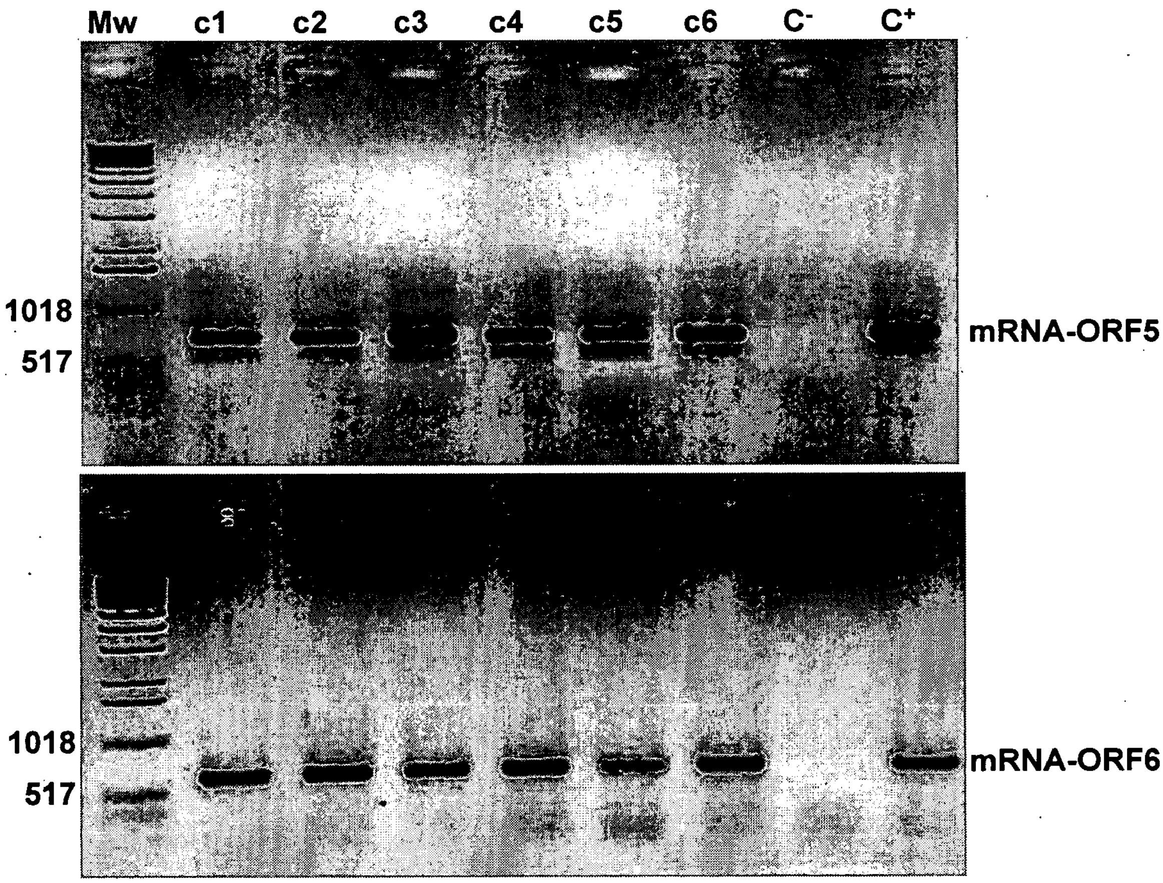


Figure 7

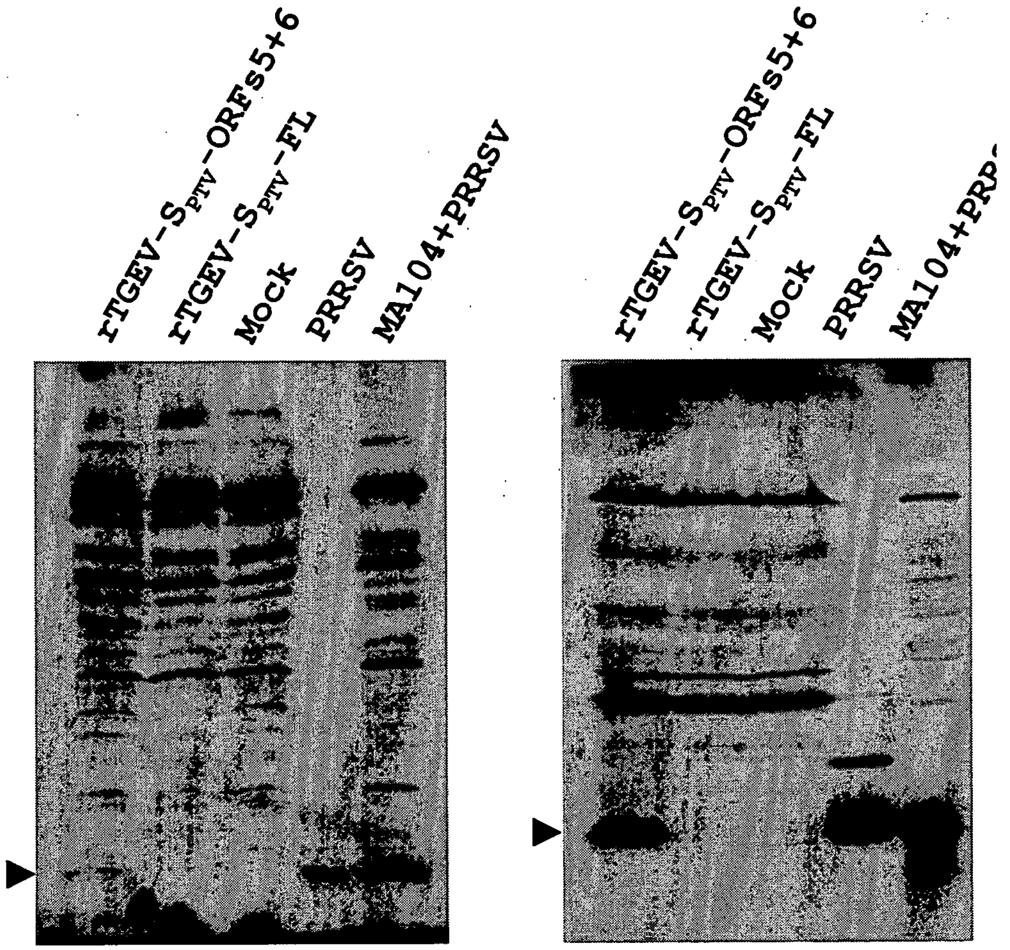
rTGEV-S$_{PTV}$-ORFs5+6
rTGEV-S$_{PTV}$-FL
Mock
PRRSV
MA104+PRRSV
rTGEV-S$_{PTV}$-ORFs5+6
rTGEV-S$_{PTV}$-FL
Mock
PRRSV
MA104+PRR

Figure 8

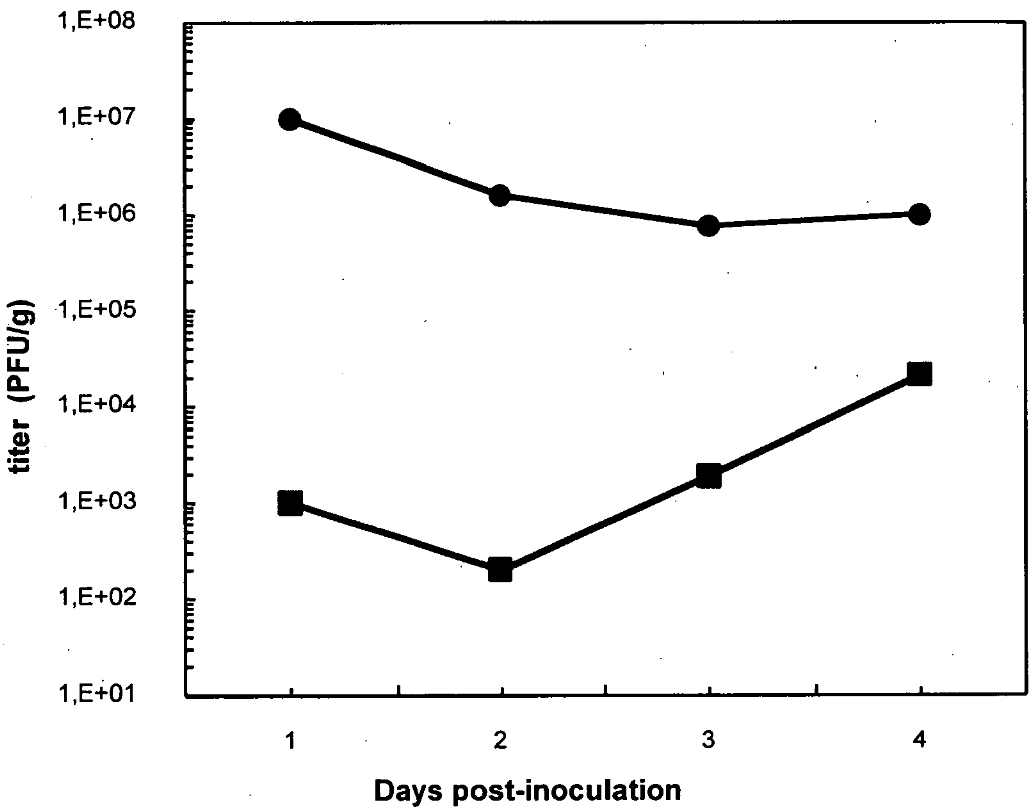
1,E+08
1,E+07
1,E+06
1,E+05
1,E+04
1,E+03
1,E+02
1,E+01
titer (PFU/g)
1
2
3
4
Days post-inoculation

Figure 9

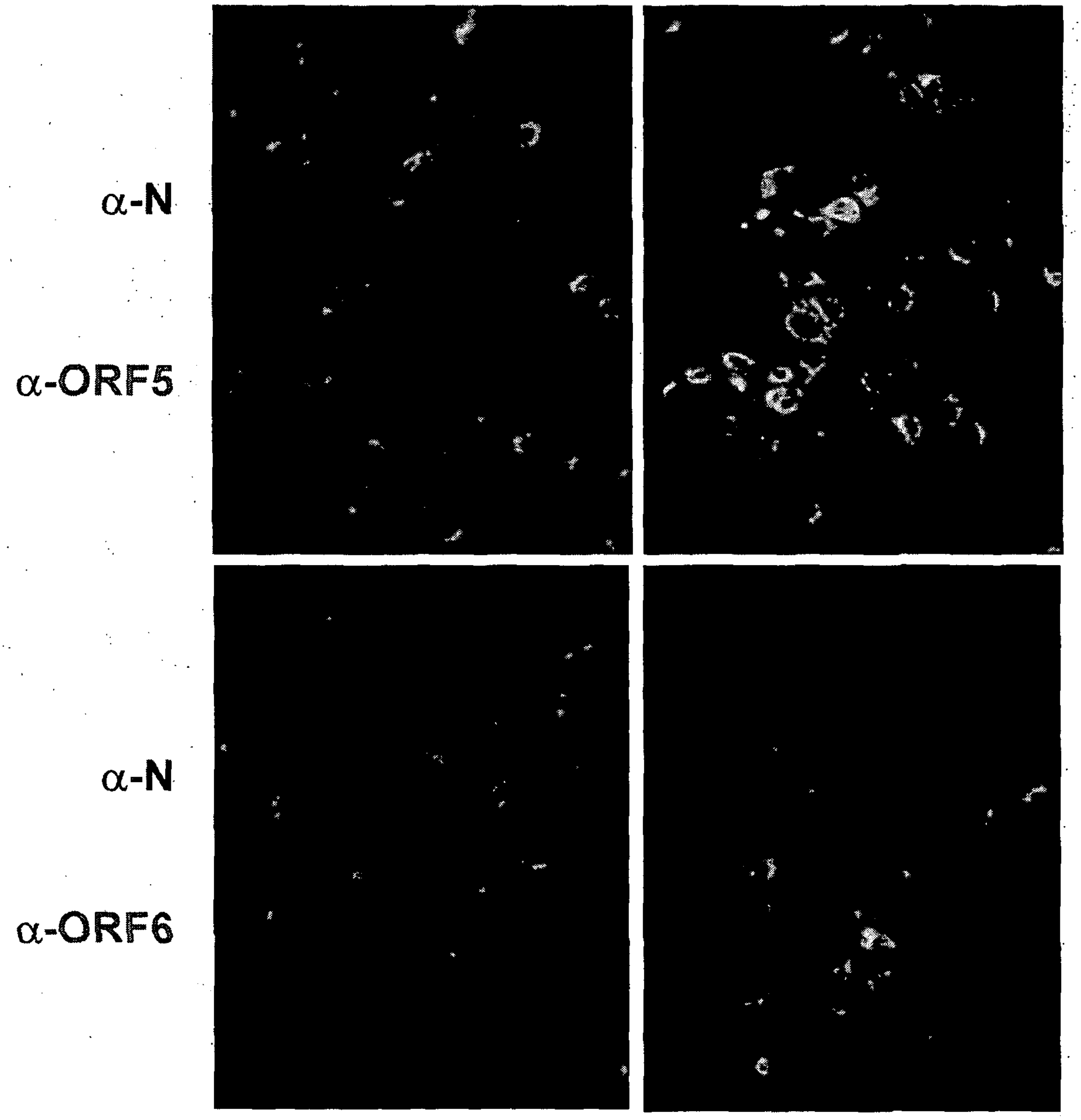


Figure 10

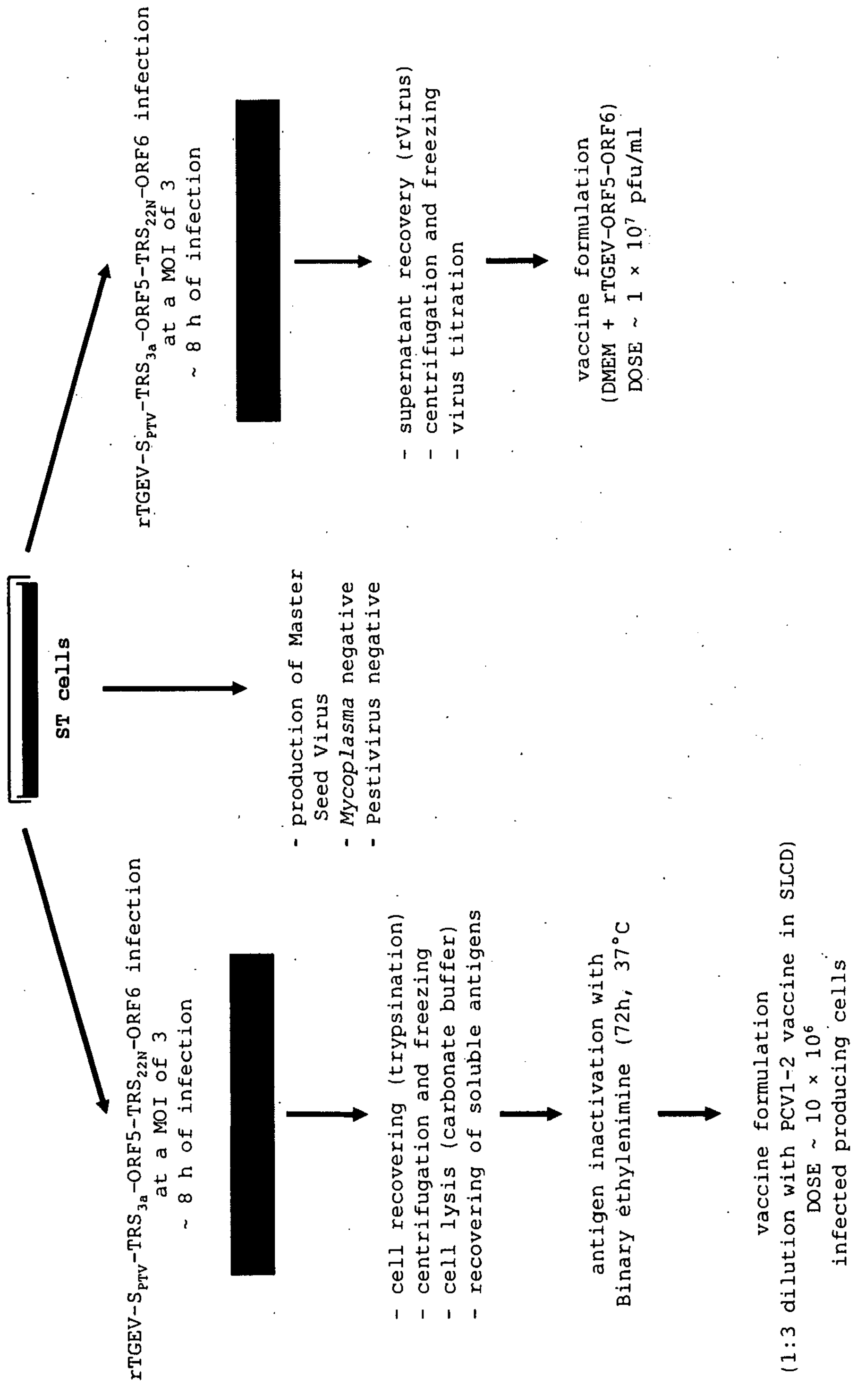
ST cells
- production of Master Seed Virus
- *Mycoplasma* negative
- Pestivirus negative
rTGEV-$S_{PTV}$-$TRS_{3a}$-ORF5-$TRS_{22N}$-ORF6 infection
at a MOI of 3
~ 8 h of infection
- cell recovering (trypsination)
- centrifugation and freezing
- cell lysis (carbonate buffer)
- recovering of soluble antigens
antigen inactivation with
Binary ethylenimine (72h, 37°C
vaccine formulation
(1:3 dilution with PCV1-2 vaccine in SLCD)
DOSE ~ 10 × $10^6$
infected producing cells
rTGEV-$S_{PTV}$-$TRS_{3a}$-ORF5-$TRS_{22N}$-ORF6 infection
at a MOI of 3
~ 8 h of infection
- supernatant recovery (rVirus)
- centrifugation and freezing
- virus titration
vaccine formulation
(DMEM + rTGEV-ORF5-ORF6)
DOSE ~ 1 × $10^7$ pfu/ml

**Figure 11**

| Histhopatological Lesions | | | | | | Complementary studies | | | |
|---|---|---|---|---|---|---|---|---|---|
| Pig reference | Pulmonary Oedema | Pulmonary Congestion | Interstitial pneumonia | | | TGEV IHC | PRRSV IHC | Lung lesions pneumonic score | Virus Titer |
| | | | Alveolar Wall thickening | Alveolar Mononuclear exudate | Alveolar PMNN exudate | | | | |
| Control Group (Group # 3) | | | | | | | | | |
| 0001(96 h.p.i) | - | - | - | - | - | - | - | 0 | 0 |
| 0002(96 h.p.i) | - | - | - | - | - | - | - | 0 | 0 |
| 0003(96 h.p.i) | - | - | - | - | - | - | - | 0 | 0 |
| rTGEV-SPTV-FL (Group # 1) | | | | | | | | | |
| 0004(24 h.p.i) | - | - | - | - | - | - | - | 2,143 | $1x10^5$ pfu/ml |
| 0005(96 h.p.i) | - | - | - | - | - | - | - | 0 | 0 |
| 0006(48 h.p.i) | - | + | - | - | - | - | - | 0 | 0 |
| 0007(72 h.p.i) | - | + | ++ | ++ | - | + | - | 1,240 | $6x10^2$ pfu/ml |
| 0008(72 h.p.i) | - | + | +++ | +++ | - | + | - | 2,143 | $2x10^3$ pfu/ml |
| 0009(96 h.p.i) | - | + | +++ | +++ | - | + | - | 1,339 | $1x10^5$ pfu/ml |
| 0010(48 h.p.i) | - | + | +++ | +++ | - | + | - | 1,691 | $2x10^3$ pfu/ml |
| rTGEV-SPTVTRS3a-ORF5-TRS22N-ORF6 (Group # 2) | | | | | | | | | |
| 0011(48 h.p.i) | - | + | ++ | ++ | - | + | - | 1,428 | $7x10^4$ pfu/ml |
| 0012(96 h.p.i) | - | - | - | - | - | - | - | 0 | 0 |
| 0013(72 h.p.i) | - | - | - | - | - | - | - | 0 | $7x10^3$ pfu/ml |
| 0014(96 h.p.i) | - | - | +++ | +++ | - | +++ | - | 2,316 | $7x10^4$ pfu/ml |
| 0015(24 h.p.i) | - | - | - | - | - | - | - | 0 | $1x10^4$ pfu/ml |
| 0016(72 h.p.i) | + | + | ++ | ++ | - | +++ | - | 2,678 | $1x10^3$ pfu/ml |
| 0017(48 h.p.i) | - | - | ++ | ++ | - | +++ | - | 3,031 | $1x10^4$ pfu/ml |

Figure 12

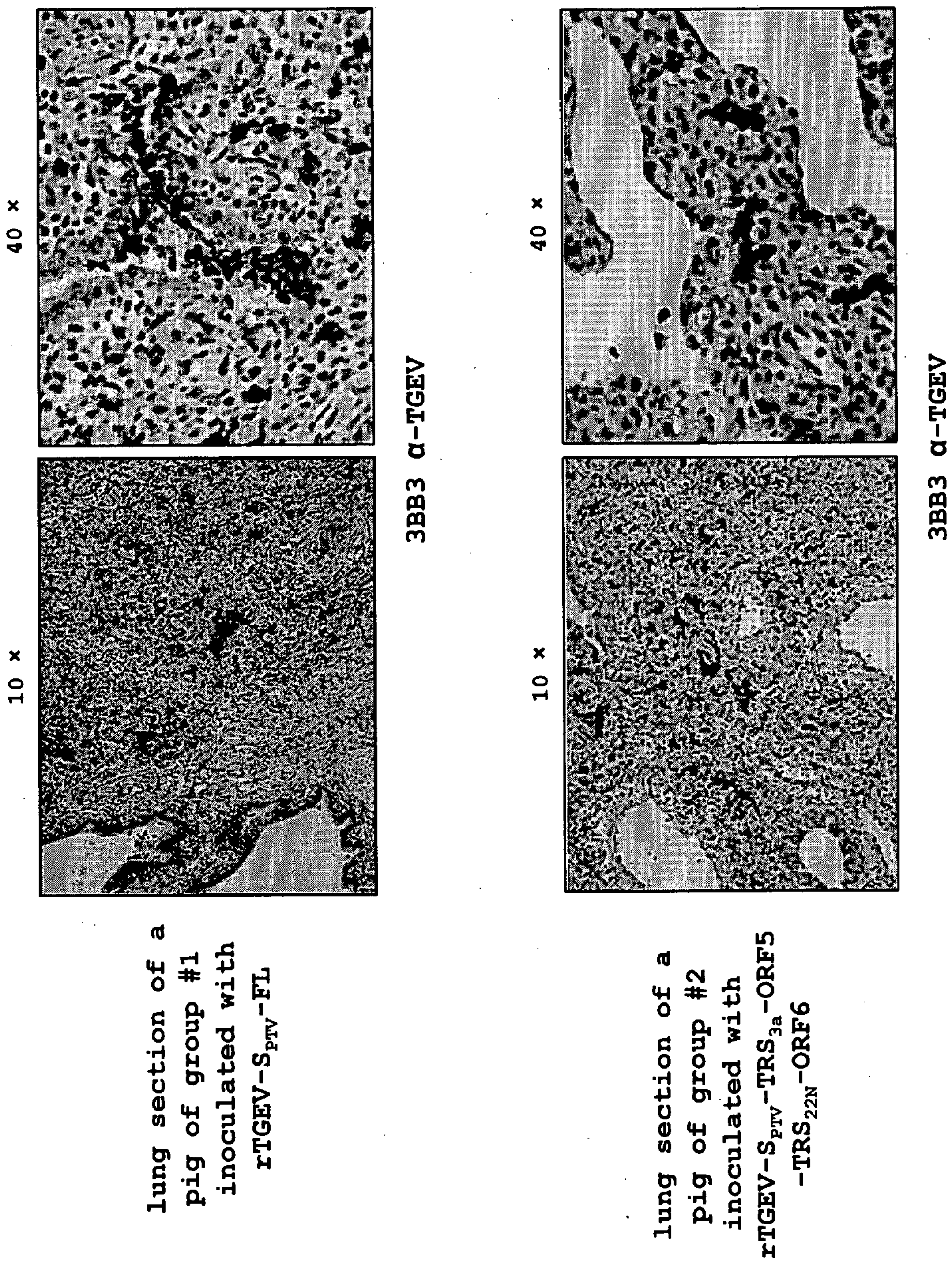
40 x
10 x
3BB3 α-TGEV
lung section of a pig of group #1 inoculated with rTGEV-S$_{PTV}$-FL
40 x
10 x
3BB3 α-TGEV
lung section of a pig of group #2 inoculated with rTGEV-S$_{PTV}$-TRS$_{3a}$-ORF5 -TRS$_{22N}$-ORF6

Figure 13

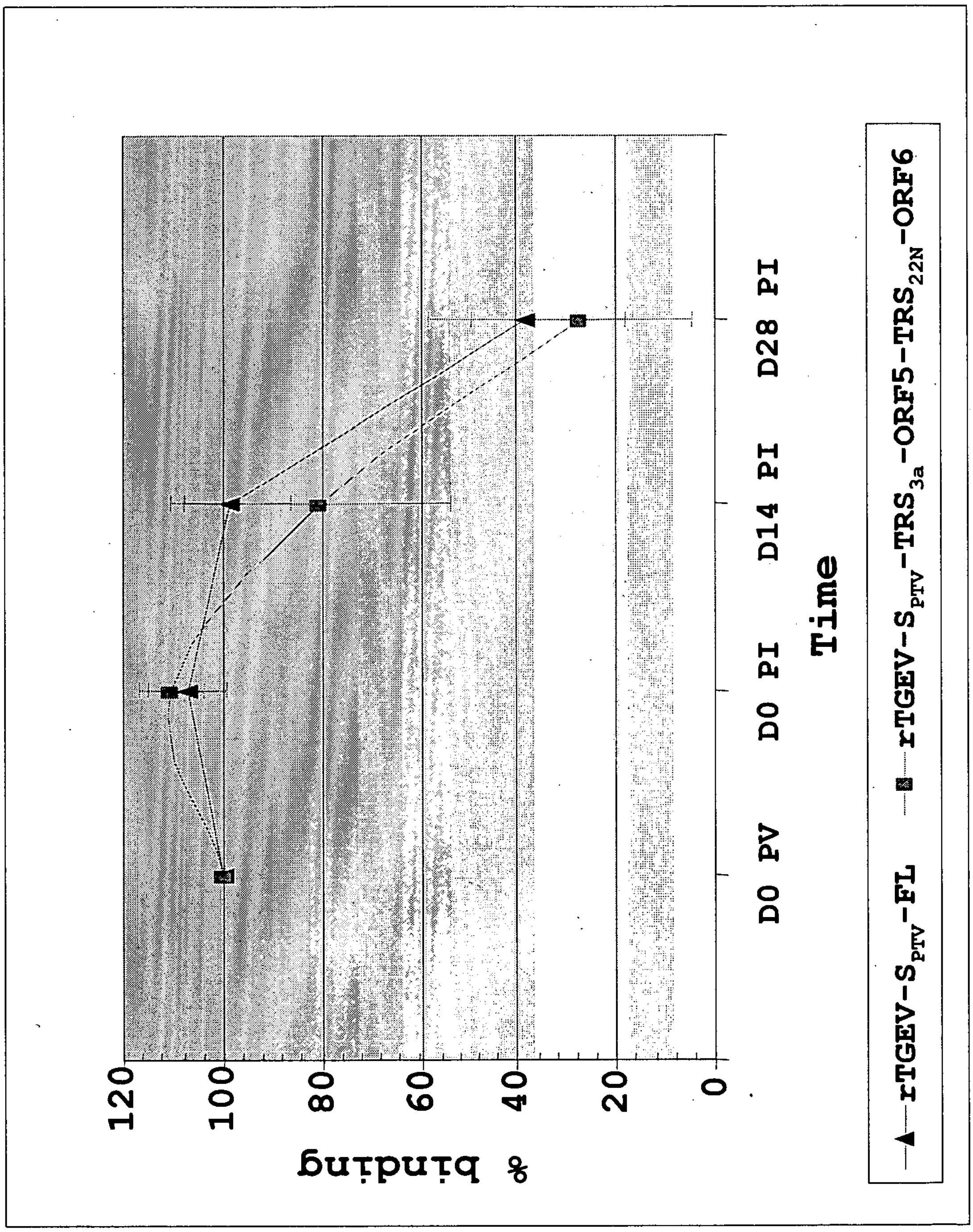
% binding
120
100
80
60
40
20
0
D0 PV
D0 PI
D14 PI
D28 PI
Time
rTGEV-$S_{PTV}$-FL
rTGEV-$S_{PTV}$-$TRS_{3a}$-ORF5-$TRS_{22N}$-ORF6

Figure 14

| IPMA anti-PRRSV | | | | | | | |
|---|---|---|---|---|---|---|---|
| Group # 1 rTGEV-$S_{PTV}$-FL | | | | Group # 2 rTGEV-$S_{PTV}$-$TRS_{3a}$-ORF5-$TRS_{22N}$-ORF6 | | | |
| Pig reference | D0PI | D14PI | D28PI | Pig reference | D0PI | D14PI | D28PI |
| 307 | 0 | 0 | 160 | 324 | 0 | 0 | 160 |
| 308 | 0 | 0 | 80 | 325 | 0 | 0 | 160 |
| 309 | 0 | 0 | 160 | 326 | 0 | 0 | 160 |
| 310 | 0 | 0 | 0 | 327 | 0 | 0 | 160 |
| 311 | 0 | 0 | 80 | 328 | 0 | 0 | 80 |
| 312 | 0 | 0 | 80 | 329 | 0 | 160 | 80 |
| 313 | 0 | 0 | 80 | 330 | 0 | 0 | 80 |
| 314 | 0 | 0 | 160 | 331 | 0 | 160 | 80 |
| 315 | 0 | 0 | 160 | 332 | 0 | 0 | 0 |
| 316 | 0 | 0 | 80 | 333 | 0 | 0 | 160 |
| 317 | 0 | 0 | 80 | 334 | 0 | 80 | 80 |
| 318 | 0 | 0 | 0 | 335 | 0 | 80 | 80 |
| 319 | 0 | 0 | 0 | ----- | ----- | ----- | ----- |
| 320 | 0 | 0 | 80 | 337 | 0 | 0 | 0 |
| 321 | 0 | 0 | 0 | 338 | 0 | 80 | 320 |
| 322 | 0 | 0 | 80 | 339 | 0 | 0 | 160 |
| 323 | 0 | 0 | 160 | 340 | 0 | 0 | 80 |

| | |
|---|---|
| Control + | 320-640 |
| Control - | 0 |

Figure 15

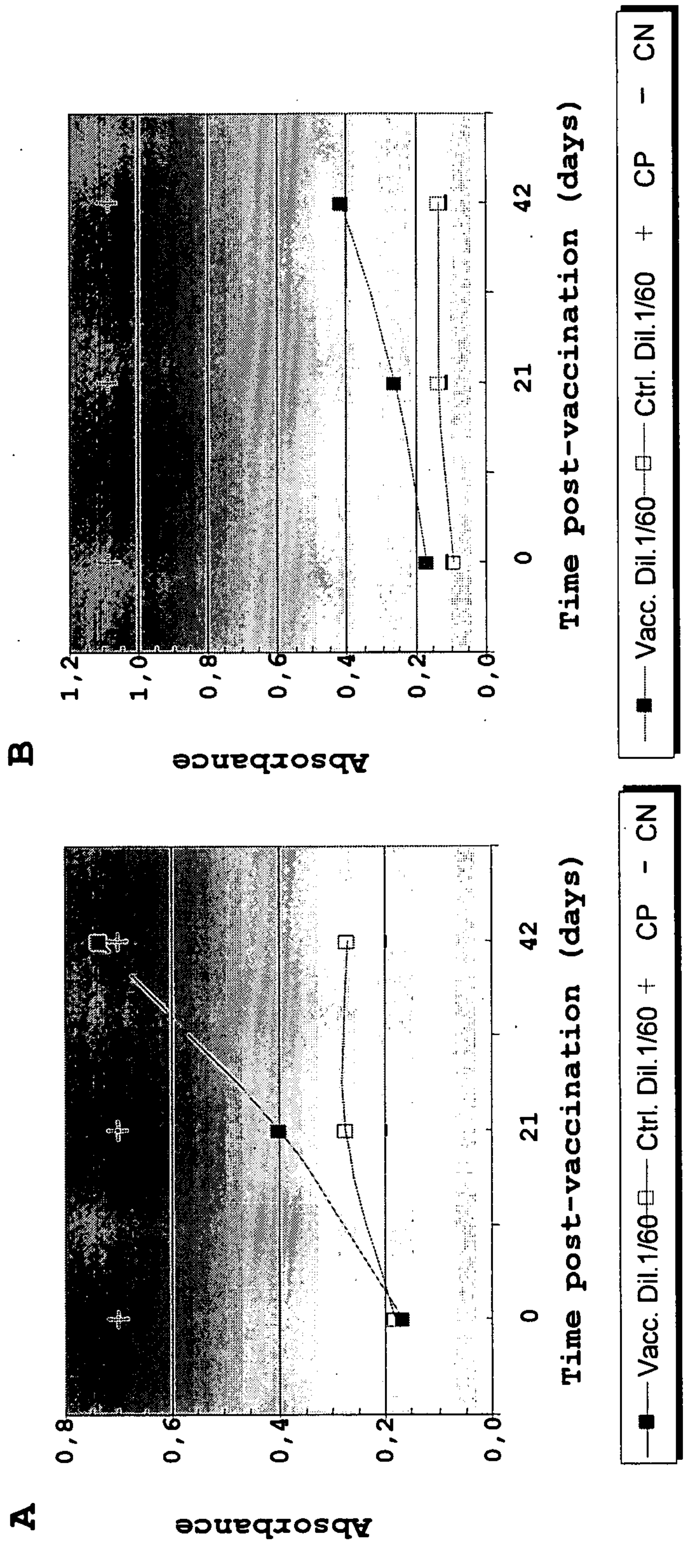
A
Absorbance
0,8
0,6
0,4
0,2
0,0
0
21
42
Time post-vaccination (days)
Vacc. Dil.1/60
Ctrl. Dil.1/60
CP
CN
B
Absorbance
1,2
1,0
0,8
0,6
0,4
0,2
0,0
0
21
42
Time post-vaccination (days)
Vacc. Dil.1/60
Ctrl. Dil.1/60
CP
CN

Figure 16

**A**

| | % of anti-TGEV antibodies | | | % of anti-PRRSV antibodies | | |
|---|---|---|---|---|---|---|
| Group #1 vaccinated animals | D0 PV | D21 PV | D42 PV | D0 PV | D21 PV | D42 PV |
| | 0,0 | 57,1 | 100,0 | 0,0 | 71,4 | 83,3 |
| Group #2 non-vaccinated control animals | D0 PV | D21 PV | D42 PV | D0 PV | D21 PV | D42 PV |
| | 0,0 | 7,7 | 15,4 | 0,0 | 15,4 | 15,4 |

**B** **C**

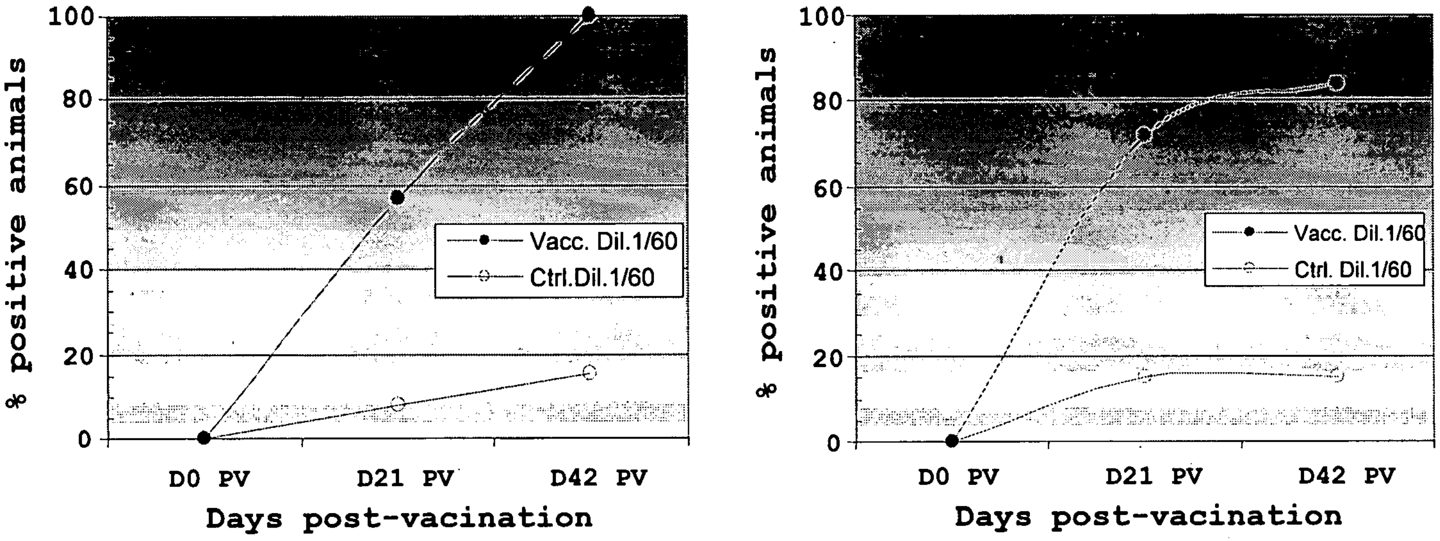


Figure 17

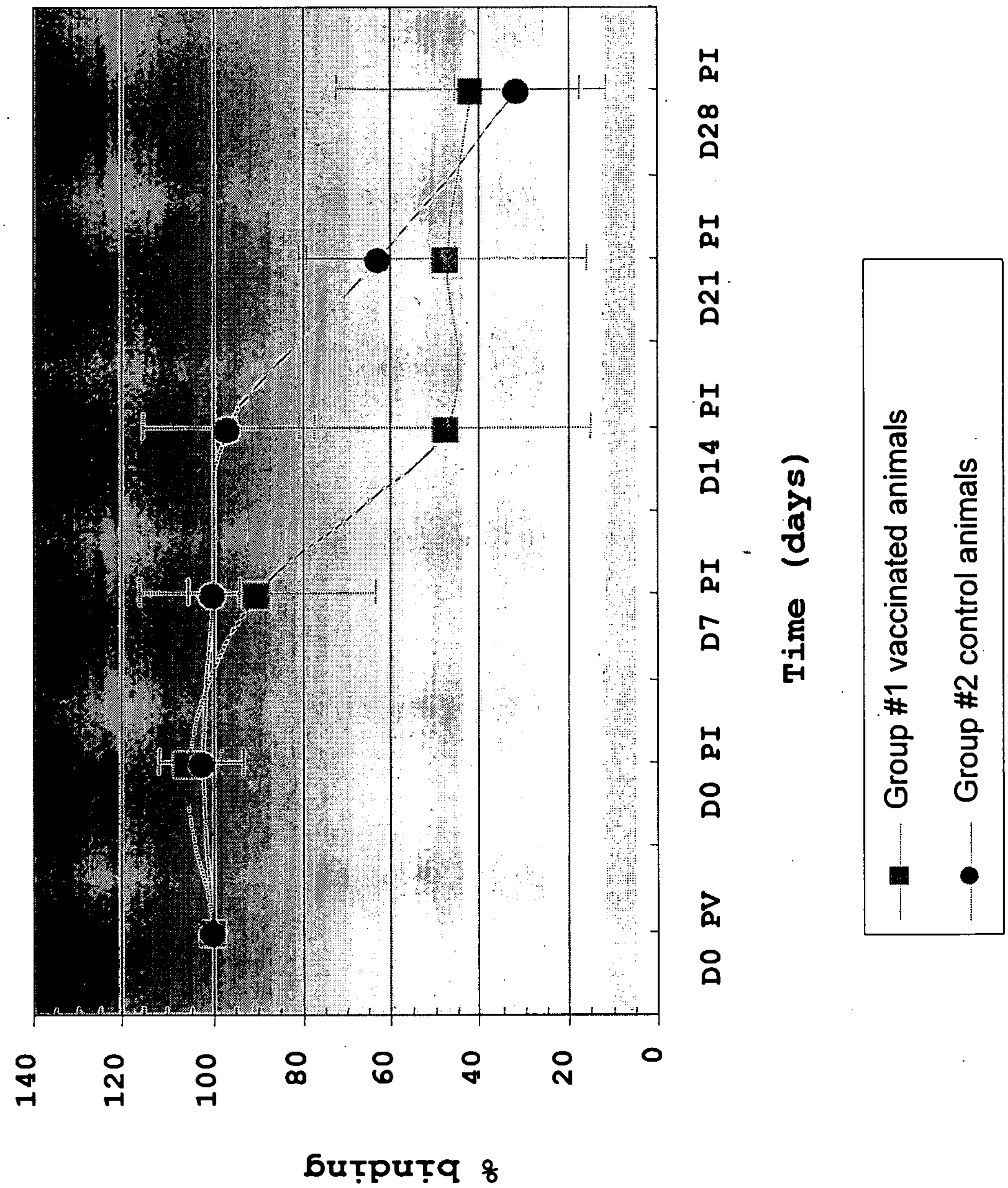
% binding
140
120
100
80
60
40
20
0
D0 PV
D0 PI
D7 PI
D14 PI
D21 PI
D28 PI
Time (days)
Group #1 vaccinated animals
Group #2 control animals

Figure 18

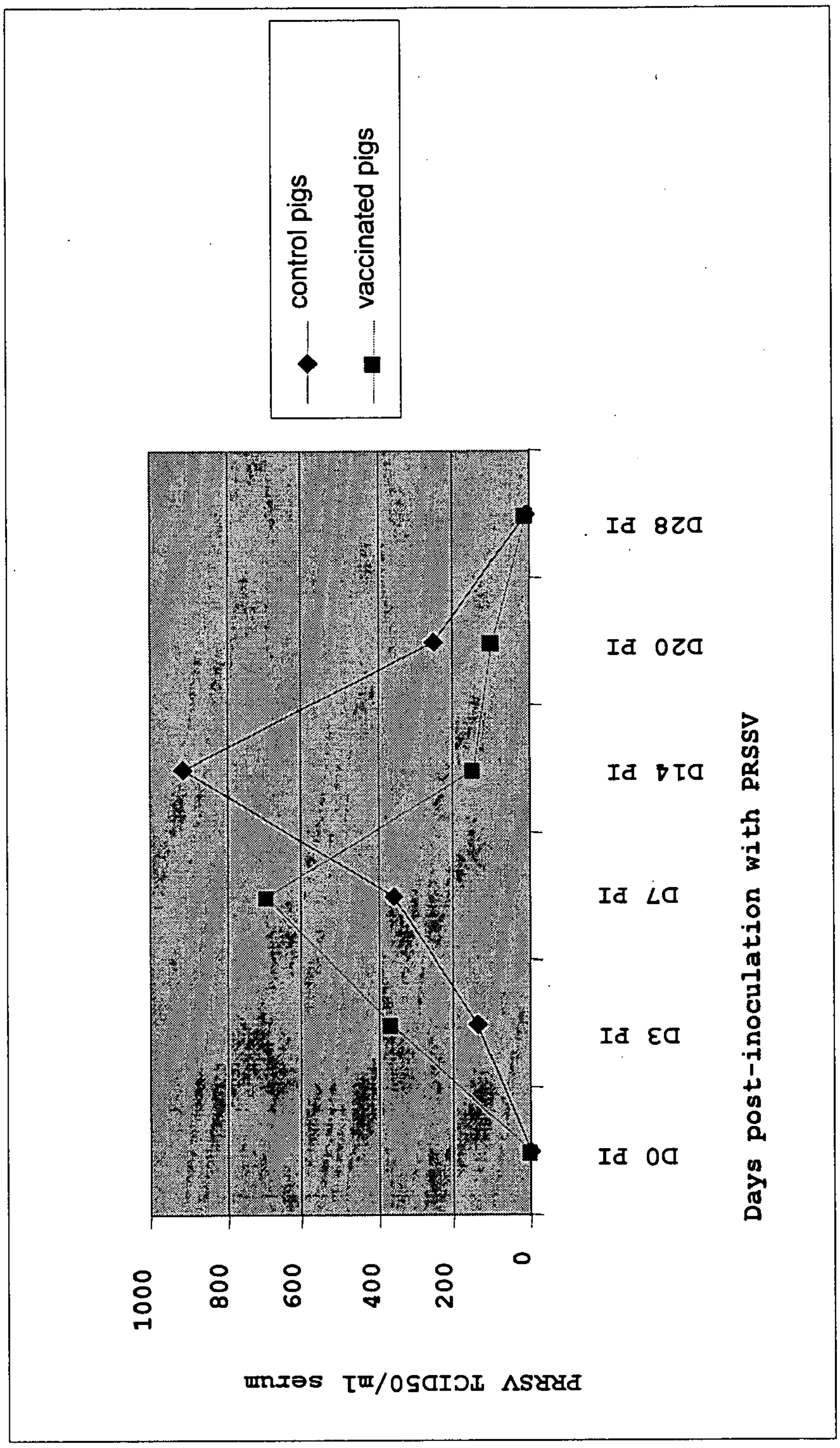
control pigs
vaccinated pigs
PRRSV TCID50/ml serum
1000
800
600
400
200
0
D0 PI
D3 PI
D7 PI
D14 PI
D20 PI
D28 PI
Days post-inoculation with PRSSV

**Figure 19**

rTGEV-Δ3-$TRS_{3a}$-ORF5-LIMP-II

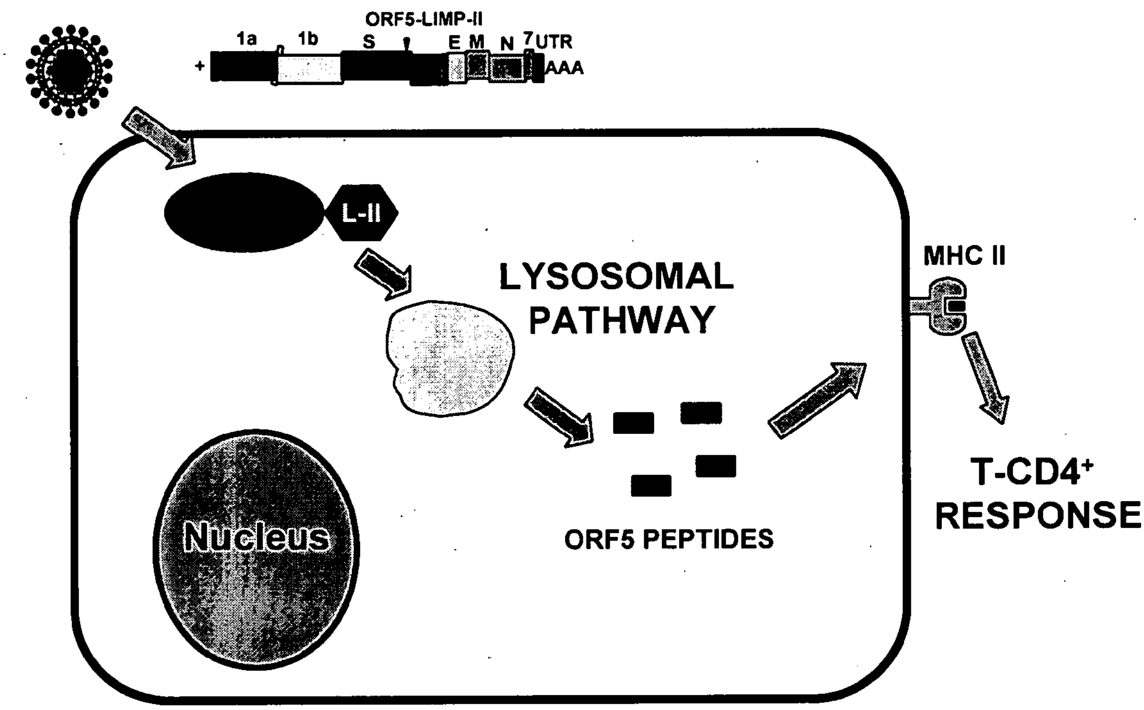


Figure 20

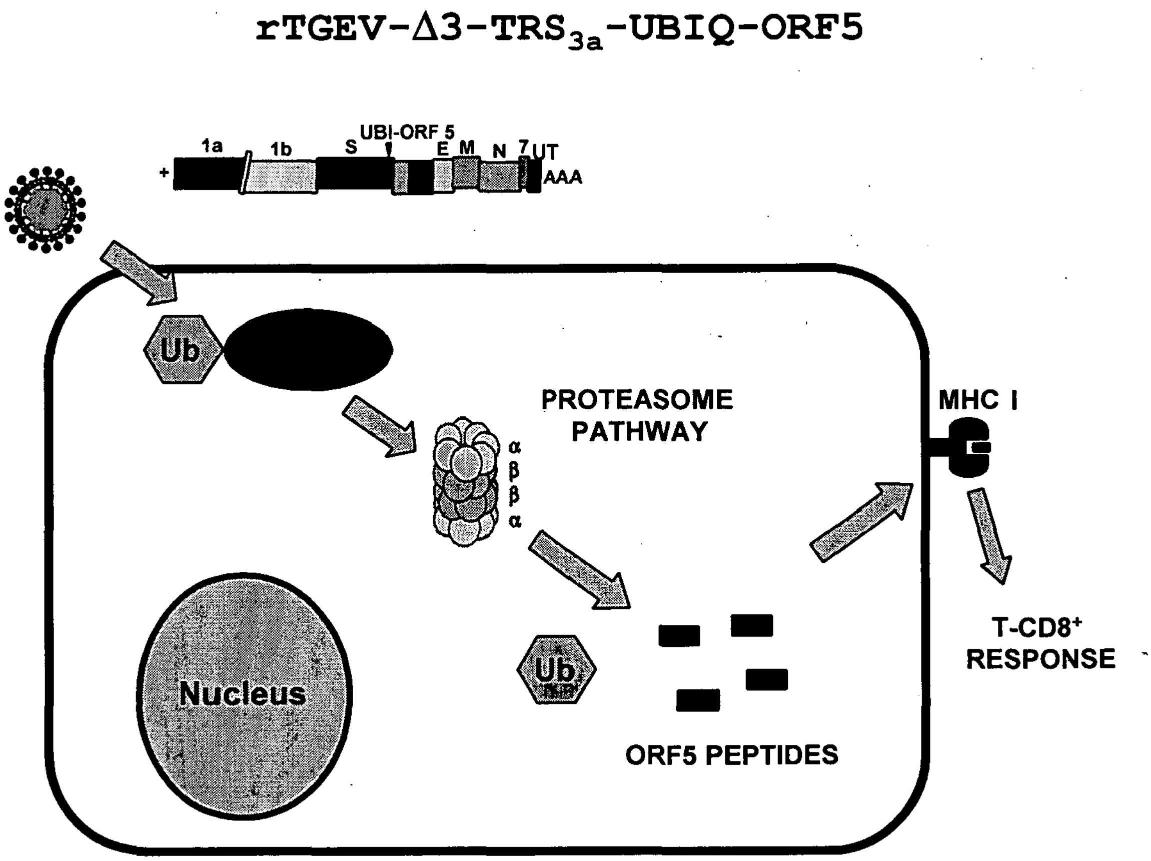
rTGEV-Δ3-TRS3a-UBIQ-ORF5
UBI-ORF 5
1a
1b
S
E
M
N
7
UT
AAA
+
Ub
PROTEASOME
PATHWAY
α
β
β
α
MHC I
T-CD8+
RESPONSE
Nucleus
Ub
ORF5 PEPTIDES

Figure 21

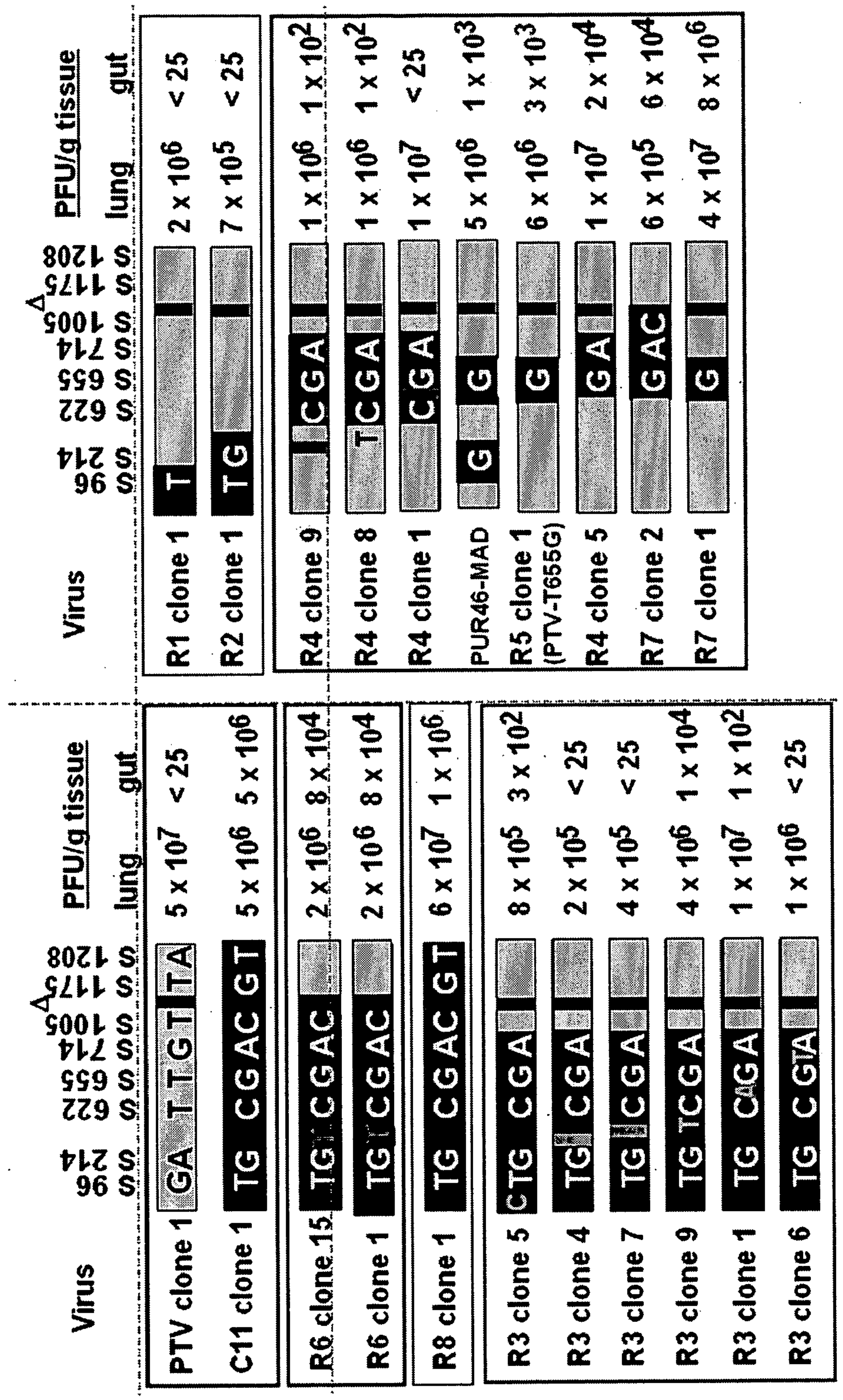

Virus
S 96
S 214
S 622
S 655
S 714
S 1005
Δ
S 1175
S 1208
PFU/g tissue
lung
gut
PTV clone 1
C11 clone 1
R6 clone 15
R6 clone 1
R8 clone 1
R3 clone 5
R3 clone 4
R3 clone 7
R3 clone 9
R3 clone 1
R3 clone 6
R1 clone 1
R2 clone 1
R4 clone 9
R4 clone 8
R4 clone 1
PUR46-MAD
R5 clone 1 (PTV-T655G)
R4 clone 5
R7 clone 2
R7 clone 1


Figure 22

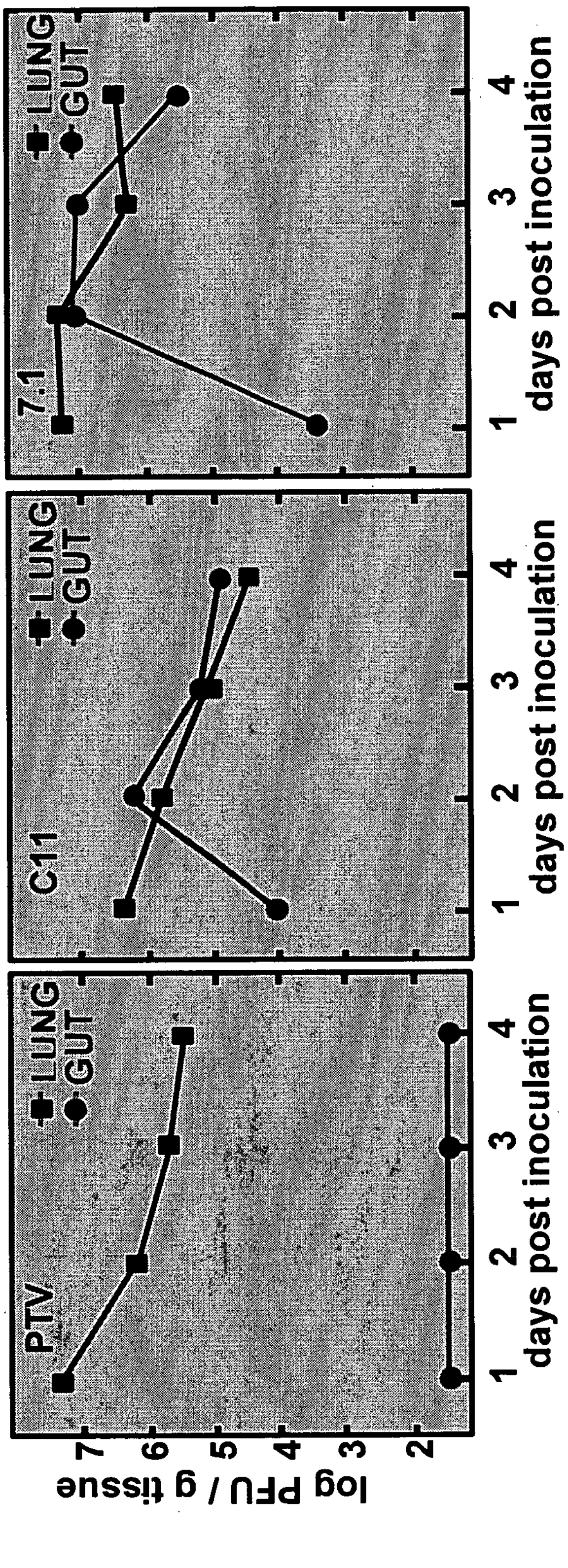
PTV
C11
7.1
LUNG
GUT
log PFU / g tissue
7 6 5 4 3 2
1 2 3 4
days post inoculation

Figure 23

Europäisches Patentamt
European Patent Office
Office européen des brevets

# EUROPEAN SEARCH REPORT

Application Number
EP 10 01 3126

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | WO 01/39797 A (CONSEJO SUPERIOR DE INVESTIGACIONES CIENTIFICAS; ENJUANES SANCHEZ, LUI) 7 June 2001 (2001-06-07)<br>* the whole document *<br>----- | 1-14 | INV.<br>C12N15/86<br>A61K39/215<br>A61K39/12 |
| A | PLANA-DURAN J ET AL: "New strategies on vaccinology: use of the transmissible gastroenteritis virus as a vector for swine diseases",<br>INTERNATIONAL SYMPOSIUM ON EMERGING AND RE-EMERGING PIG DISEAESES,<br>2003, pages 115-116, XP002324855,<br>* the whole document *<br>----- | 1-14 | |
| A | WO 02/092827 A (UNIV UTRECHT [NL]; STICHTING TECH WETENSCHAPP [NL]; ROTTIER PETRUS JOS) 21 November 2002 (2002-11-21)<br>* page 67, line 1 - line 20 *<br>----- | 1-14 | |
| A,P | WO 2006/024542 A (CONSEJO SUPERIOR INVESTIGACION [ES]; FORT DODGE VETERINARIA S A [ES];) 9 March 2006 (2006-03-09)<br>* the whole document *<br>----- | 1-14 | TECHNICAL FIELDS SEARCHED (IPC)<br>C12N<br>A61K |
| A | ENJUANES L ET AL: "CORONAVIRUS REVERSE GENETICS AND DEVELOPMENT OF VECTORS FOR GENE EXPRESSION",<br>CURRENT TOPICS IN MICROBIOLOGY AND IMMUNOLOGY, SPRINGER, BERLIN,, DE,<br>vol. 287, 2005, pages 161-197, XP008044764,<br>ISSN: 0070-217X<br>* page 179, last paragraph - page 186 *<br>-----<br>-/-- | 1-14 | |

The present search report has been drawn up for all claims

1

| Place of search | Date of completion of the search | Examiner |
|---|---|---|
| The Hague | 22 March 2011 | Piret, Bernard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 10 01 3126

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | LUIS ENJUAMES ET AL: "VIRUS BASED VECTORS FOR GENE EXPRESSION IN MAMMALIAN CELLS: CORONAVIRUS", GENE TRANSFER AND EXPRESSION IN MAMMALIAN CELLS, ELSEVIER SCIENCE, AMSTERDAM, NL, 2003, pages 151-158, XP008075797, ----- | 1-14 | |
| A | EP 1 008 652 A (CYANAMID IBERICA, SA) 14 June 2000 (2000-06-14) ----- | 1-14 | |
| X | WO 01/89559 A (MERIAL SAS [FR]) 29 November 2001 (2001-11-29) * the whole document * ----- | 1-14 | |
| X | BASTOS R G ET AL: "Immune response of pigs inoculated with Mycobacterium bovis BCG expressing a truncated form of GP5 and M protein of porcine reproductive and respiratory syndrome virus", VACCINE, BUTTERWORTH SCIENTIFIC. GUILDFORD, GB, vol. 22, no. 3-4, 2 January 2004 (2004-01-02), pages 467-474, XP004479368, ISSN: 0264-410X * the whole document * ----- | 1-14 | TECHNICAL FIELDS SEARCHED (IPC) |
| A | XUE Q ET AL: "Immune responses of swine following DNA immunization with plasmids encoding porcine reproductive and respiratory syndrome virus ORFs 5 and 7, and porcine IL-2 and IFNgamma", VETERINARY IMMUNOLOGY AND IMMUNOPATHOLOGY, AMSTERDAM, NL, vol. 102, no. 3, 8 December 2004 (2004-12-08), pages 291-298, XP004613296, ISSN: 0165-2427 ----- -/-- | 1-14 | |

The present search report has been drawn up for all claims

1

| Place of search | Date of completion of the search | Examiner |
|---|---|---|
| The Hague | 22 March 2011 | Piret, Bernard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 10 01 3126

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | HILGERS L A T ET AL: "Sulfolipo-cyclodextrin in squalane-in-water as a novel and safe vaccine adjuvant", VACCINE, BUTTERWORTH SCIENTIFIC. GUILDFORD, GB, vol. 17, no. 3, February 1999 (1999-02), pages 219-228, XP004144730, ISSN: 0264-410X<br>----- | 1-14 | |
| A | WO 99/18214 A (MERIAL SAS [FR]; UNIV BELFAST [GB]; UNIV SASKATCHEWAN [CA]) 15 April 1999 (1999-04-15)<br>----- | 1-14 | |
| A,D | RODRIGUEZ F ET AL: "CD4(+) T cells induced by a DNA vaccine: immunological consequences of epitope-specific lysosomal targeting.", JOURNAL OF VIROLOGY NOV 2001, vol. 75, no. 21, November 2001 (2001-11), pages 10421-10430, XP002423899, ISSN: 0022-538X<br>----- | 1-14 | TECHNICAL FIELDS SEARCHED (IPC) |
| A | PLAGEMANN P G W: "The primary GP5 neutralization epitope of North American isolates of porcine reproductive and respiratory syndrome virus", VETERINARY IMMUNOLOGY AND IMMUNOPATHOLOGY, AMSTERDAM, NL, vol. 102, no. 3, 8 December 2004 (2004-12-08), pages 263-275, XP004613294, ISSN: 0165-2427<br>-----<br>-/-- | 1-14 | |

The present search report has been drawn up for all claims

1

| Place of search | Date of completion of the search | Examiner |
|---|---|---|
| The Hague | 22 March 2011 | Piret, Bernard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 10 01 3126

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,P | JIANG ET AL: "DNA vaccines co-expressing GP5 and M proteins of porcine reproductive and respiratory syndrome virus (PRRSV) display enhanced immunogenicity", VACCINE, BUTTERWORTH SCIENTIFIC. GUILDFORD, GB, vol. 24, no. 15, 5 April 2006 (2006-04-05), pages 2869-2879, XP005337653, ISSN: 0264-410X<br>----- | 1-14 | |
| A,P | CHARERNTANTANAKUL ET AL: "Immune responses and protection by vaccine and various vaccine adjuvant candidates to virulent porcine reproductive and respiratory syndrome virus", VETERINARY IMMUNOLOGY AND IMMUNOPATHOLOGY, AMSTERDAM, NL, vol. 109, no. 1-2, 15 January 2006 (2006-01-15), pages 99-115, XP005196453, ISSN: 0165-2427<br>----- | 1-14 | TECHNICAL FIELDS SEARCHED (IPC) |
| A,P | JIANG ET AL: "Recombinant adenovirus expressing GP5 and M fusion proteins of porcine reproductive and respiratory syndrome virus induce both humoral and cell-mediated immune responses in mice", VETERINARY IMMUNOLOGY AND IMMUNOPATHOLOGY, AMSTERDAM, NL, vol. 113, no. 1-2, 15 September 2006 (2006-09-15), pages 169-180, XP005583043, ISSN: 0165-2427<br>-----<br>-/-- | 1-14 | |

The present search report has been drawn up for all claims

1

| Place of search | Date of completion of the search | Examiner |
|---|---|---|
| The Hague | 22 March 2011 | Piret, Bernard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 10 01 3126

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SÁNCHEZ C M ET AL: "Targeted recombination demonstrates that the spike gene of transmissible gastroenteritis coronavirus is a determinant of its enteric tropism and virulence.", JOURNAL OF VIROLOGY SEP 1999, vol. 73, no. 9, September 1999 (1999-09), pages 7607-7618, XP002440257, ISSN: 0022-538X<br>* the whole document *<br>----- | 1-14 | |
| A | DATABASE EMBL [Online]<br><br>6 November 2000 (2000-11-06), "Porcine transmissible gastroenteritis virus spike protein S mRNA, complete cds.", XP002440271, retrieved from EBI accession no. EMBL:AF302263<br>Database accession no. AF302263<br>* the whole document *<br>----- | 1-14 | TECHNICAL FIELDS SEARCHED (IPC) |
| A,P | ZHANG ET AL: "Complete genomic sequences, a key residue in the spike protein and deletions in nonstructural protein 3b of US strains of the virulent and attenuated coronaviruses, transmissible gastroenteritis virus and porcine respiratory coronavirus", VIROLOGY, ACADEMIC PRESS,ORLANDO, US, vol. 358, no. 2, 25 January 2007 (2007-01-25), pages 424-435, XP005853993, ISSN: 0042-6822<br>* the whole document *<br>-----<br>-/-- | 1-14 | |

The present search report has been drawn up for all claims

1

| Place of search | Date of completion of the search | Examiner |
|---|---|---|
| The Hague | 22 March 2011 | Piret, Bernard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 10 01 3126

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | PENZES ZOLTAN ET AL: "Complete genome sequence of transmissible gastroenteritis coronavirus PUR46-MAD clone and evolution of the Purdue virus cluster", VIRUS GENES, vol. 23, no. 1, August 2001 (2001-08), pages 105-118, XP002440258, ISSN: 0920-8569<br>----- | 1-14 | |
| A | BALLESTEROS M L ET AL: "Two Amino Acid Changes at the N-Terminus of Transmissible Gastroenteritis Coronavirus Spike Protein Result in the Loss of Enteric Tropism", VIROLOGY, ACADEMIC PRESS,ORLANDO, US, vol. 227, no. 2, 20 January 1997 (1997-01-20), pages 378-388, XP004460001, ISSN: 0042-6822<br>----- | 1-14 | |
| E | WO 2007/040876 A2 (UNIV NEBRASKA [US]; ANSARI ISRARUL H [US]; OSORIO FERNANDO A [US]; PAT) 12 April 2007 (2007-04-12)<br>* the whole document *<br>----- | 1-14 | TECHNICAL FIELDS SEARCHED (IPC) |
| A,P | ANSARI ISRARUL H ET AL: "Influence of N-linked glycosylation of porcine reproductive and respiratory syndrome virus GP5 on virus infectivity, antigenicity, and ability to induce neutralizing antibodies", JOURNAL OF VIROLOGY, THE AMERICAN SOCIETY FOR MICROBIOLOGY, US, vol. 80, no. 8, 1 April 2006 (2006-04-01), pages 3994-4004, XP002436516, ISSN: 0022-538X, DOI: DOI:10.1128/JVI.80.8.3994-4004.2006<br>-----<br>-/-- | 1-14 | |

The present search report has been drawn up for all claims

1

| Place of search | Date of completion of the search | Examiner |
|---|---|---|
| The Hague | 22 March 2011 | Piret, Bernard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 10 01 3126

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CHEN Z ET AL: "Neuropathogenicity and Sensitivity to Antibody Neutralization of Lactate Dehydrogenase-Elevating Virus Are Determined by Polylactosaminoglycan Chains on the Primary Envelope Glycoprotein", VIROLOGY, ACADEMIC PRESS,ORLANDO, US, vol. 266, no. 1, 5 January 2000 (2000-01-05), pages 88-98, XP004436186, ISSN: 0042-6822, DOI: DOI:10.1006/VIRO.1999.0050 ----- | 1-14 | |
| | | | TECHNICAL FIELDS SEARCHED (IPC) |

The present search report has been drawn up for all claims

| Place of search | Date of completion of the search | Examiner |
|---|---|---|
| The Hague | 22 March 2011 | Piret, Bernard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

1

EPO FORM 1503 03.82 (P04C01)

## ANNEX TO THE EUROPEAN SEARCH REPORT ON EUROPEAN PATENT APPLICATION NO. EP 10 01 3126

This annex lists the patent family members relating to the patent documents cited in the above-mentioned European search report.
The members are as contained in the European Patent Office EDP file on
The European Patent Office is in no way liable for these particulars which are merely given for the purpose of information.

22-03-2011

| Patent document cited in search report | Publication date | Patent family member(s) | Publication date |
|---|---|---|---|
| WO 0139797 A | 07-06-2001 | AR 027893 A1 | 16-04-2003 |
| | | AT 267246 T | 15-06-2004 |
| | | AU 785081 B2 | 14-09-2006 |
| | | AU 3005101 A | 12-06-2001 |
| | | AU 2006203327 A1 | 31-08-2006 |
| | | BR 0016145 A | 13-08-2002 |
| | | CA 2393325 A1 | 07-06-2001 |
| | | CN 1402780 A | 12-03-2003 |
| | | CN 1616670 A | 18-05-2005 |
| | | CN 1872994 A | 06-12-2006 |
| | | CZ 20021882 A3 | 13-11-2002 |
| | | DE 60010921 D1 | 24-06-2004 |
| | | DE 60010921 T2 | 11-05-2006 |
| | | DK 1234024 T3 | 13-09-2004 |
| | | EP 1234024 A2 | 28-08-2002 |
| | | ES 2170622 A1 | 01-08-2002 |
| | | ES 2218276 T3 | 16-11-2004 |
| | | HU 0203305 A2 | 28-01-2003 |
| | | IL 149859 A | 20-07-2009 |
| | | JP 2003515335 T | 07-05-2003 |
| | | MX PA02005525 A | 10-09-2004 |
| | | NO 20022419 A | 30-07-2002 |
| | | NZ 518990 A | 26-03-2004 |
| | | PL 356390 A1 | 28-06-2004 |
| | | PT 1234024 E | 31-08-2004 |
| | | SK 7612002 A3 | 06-11-2002 |
| | | TR 200401186 T4 | 21-07-2004 |
| | | US 2004086846 A1 | 06-05-2004 |
| | | US 2003148325 A1 | 07-08-2003 |
| | | US 2010167352 A1 | 01-07-2010 |
| | | ZA 200203560 A | 26-03-2003 |
| WO 02092827 A | 21-11-2002 | AT 473274 T | 15-07-2010 |
| | | BR 0209837 A | 14-12-2004 |
| | | CA 2447450 A1 | 21-11-2002 |
| | | CN 1620500 A | 25-05-2005 |
| | | EP 1470218 A2 | 27-10-2004 |
| | | ES 2348600 T3 | 09-12-2010 |
| | | HU 0400042 A2 | 28-04-2004 |
| | | JP 4463481 B2 | 19-05-2010 |
| | | JP 2005503132 T | 03-02-2005 |
| | | MX PA03010427 A | 06-12-2004 |
| | | NZ 529691 A | 21-12-2007 |
| | | US 2004071709 A1 | 15-04-2004 |
| | | US 2005130127 A1 | 16-06-2005 |
| | | US 2005186575 A1 | 25-08-2005 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO.

EP 10 01 3126

This annex lists the patent family members relating to the patent documents cited in the above-mentioned European search report.
The members are as contained in the European Patent Office EDP file on
The European Patent Office is in no way liable for these particulars which are merely given for the purpose of information.

22-03-2011

| Patent document cited in search report | Publication date | Patent family member(s) | Publication date |
|---|---|---|---|
| WO 02092827 A | | ZA 200309098 A | 21-02-2005 |
| WO 2006024542 A | 09-03-2006 | AR 051923 A1 | 21-02-2007 |
| | | AU 2005279303 A1 | 09-03-2006 |
| | | BR PI0514911 A | 24-06-2008 |
| | | CA 2591532 A1 | 09-03-2006 |
| | | EP 1789566 A1 | 30-05-2007 |
| | | EP 2295590 A1 | 16-03-2011 |
| | | US 2006165723 A1 | 27-07-2006 |
| EP 1008652 A | 14-06-2000 | AU 729044 B2 | 25-01-2001 |
| | | AU 1927797 A | 01-10-1997 |
| | | BR 9708061 A | 04-01-2000 |
| | | CA 2248978 A1 | 18-09-1997 |
| | | CN 1218513 A | 02-06-1999 |
| | | ES 2109189 A1 | 01-01-1998 |
| | | WO 9734008 A1 | 18-09-1997 |
| | | HU 0000356 A2 | 28-06-2000 |
| | | JP 3769300 B2 | 19-04-2006 |
| | | JP 2000513565 T | 17-10-2000 |
| | | PL 328791 A1 | 15-02-1999 |
| | | RU 2199584 C2 | 27-02-2003 |
| | | US 7041300 B1 | 09-05-2006 |
| WO 0189559 A | 29-11-2001 | AU 5658201 A | 03-12-2001 |
| | | BR 0111366 A | 20-05-2003 |
| | | CA 2409874 A1 | 29-11-2001 |
| | | CN 1443076 A | 17-09-2003 |
| | | EP 1283718 A2 | 19-02-2003 |
| | | JP 2003533989 T | 18-11-2003 |
| | | MX PA02011545 A | 06-06-2003 |
| | | PL 360096 A1 | 06-09-2004 |
| | | ZA 200210230 A | 27-10-2003 |
| WO 9918214 A | 15-04-1999 | AT 299531 T | 15-07-2005 |
| | | AT 387914 T | 15-03-2008 |
| | | AT 245191 T | 15-08-2003 |
| | | AU 756554 B2 | 16-01-2003 |
| | | AU 9355598 A | 27-04-1999 |
| | | BR 9812845 A | 08-08-2000 |
| | | CA 2305623 A1 | 15-04-1999 |
| | | CN 1278301 A | 27-12-2000 |
| | | CN 101724606 A | 09-06-2010 |
| | | DE 05014998 T1 | 20-11-2008 |
| | | DE 69816460 D1 | 21-08-2003 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 10 01 3126

This annex lists the patent family members relating to the patent documents cited in the above-mentioned European search report.
The members are as contained in the European Patent Office EDP file on
The European Patent Office is in no way liable for these particulars which are merely given for the purpose of information.

22-03-2011

| Patent document cited in search report | Publication date | Patent family member(s) | Publication date |
|---|---|---|---|
| | | DE 69816460 T2 | 27-05-2004 |
| WO 9918214 A | | DE 69830858 D1 | 18-08-2005 |
| | | DE 69830858 T2 | 20-04-2006 |
| | | DE 69839227 T2 | 12-03-2009 |
| | | DE 122005000009 I1 | 04-08-2005 |
| | | DE 122008000069 I1 | 05-11-2009 |
| | | DK 1386617 T3 | 07-07-2008 |
| | | DK 1019510 T3 | 10-11-2003 |
| | | EP 1386617 A1 | 04-02-2004 |
| | | EP 1741785 A1 | 10-01-2007 |
| | | EP 1019510 A1 | 19-07-2000 |
| | | ES 2246001 T3 | 01-02-2006 |
| | | ES 2302885 T3 | 01-08-2008 |
| | | ES 2203984 T3 | 16-04-2004 |
| | | HU 0003756 A2 | 28-02-2001 |
| | | JP 2001519159 T | 23-10-2001 |
| | | JP 2008086317 A | 17-04-2008 |
| | | KR 20060079256 A | 05-07-2006 |
| | | KR 20080023271 A | 12-03-2008 |
| | | KR 20090051274 A | 21-05-2009 |
| | | NL 300326 I1 | 03-03-2008 |
| | | PL 339631 A1 | 02-01-2001 |
| | | PT 1281760 E | 30-11-2005 |
| | | PT 1386617 E | 17-06-2008 |
| | | PT 1019510 E | 28-11-2003 |
| | | US 6368601 B1 | 09-04-2002 |
| WO 2007040876 A2 | 12-04-2007 | AT 461295 T | 15-04-2010 |
| | | CA 2620667 A1 | 12-04-2007 |
| | | CN 101300363 A | 05-11-2008 |
| | | DK 1929047 T3 | 12-07-2010 |
| | | EP 1929047 A2 | 11-06-2008 |
| | | ES 2343371 T3 | 29-07-2010 |
| | | JP 2009506129 T | 12-02-2009 |
| | | KR 20080064813 A | 09-07-2008 |
| | | US 2008233083 A1 | 25-09-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9734008 A
- ES P9600620
- WO 0139797 A


### Non-patent literature cited in the description


- **SIDDELL S.G.** *The Coronaviridae,* 1995
- **Alamazan F. ; Gonzales J.M. ; Penzes Z. ; Izeta A. ; Calvo E. ; Plana-Duran J. ; Enjuanes L.** Engineering the largest RNA virus genome as an infectious bacterial artificial chromosome. *Proc. Natl. Acad. Sci. USA,* 2000, vol. 97, 5516-5521
- **Alonso S. ; Izeta A. ; Sola I. ; Enjuanes L.** Transcription regulatory sequences and mRNA expression levels in the coronavirus transmissible gastroenteritis virus. *J. Virol.,* 2002, vol. 76, 1293-1308
- **Ansari I.H. ; Kwon B. ; Osorio F.A. ; Pattnaik A.K.** Influence of N-linked glycosylation of porcine reproductive and respiratory syndrome virus GP5 on virus infectivity, antigenicity, and ability to induce neutralizing antibodies. *J Virol.,* 2006, vol. 80 (8), 3994-4004
- **Botner A. ; Strandbygaard B. ; Sorensen K.J. ; Have P. ; Madsen K.G. ; Madsen E.S. ; Alexandersen S.** Appearance of acute PRRS-like symptoms in sow herds after vaccination with a modified live PRRS vaccine. *Vet. Rec.,* 1997, vol. 141, 497-499
- **Bullock W. ; Fernández J.M. ; Short J.M.** XL1-Blue: a high efficiency plasmid transforming recA E.coli strain with P-galactosidase selection. *Biotechniques,* 1987, vol. 8, 26-27
- **Carter Q.L. ; Curiel R.E.** Interleukin-12 (IL-12) ameliorates the effects of porcine respiratory and reproductive syndrome virus (PRRSV) infection. *Vet Immunol Immunopathol,* 2005, vol. 107, 105-118
- **Chang K.-Y. ; Tinoco I.** Characterization of a "kissing" hairpin complex derived from the human immunodeficiency virus genome. *Proc. Natl. Acad. Sci. USA,* 1994, vol. 91, 8705-8709
- **Charley B. ; Laude H.** Induction of alpha-interferon by transmissible gastroenteritis coronavirus: Role of transmembrane glycoprotein E1. *J. Virol.,* 1988, vol. 62, 8-10
- **Chung H.K. ; Chae C.** Expression of interleukin-10 and interleukin-12 in piglets experimentally infected with porcine reproductive and respiratory syndrome virus (PRRSV). *J. Comp. Path.,* 2003, vol. 129 (2-3), 205-212
- **Cousens L.P. ; Peterson R. ; Hsu S. ; Dorner A. ; Altman J.D. ; Ahmed R. ; Biron C.A.** Two roads diverged: interferon alpha/beta- and interleukin 12-mediated pathways in promoting T cell interferon gamma responses during viral infection. *J. Exp. Med,* 1999, vol. 189, 1315-1328
- **Delmas B ; Gelfi J. ; Kut E. ; Sjostrom H. ; Noren O. ; Laude H.** Determinants essential for the transmissible gastroenteritis virus-receptor interaction reside within a domain of aminopeptidase-N that is distinct from the enzymatic site. *J Virol.,* 1994, vol. 68 (8), 5216-24
- **Doyle L.P. ; Hutchings L.M.** A transmissible gastroenteritis in pigs. *J. Amer. Vet. Med. Assoc.,* 1946, vol. 108, 257-259
- Virus based vectors for gene expression in mammalian cells; Coronaviruses. **Enjuanes L. et al.** Gene transfer and expression in mammalian cells. 2003, 151-158
- **Fenaux M. ; Opriessnig T. ; Halbur P.G. ; Meng X.J.** Immunogenicity and pathogenicity of chimeric infectious DNA clones of pathogenic porcine circovirus type 2 (PCV2) and nonpathogenic PCV1 in weanling pigs. *J. Virol.,* 2003, vol. 77 (20), 11232-43
- **Fenaux M. ; Opriessnig T. ; Halbur P.G. ; Elvinger F. ; Meng X.J.** A chimeric porcine circovirus (PCV) with the immunogenic gene of the pathogenic PCV type 2 (PCV2) cloned into the genomic backbone of the non-pathogenic PCV1 induces protective immunity against PCV2 infection in pigs. *J. Virol.,* 2004, vol. 78 (12), 6297-6303
- **Foss D.L. ; Zilliox M.J. ; Meier W. ; Zuckermann F. ; Murtaugh M.P.** Adjuvant danger signals increase the immune response to porcine reproductive and respiratory syndrome virus. *Viral Immunol.,* 2002, vol. 15 (4), 557-566

- **Gonzalez J.M. ; Penzes Z. ; Almazan F. ; Calvo E. ; Enjuanes L.** Stabilization of a full-length infectious cDNA clone of transmissible gastroenteritis coronavirus by the insertion of an intron. *J. Virol.,* 2002, vol. 76, 4655-4661
- **Graham R.L. ; Sims A.C. ; Brockway S.M. ; Baric R.S. ; Denison M.R.** The nsp2 replicase proteins of murine hepatitis virus and severe acute respiratory syndrome coronavirus are dispensable for viral replication. *J. Virol.,* 2005, vol. 79, 13399-13411
- **Haelterman E.O. ; Pensaert M.B.** Pathogenesis of transmissible gastroenteritis of swine. *Proc. 18th World Vet. Congress,* 1967, vol. 2, 569-572
- **Hanahan D. ; Jessee J. ; Bloom F.R.** Plasmid transformation of Escherichia coli and other bacteria. *Methods Enzymol.,* 1991, vol. 204, 63-113
- **Hannan P.C. ; Bhogal B.S. ; Fish J.P.** Tylosin tartrate and tiamutilin effects on experimental piglet pneumonia induced with pneumonic pig lung homogenate containing mycoplasmas, bacteria and viruses. *Res. Vet. Sci.,* 1982, vol. 33, 76-88
- **Hertzig T. ; Scandella E. ; Schelle B. ; Ziebuhr J. ; Siddell S.G. ; Ludewig B. ; Thiel V.** Rapid identification of coronavirus replicase inhibitors using a selectable replicon RNA. *J. Gen. Virol,* 2004, vol. 85, 1717-1725
- **Izeta A. ; Smerdou C. ; Alonso S. ; Penzes Z. ; Méndez A. ; Plana-Durán J. ; Enjuanes L.** Replication and packaging of transmissible gastroenteritis coronavirus-derived synthetic minigenomes. *J. Virol.,* 1999, vol. 73, 1535-1545
- **Kiyono et al.** Mucosal Vaccines. Academic Press, 1996
- **Labarque G. ; Reeth K.V. ; Nauwynck H. ; Drexler C. ; Van Gucht S. ; Pensaert S.** Impact of genetic diversity of European-type porcine reproductive and respiratory syndrome virus strain on vaccine efficacy. *Vaccine,* 2004, vol. 22, 4183-4190
- **Laude H. ; Rasschaert D. ; Delmas B. ; Godet M. ; Gelfi J. ; Bernard C.** Molecular biology of transmissible gastroenteritis virus. *Vet. Microbiol.,* 1990, vol. 23, 147-154
- **Le Bon A. ; Schiavoni G. ; D'Agostino G. ; Gresser I. ; Belardelli F. ; Tough D.F.** Type I interferons potently enhance humoral immunity and can promote isotype switching by stimulating dendritic cells in vivo. *Immunity,* 2001, vol. 14, 461-470
- **Lopez O.J. ; Osorio F.A.** Role of neutralizing antibodies in PRRSV protective immunity. *Vet Immunol Immunopathol,* 2004, vol. 102, 155-163
- **McClurkin A.W. ; Noman J.O.** Studies on transmissible gastroenteritis of swine. II. Selected characteristics of a cytopathogenic virus common to five isolates from transmissible gastroenteritis. *Can. J. Comp. Med. Vet. Sci.,* 1966, vol. 30, 190-198
- **Meier W.A. ; Galeota J. ; Osorio F.A. ; Husmann R.J. ; Schnitzlein W.M. ; Zuckermann F.A.** Gradual development of the interferon-gamma response of swine to porcine reproductive and respiratory syndrome virus infection or vaccination. *Virology,* 2003, vol. 309, 18-31
- **Meng X.J.** Heterogeneity of porcine reproductive and respiratory syndrome virus: implications for current vaccine efficacy and future vaccine development. *Vet. Microbiol.,* 2000, vol. 74, 309-329
- **Mengeling W.L. ; Lager K.M. ; Vorwald A.C. ; Koehler K.J.** Strain specificity of the immune response of pigs following vaccination with various strains of porcine reproductive and respiratory syndrome virus. *Vet. Microbiol.,* 2003, vol. 93 (1), 13-24
- **Mengeling W.L. ; Lager K.M. ; Vorwald A.C. ; Clouser D.F.** Comparative safety and efficacy of attenuated single-strain and multi-strain vaccines for porcine reproductive and respiratory syndrome. *Vet. Microbiol.,* 2003, vol. 93 (1), 25-38
- **Murtaugh M.P. ; Xiao Z. ; Zuckermann F.** Comparison of the structural protein coding sequences of the VR-2332 and Lelystad virus strains of the PRRS virus. *Arch. Virol.,* 1995, vol. 140, 1451-1460
- **Nielsen H.S. ; Oleksiewicz M.B. ; Forsberg R. ; Stadejek T. ; Botner A. ; Storgaard T.** Reversion of a live porcine reproductive and respiratory syndrome virus vaccine investigated by parallel mutations. *J. Gen. Virol.,* 2001, vol. 82, 1263-1272
- **Ortego J. ; Escors D. ; Laude H. ; Enjuanes L.** Generation of a replication-competent, propagation deficient virus vector based on the transmissible gastroenteritis coronavirus genome. *J. Virol.,* 2002, vol. 76, 11518-11529
- **Ortego J. ; Sola I. ; Almazan F. ; Ceriani J.E. ; Riquelme C. ; Balach M. ; Plana-Duran J. ; Enjuanes L.** Transmissible gastroenteritis coronavirus gene 7 is not essential but influences in vivo replication and virulence. *Virol,* 2003, vol. 308, 13-22
- **Osorio F.A. ; Zuckermann F. ; Wills R. ; Meier W. ; Christian S. ; Galeota J. ; Doster A.R.** PRRSV: comparison of commercial vaccines in their ability to induce protection against current PRRSV strains of high virulence. *Allen D. Lennan Swine Conf.,* 1998, 179-182
- **Osorio Y. ; Ghiasi H.** Recombinant herpes simplex virus type 1 (HSV-1) codelivering interleukin-12p35 as a molecular adjuvant enhances the protective immune response against ocular HSV-1 challenge. *J. Virol.,* 2005, vol. 79 (6), 3297-3308
- **Ostrowski M. ; Galeota J.A. ; Jar A.M. ; Platt K.B. ; Osorio F.A. ; Lopez O.J.** Identification of neutralizing and nonneutralizing epitopes in the porcine reproductive and respiratory syndrome virus GP5 ectodomain. *J. Virol.,* 2000, vol. 76, 4241-4250
- Progress in Vaccinology. Veterinary Vaccines. Springer Verlag, 1989, vol. 4

- Veterinary Vaccinology. Elsevier Science B.V, 1997
- **Penzes Z. ; Gonzales J.M. ; Calvo E. ; Izeta A. ; Smerdou C. ; Mendez A. ; Sanches C.M. ; Sola I. ; Almazan F. ; Enjuanes L.** Complete genome sequence of Transmissible Gastroenteritis Coronavirus PUR46-MAD clone and evolution of the purdue virus cluster. *Virus Genes,* 2001, vol. 23 (1), 105-118
- **Pesch S. ; Meyer C. ; Ohlinger V.F.** New insights into genetic diversity of European porcine reproductive and respiratory syndrome virus (PRRSV). *Vet. Microbiol.,* 2005, vol. 107, 31-48
- **Pfeffer L.M. ; Dinarello C.A. ; Herberman R.B. ; Williams B.R. ; Borden E.C. ; Bordens R. ; Walter M.R. ; Nagabhushan T.L. ; Trotta P.P. ; Pestka S.** Biological properties of recombinant alpha-interferons: 40th anniversary of the discovery of interferons. *Cancer Res.,* 1998, vol. 58, 2489-2499
- **Rodriguez F. ; Harkins S. ; Redwine J.M. ; De Pereda J.M. ; Whitton J.L.** CD4+ T cells induced by a DNA vaccine: immunological consequences of epitope-specific lysosomal targeting. *J. Virol.,* 2001, vol. 75 (21), 10421-10430
- **Sambrook et al.** Molecular Cloning: A Laboratory Manual. Cold Spring Harbor Laboratory, 1989
- **Sánchez C.M. ; Izeta A. ; Sánchez-Morgado J.M. ; Alonso S. ; Sola I. ; Balasch M. ; Plana-Durán J. ; Enjuanes, L.** Targeted recombination demonstrates that the spike gene of transmissible gastroenteritis coronavirus is a determinant of its enteric tropism and virulence. *J. Virol.,* 1999, vol. 73, 7607-7618
- **Sawicki ; Sawicki.** Coronavirus transcription: subgenomic mouse hepatitis virus replicative intermediates function in RNA synthesis. *J. Virol.,* 1990, vol. 64 (3), 1050-6
- **Sethna P.B. ; Hung S.-L. ; Brian D.A.** Coronavirus subgenomic minus-strand RNAs and the potential for mRNA replicons. *Proc. Natl. Acad. Sci. USA,* 1989, vol. 86, 5626-5630
- The Coronaviridae. **Siddell S.G.** The Viruses. Plenum Press, 1985
- **Sola I ; Alonso S. ; Zúñiga S. ; Balasch M. ; Plana-Duran J. ; Enjuanes L.** Engineering the transmissible gastroenteritis virus genome as an expression vector inducing lactogenic immunity. *J. Virol.,* 2003, vol. 77, 4357-4369
- **Suradhat S. ; Thanawongnuwech R. ; Poovorawan Y.** Upregulation of IL-10 gene expression in porcine peripheral blood mononuclear cells by porcine reproductive and respiratory syndrome virus. *J. Gen. Virol.,* 2003, vol. 84, 453-459
- **Thacker E.L. ; Halbur P.G. ; Ross R.F. ; Thanawongnuwech R. ; Thacker B.J.** Mycoplasma hyopneumoniae potentiation of porcine reproductive and respiratory syndrome virus-induced pneumonia. *J Clin Microbiol.,* 1999, vol. 37 (3), 620-627
- **Thacker E.L. ; Thacker B.J. ; Young T.F. ; Halbur P.G.** Effect of vaccination on the potentiation of porcine reproductive and respiratory syndrome virus (PRRSV)-induced pneumonia by Mycoplasma hyopneumoniae. *Vaccine,* 2000, vol. 18 (13), 1244-52
- **Toka F.N. et al.** Molecular adjuvants for mucosal immunity. *Immunol Rev.,* 2004, vol. 199, 100-112
- Coronavirus replication, transcription, and RNA recombination. **van der Most R.G. ; Spaan W.J.M.** The Coronaviridae. Plenum Press, 1995, 11-31
- **Vezina S.A. ; Loemba H. ; Fournier M. ; Dea S. ; Archambault D.** Antibody production and blastogenic response in pigs experimentally infected with porcine reproductive and respiratory syndrome virus. *Vet. Q.,* 1996, vol. 13, 121-130
- **Wensvoort G. ; Terpstra C. ; Boonstra J. ; Bloemraad M. ; Van Zaane D.** Production of monoclonal antibodies against swine fever virus and their use in laboratory diagnosis. *Vet. Microbiol.,* 1986, vol. 12 (2), 101-108
- **Wensvoort G. ; Terpstra C. ; Pol J.M. ; ter Laak E.A. ; Bloemraad M. ; de Kluyver E.P. ; Kragten C. ; van Buiten L. ; den Besten A. ; Wagenaar F. et al.** Mystery swine disease in The Netherlands: the isolation of Lelystad virus. *Vet. Q.,* 1991, vol. 13, 121-130
- **Wissink E.H.J. ; Kroeke M.V. ; van Wijk H.A.R. ; Rijsewijk F.A.M. ; Meulenberg J.J.M. ; Rottier P.J.M.** Envelope protein requirements for the assembly of infectious virions of porcine reproductive and respiratory syndrome virus. *J. virol.,* 2005, vol. 79 (19), 12495-12506
- **Yoon K.J. ; Zimmernan J.J. ; Swenson S.L. ; McGinley M.J. ; Eernisse K.A. ; Brevik A. ; Rhinehart L.L. ; Frey M.L. ; Hill H.T. ; Platt K.B.** Characterization of the humoral immune response to porcine reproductive and respiratory syndrome (PRRSV) virus infection. *J. Vet. Diagn. Invest.,* 1995, vol. 7, 305-312

SEQUENCE LISTING

<110> Consejo Superior de Investigaciones Cientificas

<120> Nucleic Acids encoding TGEV and PRRSV Sequences for Improved Expression of PRRSV Sequences

<130> P71617

<150> EP05026255
<151> 2005-12-01

<160> 19

<170> PatentIn version 3.3

<210> 1
<211> 4350
<212> DNA
<213> PRRSV

<400> 1
atgaaaaaac tatttgtggt tttggtcgta atgccattga tttatggaga caattttcct 60

tgttctaaat tgactaatag aactataggc aaccagtgga atctcattga aaccttcctt 120

ctaaactata gtagtaggtt accacctaat tcagatgtgg tgttaggtga ttattttcct 180

actgtacaac cttggtttaa ttgcattcgc aataatagta atgaccttta tgttacactg 240

gaaaatctta aagcattgta ttgggattat gctacagaaa atatcacttg gaatcacaga 300

caacggttaa acgtagtcgt taatggatac ccatactcca tcacagttac aacaacccgc 360

aattttaatt ctgctgaagg tgctattata tgcatttgta agggctcacc acctactacc 420

accacagaat ctagtttgac ttgcaattgg ggtagtgagt gcaggttaaa ccataagttc 480

```
cctatatgtc cttctaattc agaggcaaat tgtggtaata tgctgtatgg cctacaatgg    540
tttgcagatg aggttgttgc ttatttacat ggtgctagtt accgtattag ttttgaaaat    600
caatggtctg gcactgtcac atttggtgat atgcgtgcga caacattaga agtcgctggc    660
acgcttgtag acctttggtg gtttaatcct gtttatgatg tcagttatta tagggttaat    720
aataaaaatg gtactaccgt agtttccaat tgcactgatc aatgtgctag ttatgtggct    780
aatgttttta ctacacagcc aggaggtttt ataccatcag attttagttt taataattgg    840
ttccttctaa ctaatagctc cacgttggtt agtggtaaat tagttaccaa acagccgtta    900
ttagttaatt gcttatggcc agtccctagc tttgaagaag cagcttctac attttgtttt    960
gagggtgctg gctttgatca atgtaatggt gctgttttaa ataatactgt agacgtcatt   1020
aggttcaacc ttaattttac tacaaatgta caatcaggta agggtgccac agtgttttca   1080
ttgaacacaa cgggtggtgt cactcttgaa atttcatgtt ataatgatac agtgagtgac   1140
tcgagctttt tcagttacgg tgaaattccg ttcggcgtaa ctgatggacc acggtactgt   1200
tacgtacact ataatggcac agctcttaag tatttaggaa cattaccacc tagtgtcaag   1260
gagattgcta ttagtaagtg gggccatttt tatattaatg gttacaattt ctttagcaca   1320
tttcctattg attgtatatc ttttaatttg accactggtg atagtgacgt tttctggaca   1380
atagcttaca catcgtacac tgaagcatta gtacaagttg aaaacacagc tattacaaag   1440
gtgacgtatt gtaatagtca cgttaataac attaaatgct ctcaaattac tgctaatttg   1500
aataatggat tttatcctgt ttcttcaagt gaagttggtc ttgtcaataa gagtgttgtg   1560
ttactaccta gcttttacac acataccatt gttaacataa ctattggtct tggtatgaag   1620
cgtagtggtt atggtcaacc catagcctca acattaagta acatcacact accaatgcag   1680
```

gatcacaaca ccgatgtgta ctgtattcgt tctgaccaat tttcagttta tgttcattct 1740

acttgcaaaa gtgctttatg ggacaatatt tttaagcgaa actgcacgga cgttttagat 1800

gccacagctg ttataaaaac tggtacttgt cctttctcat ttgataaatt gaacaattac 1860

ttaactttta acaagttctg tttgtcgttg agtcctgttg gtgctaattg taagtttgat 1920

gtagctgccc gtacaagaac caatgagcag gttgttagaa gtttgtatgt aatatatgaa 1980

gaaggagaca acatagtggg tgtaccgtct gataatagtg gtgtgcacga tttgtcagtg 2040

ctacacctag attcctgcac agattacaat atatatggta gaactggtgt tggtattatt 2100

agaaaaacta acaggacgct acttagtggc ttatattaca catcactatc aggtgatttg 2160

ttaggtttta aaaatgttag tgatggtgtc atctactctg taacgccatg tgatgtaagc 2220

gcacaagcag ctgttattga tggtaccata gttggggcta tcacttccat taacagtgaa 2280

ctgttaggtc taacacattg gacaacaaca cctaattttt attactactc tatatataat 2340

tacacaaatg ataggactcg tggcactgca attgacagta atgatgttga ttgtgaacct 2400

gtcataacct attctaacat aggtgtttgt aaaaatggtg cttttgtttt tattaacgtc 2460

acacattctg atggagacgt gcaaccaatt agcactggta atgtcacgat acctacaaac 2520

tttaccatat ccgtgcaagt cgaatatatt caggtttaca ctacaccagt gtcaatagac 2580

tgttcaagat atgtttgtaa tggtaaccct aggtgtaaca aattgttaac acaatacgtt 2640

tctgcatgtc aaactattga gcaagcactt gcaatgggtg ccagacttga aaacatggag 2700

gttgattcca tgttgtttgt ttctgaaaat gcccttaaat tggcatctgt tgaagcattc 2760

aatagttcag aaactttaga ccctatttac aaagaatggc ctaatatagg tggttcttgg 2820

ctagaaggtc taaaatacat acttccgtcc cataatagca aacgtaagta tcgttcagct 2880

atagaggact tgctttttga taaggttgta acatctggtt taggtacagt tgatgaagat 2940

tataaacgtt gtacaggtgg ttatgacata gctgacttag tatgtgctca atactataat 3000

ggcatcatgg tgctacctgg tgtggctaat gctgacaaaa tgactatgta cacagcatcc 3060

cttgcaggtg gtataacatt aggtgcactt ggtggaggcg ccgtggctat acctttgca 3120

gtagcagttc aggctagact taattatgtt gctctacaaa ctgatgtatt gaacaaaaac 3180

cagcagattc tggctagtgc tttcaatcaa gctattggta acattacaca gtcatttggt 3240

aaggttaatg atgctataca tcaaacatca cgaggtcttg ctactgttgc taaagcattg 3300

gcaaaagtgc aagatgttgt caacatacaa gggcaagctt taagccacct aacagtacaa 3360

ttgcaaaata atttccaagc cattagtagt tctattagtg acatttataa taggcttgac 3420

gaattgagtg ctgatgcaca agttgacagg ctgatcacag gaagacttac agcacttaat 3480

gcatttgtgt ctcagactct aaccagacaa gcggaggtta gggctagtag acaacttgcc 3540

aaagacaagg ttaatgaatg cgttaggtct cagtctcaga gattcggatt ctgtggtaat 3600

ggtacacatt tgttttcact cgcaaatgca gcaccaaatg gcatgatttt ctttcacaca 3660

gtgctattac caacggctta tgaaactgtg actgcttggc caggtatttg tgcttcagat 3720

ggtgatcgca cttttggact tgtcgttaaa gatgtccagt tgactttgtt tcgtaatcta 3780

gatgacaagt tctatttgac ccccagaact atgtatcagc ctagagttgc aactagttct 3840

gactttgttc aaattgaagg gtgcgatgtg ctgtttgtta atgcaactgt aagtgatttg 3900

cctagtatta tacctgatta tattgatatt aatcagactg ttcaagacat attagaaaat 3960

tttagaccaa attggactgt acctgagttg acatttgaca tttttaacgc aacctattta 4020

aacctgactg gtgaaattga tgacttagaa tttaggtcag aaaagctaca taacaccact 4080

gtagaacttg ccattctcat tgacaacatt aacaatacat tagtcaatct tgaatggctc 4140

aatagaattg aaacctatgt aaaatggcct tggtatgtgt ggctactaat aggcttagta 4200

gtaatatttt gcataccatt actgctattt tgctgttgta gtacaggttg ctgtggatgc 4260

ataggttgtt taggaagttg ttgtcactct atatgtagta gaagacaatt tgaaaattac 4320

gaaccaattg aaaaagtgca cgtccattaa 4350

<210> 2
<211> 606
<212> DNA
<213> PRRSV

<400> 2
atgagatgtt ctcacaaatt ggggcgtttc ttgactcctc actcttgctt ctggtggctt 60

tttttgctgt gtaccggctt gtcctggtcc tttgtcgctg gcagcggcag cagctcgaca 120

taccaataca tatataactt aacgatatgc gagctgaatg ggaccgactg gttgtccaac 180

cattttgatt gggcagtcga gacctttgtg ctttacccgg ttgccactca tatcctctca 240

ctgggttttc tcacaacaag ccattttttt gacgcgctcg gtctcggcgc tgtgtccact 300

acaggatttg ttggcgggcg gtatgtactc agcagcgtgt acggcgcttg tgctttcgca 360

gcgctcgtat gttttgtcat ccgtgctgtt aaaaattgca tggcttgccg ctatgcccac 420

acccggttta ccaacttcat tgtggacgac cgggggagaa tccatcggtg gaagtctcca 480

atagtggtag agaaattggg caaagctgaa gtcggtggcg accttgtcac catcaaacat 540

gtcgtcctcg aaggggttaa agctcaaccc ttgacgagga cttcggctga gcaatgggaa 600

gcctag 606

<210> 3
<211> 522
<212> DNA
<213> PRRSV

<400> 3
atgggaagcc tagacgattt ttgcaatgat tctaccgccg cacaaaagct tgtgctagcc 60

tttagcatta catatacacc tataatgata tacgccctta aggtgtcacg cggccgactc 120

ctggggctgt tgcacatcct aatattcctg aattgttctt tcacattcgg atacatgaca 180

tatgtgcgtt ttcaatccac caaccgtgtc gcacttactc tggggctgt tgtcgccctt 240

ctgtggggtg tttacagctt cacagagtca tggaagtttg ttacttccag atgcagattg 300

tgttgcctag gccggcgata cattctggcc cctgcccatc acgtagaaag tgctgcaggt 360

ctccattcaa tcccagcgtc tggtaaccga gcatacgctg tgagaaagcc cggactaaca 420

tcagtgaacg gcactctagt tccaggactt cggagcctcg tgctgggcgg caaacgagct 480

gttaaacgag gagtggttaa cctcgtcaag tatggccggt aa 522

<210> 4
<211> 37
<212> DNA
<213> Artificial
<220>
<223> oligonucleotide primer

<400> 4
aacaggtcct accatgagat gttctcacaa attgggg 37

<210> 5
<211> 38
<212> DNA

<213> Artificial

<220>
<223> oligonucleotide primer

<400> 5
ccgctaagcc taggcttccc attgctcagc cgaagtcc 38

<210> 6
<211> 77
<212> DNA
<213> Artificial
<220>
<223> oligonucleotide primer

<400> 6
cggctgagca atgggaagcc tagaaaatta ttacatatgg tataactaaa caaaatggga 60

agcctagacg atttttg 77

<210> 7
<211> 21
<212> DNA
<213> Artificial
<220>
<223> oligonucleotide primer

<400> 7
gccggcctag acaacacaat c 21

<210> 8
<211> 21
<212> DNA
<213> Artificial
<220>
<223> oligonucleotide primer

<400> 8
gattgtgttg tctaggccgg c 21

<210> 9
<211> 29
<212> DNA
<213> Artificial
<220>
<223> oligonucleotide primer

<400> 9
gctaagctta ccggccatac ttgacgagg 29

<210> 10
<211> 7
<212> PRT
<213> PRRSV

<400> 10

Thr Tyr Gln Tyr Ile Tyr Asn
1 5

<210> 11
<211> 20
<212> PRT
<213> Sus scrofa

<400> 11

Arg Gly Gln Gly Ser Met Asp Glu Gly Thr Ala Asp Glu Arg Ala Pro
1 5 10 15

Leu Ile Arg Thr
20

<210> 12
<211> 60
<212> DNA

<213> Sus scrofa

<400> 12

agaggacagg gatccatgga tgagggaact gcagacgaac gagcacccct catacgaacc 60

<210> 13

<211> 564

<212> DNA

<213> Artificial

<220>

<223> neutralizing PRRSV epitope ORF5-ORF6

<400> 13

tcgacatacc aatacatata taacttaacg atatgcgagc tgatgggaag cctagacgat 60

ttttgcaatg attctaccgc cgcacaaaag cttgtgctag cctttagcat tacatataca 120

cctataatga tatacgccct taaggtgtca cgcggccgac tcctggggct gttgcacatc 180

ctaatattcc tgaattgttc tttcacattc ggatacatga catatgtgcg ttttcaatcc 240

accaaccgtg tcgcacttac tctgggggct gttgtcgccc ttctgtgggg tgtttacagc 300

ttcacagagt catggaagtt tgttacttcc agatgcagat tgtgttgcct aggccggcga 360

tacattctgg cccctgccca tcacgtagaa agtgctgcag gtctccattc aatcccagcg 420

tctggtaacc gagcatacgc tgtgagaaag cccggactaa catcagtgaa cggcactcta 480

gttccaggac ttcggagcct cgtgctgggc ggcaaacgag ctgttaaacg aggagtggtt 540

aacctcgtca agtatggccg gtaa 564

<210> 14

<211> 624

<212> DNA

<213> Artificial

<220>

<223> Epitope ORF5-ORF6-LIMP-II

<400> 14

tcgacatacc aatacatata taacttaacg atatgcgagc tgatgggaag cctagacgat 60

ttttgcaatg attctaccgc cgcacaaaag cttgtgctag cctttagcat tacatataca 120

cctataatga tatacgccct taaggtgtca cgcggccgac tcctggggct gttgcacatc 180

ctaatattcc tgaattgttc tttcacattc ggatacatga catatgtgcg ttttcaatcc 240

accaaccgtg tcgcacttac tctgggggct gttgtcgccc ttctgtgggg tgtttacagc 300

ttcacagagt catggaagtt tgttacttcc agatgcagat tgtgttgcct aggccggcga 360

tacattctgg cccctgccca tcacgtagaa agtgctgcag gtctccattc aatcccagcg 420

tctggtaacc gagcatacgc tgtgagaaag cccggactaa catcagtgaa cggcactcta 480

gttccaggac ttcggagcct cgtgctgggc ggcaaacgag ctgttaaacg aggagtggtt 540

aacctcgtca agtatggccg gagaggacag ggatccatgg atgagggaac tgcagacgaa 600

cgagcacccc tcatacgaac ctaa 624

<210> 15
<211> 5
<212> PRT
<213> Artificial
<220>
<223> peptide linker

<400> 15

Gly Gly Pro Gly Gly
1 5

<210> 16
<211> 19
<212> DNA
<213> Artificial

<220>
<223> oligonucleotide primer

<400> 16
ttccctctgc ttgcaatcg 19

<210> 17
<211> 20
<212> DNA
<213> Artificial

<220>
<223> oligonucleotide primer

<400> 17
ggatgaaagc gacgcagttc 20

<210> 18
<211> 18
<212> DNA
<213> Artificial

<220>
<223> oligonucleotide probe (MGB probe)

<400> 18
acggctttta atcaaggc 18

<210> 19
<211> 31
<212> DNA

<213> Artificial

<220>

<223> artificial sequence derived from the N gene

<400> 19

```
aaaattatta catatggtat aactaaacaa a                                      31
```